# EUROPEAN PATENT SPECIFICATION

(11) **EP 2 262 889 B1**
(45) Date of publication and mention of the grant of the patent: **03.05.2017**
(21) Application number: 09717438.7
(22) Date of filing: 02.03.2009
(51) Int. Cl.: C12N 5/0784

(54) **METHOD FOR THE PRODUCTION OF DENDRITIC PRECURSOR CELL LINE**
METHODE ZUR HERSTELLUNG DENDRITISCHER ZELLVORLÄUFERPOPULATIONEN
MÉTHODE POUR LA PRÉPARATION DES POPULATIONS DE PRÉCURSEURS DE CELLULES DENDRITIQUES

(30) Priority: 03.03.2008 US 67870 P
(43) Date of publication of application: 22.12.2010
(73) Proprietor: UNIVERSITY OF TOLEDO, Toledo, OH 43606 (US)
(72) Inventor: TAKASHIMA, Akira, Sylvania, OH 43560 (US); MATSUSHIMA, Hironori, Toledo, OH 43528 (US); GENG, Shuo, Toledo, OH 43614 (US)
(74) Representative: Cottrill, Emily Elizabeth Helen
(86) International application number: PCT/US2009/035727
(87) International publication number: WO 2009/111396

(56) References cited:
- WO-A2-2004/065408
- US-A1- 2007 269 414
- STEPTOE RAYMOND J ET AL: "Autointmune diabetes is suppressed by transfer of proinsulin-encoding Gr-1+ myeloid progenitor cells that differentiate in vivo into resting dendritic cells", DIABETES, vol. 54, no. 2, February 2005 (2005-02), pages 434-442, XP002665244, ISSN: 0012-1797
- LAMB ROBERTA J ET AL: "Identification and characterization of novel bone marrow myeloid DEC205(+)Gr-1(+) cell subsets that differentially express chemokine and TLRs", JOURNAL OF IMMUNOLOGY, vol. 178, no. 12, June 2007 (2007-06), pages 7833-7839, XP002665245, ISSN: 0022-1767
- KOIKE EIKO ET AL: "Accelerated differentiation of bone marrow-derived dendritic cells in atopic prone mice", INTERNATIONAL IMMUNOPHARMACOLOGY, vol. 8, no. 13-14, December 2008 (2008-12) , pages 1737-1743, XP002665246, ISSN: 1567-5769
- MORANDI BARBARA ET AL: "Distinctive lack of CD48 expression in subsets of human dendritic cells tunes NK cell activation", JOURNAL OF IMMUNOLOGY, vol. 175, no. 6, September 2005 (2005-09), pages 3690-3697, XP002665247, ISSN: 0022-1767
- KAHN ET AL.: 'Characterization of dendritic cells generated in vivo by an E. coli derived chimeric dual receptor agonist.' MED SCI MONIT vol. 8, no. 12, December 2002, pages BR504 - BR514, XP008141628
- SIOUD ET AL.: 'TLR agonists induce the differentiation of human bone marrow CD34+ progenitors into CD11c+ CD80/86+ DC capable of inducing a Th1-type response' EUR JOUR IMMUNOL vol. 37, no. 10, October 2007, pages 2834 - 2846, XP008141629
- DIETLIN ET AL.: 'Mycobacteria-induced Gr-1+ subsets from distinct myeloid lineages have opposite effects on T cell expansion' JOUR LEUKO BIOL vol. 81, no. 5, 16 February 2007, pages 1205 - 1212, XP008141630
- EBIHARA ET AL.: 'Immortalized dendritic cell line with efficient cross-priming ability established from transgenic mice harboring the temperature-sensitive SV40 large T-antigen gene' JOUR BIOCHEM vol. 136, no. 3, 2004, pages 321 - 328, XP008141631
- PILLARISETTY ET AL.: 'GM-CSF Expands Dendritic Cells and Their Progenitors in Mouse Liver' HEPATOLOGY vol. 37, no. 3, March 2003, pages 641 - 652, XP008141634
- WANG ET AL.: 'Antigen targeting to dendritic cells with bispecific antibodies' J IMMUNOL METHODS. vol. 306, no. 1-2, 30 November 2005, pages 80 - 92, XP008141635
- NAIK: 'Distinct precursors of the dendritic cell subtypes' PH.D. THESIS AT THE WALTER & ELIZA HALL INSTITUTE OF MEDICAL RESEARCH, [Online] XP008141636 Retrieved from the Internet: <URL:http://eprints.infodiv.unimelb.edu.au/ archive/00001885> [retrieved on 2009-06-29]
- SHORTMAN ET AL.: 'Steady-state and inflammatory dendritic-cell development' NAT REV IMMUNOL vol. 7, no. 1, January 2007, pages 19 - 30, XP008141646
- 'JAX DATA SHEET' STRAIN NAME: B6.CG-TG(ACTB-DSRED'MST)1NAGY/J, [Online] 05 January 2008, Retrieved from the Internet: <URL:http://web.archive.org/web/20080105221 828/http:/ljaxmice.jax.org/strain/006051.ht ml> [retrieved on 2009-06-30]

### SEQUENCE LISTING

<110> UNIVERSITY OF TOLEDO
<120> DENDRITIC CELL PRECURSOR POPULATIONS, DENDRITIC CELL POPULATIONS DERIVED THEREFROM AND USES THEREOF
<130> 53-29878
<140> PCT/US2009/035727
   <141> 2009-03-02
<150> 61/067,870
   <151> 2008-03-03
<160> 4
<170> PatentIn version 3.5
<210> 1
   <211> 27
   <212> DNA
   <213> Artificial Sequence
<220>
   <223> Description of Artificial Sequence: Synthetic primer
<400> 1
   gggaattccg gtcgccacca tggcctc 27
<210> 2
   <211> 29
   <212> DNA
   <213> Artificial Sequence
<220>
   <223> Description of Artificial Sequence: Synthetic primer
<400> 2
   ggagatctac acattgatcc tagcagaag 29
<210> 3
   <211> 24
   <212> DNA
   <213> Artificial Sequence
<220>
   <223> Description of Artificial sequence: Synthetic primer
<400> 3
   tgctggttgt tgtgctgtct catc 24
<210> 4
   <211> 24
   <212> DNA
   <213> Artificial sequence
<220>
   <223> Description of Artificial Sequence: Synthetic primer
<400> 4
   cacgtacacc ttggagccgt actg 24

## Description

### FIELD OF THE INVENTION

The invention relates generally to methods of making and using certain defined subsets of dendritic cells, more particularly, to methods of *in vitro* production of a subset of dendritic cells - gr-DC.

### BACKGROUND

There is no admission that the background art disclosed in this section legally constitutes prior art.

Dendritic cells (DC) represent a family of professional antigen presenting cells that are found in virtually all tissues in the body. DCs play crucial roles in the induction of both innate and adaptive immune responses against infectious microorganisms, cancer cells, autoimmune diseases, graft rejection, graft versus host rejection disease, and other potentially harmful antigens. DCs in the steady state play equally important roles in the maintenance of immunological tolerance against self-antigens and harmless environmental antigens.

Several phenotypically distinct DC subsets have been reported in the literature, including monocyte-derived DCs, myeloid DCs, plasmacytoid DCs, and lymphoid DCs (Shortman and Liu, 2002) (Villadangos and Schnorrer, 2007). It still remains somewhat unclear whether these DC subsets differ from each other in functional properties as well as developmental pathways. Indeed, several different hematopoietic populations have been shown to give rise to DCs in culture or tissues (Rossner *et al.,* 2005) (Naik *et al.,* 2007) (Randolph *et al.,* 1999) (Fogg *et al.,* 2006) (del Hoyo *et al.,* 2002) (Larregina *et al.,* 2001) (D'Amico and Wu, 2003) (Diao *et al.,* 2006) (Onai *et al.,* 2007) (O'Keeffe *et al.,* 2003) (Naik *et al.,* 2006) (Ginhoux *et al.,* 2006) (Zuniga *et al.,* 2004) (Bruno *et al.,* 2001) (Wang *et al.,* 2002) (Mende *et al.,* 2006) (Diao *et al.,* 2004) (Karsunky *et al.,* 2003) (Iijima *et al.,* 2007) (Welner *et al.,* 2007) (Wu and Liu, 2007) (Wu and Dakic, 2004) (Shortman and Naik, 2007) (Naik, 2008) (Dakic and Wu, 2003)).
WO2004/065408 relates to an isolated myeloid cell and progenitors and progeny thereof, having a phenotype of CD11b+/CD11c^{-low}/MHC Class^{II-}.
Morandi B et al. "Distinctive lack of CD48 expression in subsets of human dendritic cells tunes NK cell activation." - the Journal of Immunology, Sep 15, 2005, 145(6):3690-7, relates to a study conducted to analysis the expression of CD48 markers in different human DC subsets, and it was concluded that CD48 is not expressed in DCs derived from monocytes, but is partially expressed in DCs derived from CD34+ hemopoietic cell percursor.

Although recent studies have identified several progenitor populations for DC, immediate precursors for DC remain to be determined. This invention addresses these issues.

### SUMMARY OF THE INVENTION

Accordingly the present invention provides an in vitro method for isolating a dendritic cell precursor cell line isolating dendritic cell precursor cells from a subject; placing in culture the isolated cells in a suitable culture medium containing an effective amount of GM-CSF for a time sufficient to generate a dendritic cell precursor cell line from the isolated cells, and multiplying the generated cell line by means of successive cell divisions, so as to obtain a dendritic cell line specific to the subject, wherein the isolated cells have a CD48 negative/MHC class I-negative phenotype. The preferred features are defined in dependent claims 2 to 4. Disclosed herein is an isolated dendritic cell precursor population (DC.com) identified by using surface markers CD48 and Gr-1.

Disclosed herein is an isolated human dendritic cell precursor (DC.com), having a characteristic surface phenotype of CD48-/MHC I-/MHC II-/CD1a-/CD1d-/CD11c-.

In certain embodiments, when the DC.com population is cultured in the presence of GM-CSF, the DC.com population differentiates into dendritic cells (DCs) showing characteristic dendritic morphology.

Disclosed herein is an isolated dendritic cell precursor population (DC.com), having a characteristic surface phenotype of CD11b+/CD11c-/Ly6G+/CD48-/MHC I-/MHC II-; wherein, when the DC.com population is cultured in the presence of GM-CSF, the DC.com population differentiates into DCs showing characteristic dendritic morphology. In certain embodiments, when the DC.com population is cultured in the presence of GM-CSF, the DC.com population differentiates into dendritic cells (DCs) showing characteristic dendritic morphology.

Disclosed herein is an isolated dendritic cell precursor population (DC.com) characterized by one or more of: - being distinguishable from CD11c+DCs based on the surface phenotype; wherein CD48, and MHC class I and class II molecules are undetectable in the DC.com population, - being distinguishable from other dendritic cell precursors based on surface phenotype; wherein Ly6G is detectable in the DC.com population;- showing no detectable antigen presenting capacity, as measured by the ability to present OVA protein and peptide to CD8 and CD4 T cells isolated from OT-I and OT-II T cell receptor transgenic mice; - having morphology characterized by lobulated nuclei and inclusion of modest numbers of cytoplasmic granules; - being homogeneous in terms of surface phenotype, cell size, granularity, and morphology; - having relatively limited mitotic potentials as compared to a CD11b+/DsRed-/CD11c- population in BM culture; - resembling a neutrophil progenitor "band cell" population; and - having the ability to differentiate into a DC subset (gr-DC) that is distinct from previously identified DC subpopulations.

Disclosed herein is an isolated dendritic cell population derived from DC.com, comprising granulocyte-derived DCs (gr-DCs).

Disclosed herein is the gr-DC cell population is identified by screening for expression of CD11c, MHC II, CD86 and DEC205.

The gr-DC cell population disclosed herein is capable of presenting antigens in *in vitro* or *ex vivo* systems so as to induce an immune response.

The antigens disclosed herein are one or more of: exogenous antigens, endogenous antigens or autoantigens.

In certain embodiments, the antigens are capable of presenting microbial antigens in *in vitro* or *ex vivo* systems so as to induce an immune response against infectious pathogens.

In certain embodiments, the antigens are capable of presenting tumor antigens in *in vitro* or *ex vivo* systems so as to induce an immune response against tumor cells.

In certain embodiments, the antigens are capable of presenting viral antigens in *in vitro* or *ex vivo* systems so as to induce an immune response against viral pathogens.

In certain embodiments, the antigens are capable of presenting non-microbial exogenous antigens in *in vitro* or *ex vivo* systems so as to induce an immune response.

In certain embodiments, the gr-DCs present OVA antigens to both CD4 and CD8 T cells.

In certain embodiments, the gr-DCs present surface expression of Ly6G.

Disclosed herein is a method for obtaining activated dendritic cells, comprising: providing a population of cells comprising at least one of DC.com and gr-DC, and
activating at least some of the cells in the population so as to trigger maturation of at least some of the cells.

In certain embodiments, at least one DC.com or gr-DC cell is activated with one or more of: at least one virus or derivatives thereof; at least one bacterium or derivatives thereof; at least one parasite; at least one fungus or derivatives thereof; at least one cytokine or at least one ligand.

In certain embodiments, the method is carried out on any type of biological sample comprising T lymphocytes. In certain embodiments, the sample is blood. In certain embodiments, the sample is autologous blood. In certain embodiments, the dendritic cells are human.

Disclosed herein is a method for activating T lymphocytes, comprising: providing a population of cells comprising at least one of DC.com and gr-DC; activating at least some of the cells in the population so as to trigger maturation of at least some of the cells; and, bringing T lymphocytes into contact with the dendritic cells.

Disclosed herein is a method for identifying compounds that activate dendritic cells, comprising the step of bringing the compound into contact with a population of cells comprising at least one of DC.com and gr-DC, and detecting the activation of the cells.

Disclosed herein is an in vitro method for isolating a dendritic cell line, comprising: isolating dendritic cell precursors from a subject; placing in culture the isolated cells in a suitable culture medium containing an effective amount of GM-CSF; inducing differentiation of the cells to granulocyte derived - dendritic cells (gr-DC); and multiplying the cells by means of successive cell divisions, so as to obtain a dendritic cell line specific to the subject. In certain embodiments, the cells are isolated after at least 20, 30, 40, 50, 60, 70, 80, 90, and preferably at least 100, cell divisions. In certain embodiments, the method further includes cloning the dendritic cell line obtained so as to obtain various dendritic cell lines or "clones", wherein the "cloning of a line" denotes the individualization of cells of this line, and a collection of genetically identical cells obtained from a single cell. In certain embodiments, the method further includes selecting one of the various dendritic cell lines or clones, so as to identify at least one clone having a phenotype of interest. In certain embodiments, the selected cells have a CD48-negative/MHC class I-negative phenotype.

In another aspect, there is provided herein an isolated cell line obtained according the methods described herein.

The present invention provides an *in vitro* method for isolating a dendritic cell precursor (DC.com) line, comprising: isolating dendritic cell precursors from a subject; placing in culture the isolated cells in a suitable culture medium containing an effective amount of GM-CSF, generating a DC precursor cell precursor (DC.com) line from the isolated cells, and multiplying the cells by means of successive cell divisions, so as to obtain a dendritic cell line specific to the subject. In certain embodiments, the cells are isolated after at least 20, 30, 40, 50, 60, 70, 80, 90, and preferably at least 100, cell divisions. In certain embodiments, the method further includes cloning the dendritic cell line obtained so as to obtain various dendritic cell lines or "clones", wherein the "cloning of a line" denotes the individualization of cells of this line, and a collection of genetically identical cells obtained from a single cell. In certain embodiments, the method further includes selecting one of the various dendritic cell lines or clones, so as to identify at least one clone having a phenotype of interest. The selected cells have a CD48-negative/MHC class I-negative phenotype.

In another aspect, there is provided herein a method for identifying compounds that activate dendritic cells, comprising: bringing a compound into contact with the DC.com or gr-DC cell line, and detecting the activation of the cell line.

Disclosed herein is a use of the dendritic cell precursor line DC.com and/or the dendritic cell line gr-DC, or functional derivatives thereof, in the treatment of at least one type of pathology associated with infectious or microbial agents (bacteria, viruses, parasites, fungi), cancers, graft v. host diseases, allergies and autoimmune diseases.

Disclosed herein is a use of the dendritic cell precursor line DC.com and/or the dendritic cell line gr-DC, in antitumor immunotherapy and cell therapy where the dendritic cell lines DC.com and/or gr-DC, or functional derivatives thereof, are immunotherapy agents.

Disclosed herein is a use of the dendritic cell precursor line DC.com and/or the dendritic cell line gr-DC, or functional derivatives thereof, for producing a pharmaceutical composition that promotes an antitumor immune response for the treatment of cancers.

Disclosed herein is a use of the dendritic cell precursor line DC.com and/or the dendritic cell line gr-DC, or functional derivatives thereof, for producing a pharmaceutical composition that promotes an antimicrobial response for the treatment of infectious diseases.

Disclosed herein is a use of the dendritic cell precursor line DC.com and/or the dendritic cell line gr-DC, or functional derivatives thereof, for producing a pharmaceutical composition that promotes an antiviral response for the treatment of viral diseases.

Disclosed herein is a use of the dendritic cell precursor line DC.com and/or the dendritic cell line gr-DC, or functional derivatives thereof, for producing a pharmaceutical composition that promotes a response for the treatment of non-microbial exogenous autoimmune diseases.

Disclosed herein is a use of the dendritic cell precursor line DC.com and/or the dendritic cell line gr-DC, or functional derivatives thereof, for producing a pharmaceutical composition that promotes a response for the treatment of graft v. host diseases.

Disclosed herein is a use of the dendritic cell precursor line DC.com and/or the dendritic cell line gr-DC, or functional derivatives thereof, for testing the number and/or function of gr-DCs in a disease in which DCs are known to play protective or pathogenic roles.

Disclosed herein is a use of the dendritic cell precursor line DC.com and/or the dendritic cell line gr-DC, or functional derivatives thereof, for customized cell-based therapies designed to selectively induce desired forms of immune responses.

Disclosed herein is a use of the dendritic cell precursor line DC.com and/or the dendritic cell line gr-DC, or functional derivatives thereof, for developing a new class of vaccine adjuvants and formulations that are designed to activate DC.com and/or gr-DCs.

Disclosed herein is a use of the dendritic cell precursor line DC.com and/or the dendritic cell line gr-DC, or functional derivatives thereof, for screening for small chemical compounds that selectively enhance or inhibit the function of DC.com and/or gr-DCs.

Disclosed herein is a method for identification of a dendritic cell-committed progenitor population (DC.com), comprising the step of determining whether the population exhibits detectable antigen presenting capacity; shows myeloid suppressor cell function; is efficient in its ability to uptake exogenous molecules; acquires the expression of one or more of CD48, MHC I, MHC II, CD1a, CD1d, CD11c.

Disclosed herein is a method for purification of a DC.com population, comprising the step of screening a dendritic cell precursor population for at least CD48 and MHC class I markers.

Disclosed herein is a method for purification of a gr-DC population, comprising the step of screening a dendritic cell population for at least C11c, MHC class II, CD86 and DEC205 markers.

Disclosed herein is a method for purification of a band cell population, comprising the step of screening a dendritic cell precursor population for at least CD48 and MHC class I markers.

Disclosed herein is a method for expansion of a dendritic cell-committed progenitor population (DC.com), comprising the step of culturing a population of isolated DC.com cells in GM-CSF, and optionally, adding bone marrow (BM) feeder cells to the culture.

Disclosed herein is a method for inducing precursor dendritic cells (DC.com) to exhibit the phenotype of granulocyte dendritic cells (gr-DC), comprising: i) purifying a CD48-negative/Gr-1-high DC.com population from bone marrow (BM) cultures of C57BL/6 mice (which are CD45.2+), ii) co-culturing the population from step i) with freshly isolated BM cells from B6/SJL mice (which are CD45.1+) in the presence of GM-CSF; iii) distinguishing cells derived from the DC.com population distinguished by differential staining with anti-CD45.2 and anti-CD45.1 antibodies; and iv) analyzing the CD45.2+ cells for surface expression of one or more indicated markers. In certain embodiments, the gr-DC.com cells acquire expression of CD11c, MHC class II, DEC205 and maintain surface expression of Ly6G. In certain embodiments, the cells are purified by fluorescence-activated cell sorting FACS.

Disclosed herein is a method for producing cells having the phenotype of granulocyte dendritic cells (gr-DC) from mammalian precursor dendritic cells, comprising: i) providing a cell fraction comprising precursor dendritic cells from a mammalian blood sample; ii) isolating at least one subset of the cell fraction of step (i) by: co-culturing the population from step i) with freshly isolated BM cells which are CD45.1+ in the presence of GM-CSF; distinguishing cells derived from the DC.com population distinguished by differential staining with anti-CD45.2 and anti-CD45.1 antibodies; and analyzing the CD45.2+ cells for surface expression of one or more indicated markers; and iii) collecting the contacted cells of step (ii).

Disclosed herein is a method for ameliorating an inflammatory condition in a subject in need thereof, comprising administering an effective amount of a DC.com derived composition.

Disclosed herein is a method comprising contacting CD48-negative/Gr-1high - early dendritic cells with an effective amount of GM-CSF, thereby inducing differentiation of the cells to granulocyte derived - dendritic cells (gr-DC), wherein Ly6G is expressed on the surface of the gr-DCs, and wherein the gr-DCs present at least one foreign antigen to CD8 and CD4 T cells. In certain embodiments, the effective amount is at least 10 ng/ml and said contacting is for at least 1 day.

Disclosed herein is a method of identifying an effector of dendritic cell (DC) interaction with T cells comprising: i) admixing a DC.com population with a T cell and a test agent, and ii) determining if the candidate substance alters the interaction of DC.com with the T cell, wherein a test agent that alters the interaction of DC.com with the T cell is an effector of dendritic cell interaction. In certain embodiments, the DC.com composition comprises purified DC.com linked to a detectable label.

Disclosed herein is a method of identifying an effector of dendritic cell (DC) interaction with T cells comprising: i) admixing a gr-DC population with a T cell and a test agent, and ii) determining if the candidate substance alters the interaction of gr-DC with the T cell, wherein a test agent that alters the interaction of gr-DC with the T cell is an effector of dendritic cell interaction. In certain embodiments, the gr-DC composition comprises purified gr-DC linked to a detectable label.

Disclosed herein is a method of screening a test agent, wherein a test agent that affects the interaction of a DC.com composition with T cells is identified by an alteration in DC.com composition binding to the T cell.

Disclosed herein is a method of screening a test agent, wherein a test agent that affects the interaction of a DC.com composition with T cells is identified by an alteration in DC.com-mediated activation of the T cell.

Disclosed herein is a method of screening a test agent, wherein a test agent that affects the interaction of a gr-DC composition with T cells is identified by an alteration in gr-DC composition binding to the T cell.

Disclosed herein is a method of screening a test agent, wherein a test agent that affects the interaction of a gr-DC composition with T cells is identified by an alteration in gr-DC-mediated activation of the T cell.

Disclosed herein is a pharmaceutical composition, comprising a population of cells comprising at least one of DC.com and gr-DC, and functional variants thereof.

In certain embodiments, the pharmaceutical composition is useful for treatment of an infectious disease, a cancer-related disease, an inflammatory disorder or disease, an immune-related disorder or disease, an autoimmune disease, host versus graft rejection disease, a hypersensitivity reaction, or allograft rejection.

Disclosed herein is a method of diagnosing whether a subject has, or is at risk for developing an, determining a prognosis, and/or treating one or more of the following disorders: an infectious disease, a cancer-related disease, an inflammatory disease, an immune-related disease, an autoimmune disease, host versus graft rejection disease, a hypersensitivity reaction, or allograft rejection, in the subject, comprising: measuring the level of at least one marker derived from one or more of DC.com or gr-DC in a test sample from the subject, wherein an alteration in the level of the marker in the test sample, relative to the level of a corresponding marker in a control sample, is indicative of the subject either having, or being at risk for developing, the disorder. In certain embodiments, the level of the at least one marker in the test sample is less than the level of the corresponding marker in the control sample. In certain embodiments, the level of the at least one marker in the test sample is greater than the level of the corresponding marker in the control sample. In certain embodiments, the at least one marker is differentially expressed between normal tissue and/or cells and affected tissue and/or cells. In certain embodiments, the sample comprises a blood sample. In certain embodiments, the sample comprises one or more of serum or plasma blood samples.

Disclosed herein is a marker comprising at least one marker is differentially expressed between normal tissue and/or cells and affected tissue and/or cells, and is derived from one or more of DC.com and gr-DC cell populations.

Disclosed herein is a method for determining the prognosis of a subject with one or more of the following disorders: an infectious disease, a cancer-related disease, an inflammatory disease, an immune-related disease, an autoimmune disease, host versus graft rejection disease, a hypersensitivity reaction, or allograft rejection, comprising the step of measuring the level of at least one marker in a test sample from the subject, wherein the marker is derived from one or more of DC.com and gr-DC; and wherein: i) the marker is associated with an adverse prognosis; and ii) an alteration in the level of the at least one marker in the test sample, relative to the level of a corresponding marker in a control sample, is indicative of an adverse prognosis.

Disclosed herein is a method of treating one or more of the following disorders: an infectious disease, a cancer-related disease, an inflammatory disease, an immune-related disease, an autoimmune disease, host versus graft rejection disease, a hypersensitivity reaction, or allograft rejection, in which at least one marker is down-regulated or up-regulated in the affected cells of the subject relative to control cells, wherein the marker is derived from one or more of DC.com and gr-DC, the method comprising: i) when the at least one marker is down-regulated in the affected cells, administering to the subject an effective amount of at least one isolated marker, or an isolated variant or biologically-active fragment thereof, such that proliferation of affected cells in the subject is inhibited; or ii) when the at least one marker is up-regulated in the affected cells, administering to the subject an effective amount of at least one compound for inhibiting expression of the at least one marker, such that proliferation of affected cells in the subject is inhibited.

Disclosed herein is a method of treating one or more of the following disorders: an infectious disease, a cancer-related disease, an inflammatory disease, an immune-related disease, an autoimmune disease, host versus graft rejection disease, a hypersensitivity reaction, or allograft rejection, in which at least one marker is down-regulated or up-regulated in the affected cells of the subject relative to control cells, wherein the marker is derived from one or more of DC.com and gr-DC, the method comprising: (1) determining the amount of at least one marker in affected cells in the subject, relative to control cells; and (2) altering the amount of marker expressed in the affected cells by: (i) administering to the subject an effective amount of at least one isolated marker, if the amount of the marker expressed in the affected cells is less than the amount of the marker expressed in control cells; or (ii) administering to the subject an effective amount of at least one compound for inhibiting expression of the at least one marker, if the amount of the marker expressed in the affected cells is greater than the amount of the marker expressed in control cells.

Disclosed herein is a pharmaceutical composition comprising at least one isolated marker, wherein the marker is derived from one or more of DC.com and gr-DC; and, a pharmaceutically-acceptable carrier. In certain embodiments, the at least one isolated marker corresponds to a marker that is down-regulated in affected cells relative to control cells.

Disclosed herein is a method of identifying an anti-inflammatory agent, comprising providing a test agent to a cell and measuring the level of at least one marker associated with decreased expression levels in affected cells, wherein an increase in the level of the marker in the affected cell, relative to a control cell, is indicative of the test agent being an anti-inflammatory cancer agent; and wherein the marker is derived from one or more of DC.com and gr-DC.

Disclosed herein is a method of identifying an anti-inflammatory agent, comprising providing a test agent to a cell and measuring the level of at least one marker associated with increased expression levels in affected cells, wherein a decrease in the level of the marker in the cell, relative to a control cell, is indicative of the test agent being an anti-inflammatory agent, and wherein the marker is derived from one or more of DC.com and gr-DC.

Disclosed herein is a method of assessing the effectiveness of a therapy to prevent, diagnose and/or treat an inflammatory disorder, comprising: i) subjecting an animal to a therapy whose effectiveness is being assessed, and ii) determining the level of effectiveness of the treatment being tested in treating or preventing the disorder, by evaluating at least one marker, wherein the marker is derived from one or more of DC.com and gr-DC.

In certain embodiments, candidate therapeutic agent comprises one or more of: pharmaceutical compositions, nutraceutical compositions, and homeopathic compositions.

In certain embodiments, the therapy being assessed is for use in a human subject.

Disclosed herein is an article of manufacture comprising: at least one capture reagent that binds to a marker for an inflammatory disorder comprising at least one marker derived from one or more of DC.com and gr-DC.

Disclosed herein is a kit for screening for a candidate compound for a therapeutic agent to treat an inflammatory disorder, wherein the kit comprises: one or more reagents of at least one marker derived from one or more of DC.com and gr-DC; and, a cell expressing at least one marker.

In certain embodiments, the presence of the marker is detected using a reagent comprising an antibody or an antibody fragment which specifically binds with at least one marker.

Disclosed herein is a use of an agent that interferes with an inflammatory disorder or associated disease response signaling pathway, for the manufacture of a medicament for treating, preventing, reversing or limiting the severity of the disease complication in an individual, wherein the agent comprises at least one marker derived from one or more of DC.com and gr-DC.

Disclosed herein is a method of treating, preventing, reversing or limiting the severity of an inflammatory disorder or associated disease complication in a subject in need thereof, comprising: administering to the subject an agent that interferes with at least an inflammatory response cascade, wherein the agent comprises at least one marker derived from one or more of DC.com and gr-DC.

Disclosed herein is a use of an agent that interferes with at least an inflammatory associated disease response cascade, for the manufacture of a medicament for treating, preventing, reversing or limiting the severity of an inflammatory -related disease complication in a subject, wherein the agent comprises at least one marker derived from one or more of DC.com and gr-DC.

Disclosed herein is a composition comprising an antisense inhibitor of one or more of markers derived from one or more of DC.com and gr-DC.

Disclosed herein is a method of treating a subject in need thereof, comprising administering to a subject a therapeutically effective amount of the composition described herein. In certain embodiments, the composition is administered prophylactically. In certain embodiments, administration of the composition delays the onset of one or more symptoms of the disorder. In certain embodiments, administration of the composition inhibits development of an inflammatory disorder. In certain embodiments, administration of the composition inhibits infection.

Disclosed herein is a method for detecting the presence of a disorder in a biological sample, the method comprising: i) exposing the biological sample suspected of containing the disorder to a marker therefor; and ii) detecting the presence or absence of the marker, if any, in the sample; wherein the marker is derived from one or more of DC.com and gr-DC. In certain embodiments, the marker includes a detectable label.

In certain embodiments, the method further includes comparing the amount of the marker in the biological sample from the subject to an amount of the marker in a corresponding biological sample from a normal subject.

In certain embodiments, the method further includes collecting a plurality of biological samples from a subject at different time points and comparing the amount of the marker in each biological sample to determine if the amount of the marker is increasing or decreasing in the subject over time.

Disclosed herein is a method for treating an inflammatory disorder in a subject, the method comprising: administering to the subject in need thereof a therapeutically effective amount of an inflammatory receptor agonist derived from one or more of DC.com or gr-DC. In certain embodiments, the receptor agonist is an antisense inhibitor of one or more of marker derived from DC.com or gr-DC.

Disclosed herein is an in vitro method to identify effective therapeutic agents or combinations of therapeutic agents to induce the differentiation of cells affected by an inflammatory disorder, the method comprising the stages of: i) culturing of affected cells, ii) adding at least one compound to the culture medium of the step i), iii) analyzing the evolution of the level of expression of at least one marker between stages (i) and (ii), and iv) identifying compounds or combinations of compounds inducing a change in the level of expression of the marker between stages (i) and (ii). In certain embodiments, stage (iii) includes the analysis of the level of expression of at least one marker. In certain embodiments, stage (iv) includes the identification of the compounds or combinations of compounds modulating the level of expression of at least one marker. In certain embodiments, stage (iv) includes the identification of compounds or combinations of compounds reducing the level of expression of at least one marker.

In certain embodiments, the compound is a therapeutic agent for the treatment of an inflammatory disorder.

Disclosed herein is a method for classifying affected tissue and/or cells from a subject having an inflammatory disorder, comprising: measuring the expression of one or more markers derived from one or more of DC.com and gr-DC in a test cell population, wherein at least one cell in the test cell population is capable of expressing one or more such markers; comparing the expression of the marker(s) to the expression of the marker(s) in a reference cell population comprising at least one cell for which a classification is known; and identifying a difference, if present, in expression levels of one or more markers selected from the group consisting, in the test cell population and reference cell population, thereby classifying the inflammatory disorder in the subject.

In certain embodiments, a difference in the expression of the marker(s) in the test cell population as compared to the reference cell population indicates that the test cell population has a different classification as the cells from the reference cell population.

In certain embodiments, a similar expression pattern of the marker(s) in the test cell population as compared to the reference cell population indicates that the test cell population has the same classification as the cells from the reference cell population.

In certain embodiments, the reference cell population is a plurality of cells or a database.

In certain embodiments, the reference cell population is selected from the group consisting of: a reference cell population classified as a cell population from normal tissue, and a reference cell population classified as a cell population from affected tissue.

Disclosed herein is a use of a defined surface phenotype to identify and purify DC.com population.

Disclosed herein is a use of a defined surface phenotype to identify and purify a band cell population.

Disclosed herein is a use of a defined surface phenotype to identify and purify a gr-DC population.

Disclosed herein is a use of GM-CSF and optionally, at least one other cytokine, to expand DC.com populations.

Disclosed herein is a use of GM-CSF and optionally at least one other cytokine, to promote differentiation of DC.com to gr-DCs.

Disclosed herein is a method to identify DC.com in tissue in a subject, comprising the step of screening for expression of one or more of CD48, MHC I, MHC II, CD1a, CD 1d, and CD11c.

Disclosed herein is a method to identify band cells in tissue in a subject, comprising the step of screening for expression of Ly6G.

Disclosed herein is a method to identify gr-DCs in tissue in a subject, comprising the step of screening for expression of one or more of CD11c, MHC II, CD86 and DEC205.

Disclosed herein is a method to identify compounds that promote or inhibit DC.com expansion, comprising the step of screening for expression of one or more of markers of DC.com.

Disclosed herein is a method to identify compounds that promote or inhibit DC.com differentiation to gr-DCs comprising the step of screening for expression of one or more of markers of DC.com and/or gr-DC.

Disclosed herein is a method to test gene expression profiles and function of DC.com, comprising the step of screening for expression of one or more of markers of DC.com.

Disclosed herein is a method to test gene expression profiles and function of gr-DCs, comprising the step of screening for expression of one or more of markers of gr-DC.

Disclosed herein is a vaccine comprising at least one compound that stimulates DC.com in a subject in need thereof.

Disclosed herein is a vaccine comprising at least one compound that stimulates gr-DC in a subject in need thereof.

Disclosed herein is an immunostimulatory therapeutic composition comprising at least one compound that stimulates DC.com in a subject in need thereof.

Disclosed herein is an immunostimulatory therapeutic composition comprising at least one compound that stimulates gr-DC in a subject in need thereof.

Disclosed herein is a use of phenotype, gene profile, or function of DC.com as markers.

Disclosed herein is a use of phenotype, gene profile, or function of gr-DCs as markers.

Disclosed herein is a use of the defined surface phenotype of DC.com and/or gr-DCs for targeted gene delivery.

Disclosed herein is a use of the defined surface phenotype of DC.com and/or gr-DCs for targeted drug delivery.

Disclosed herein is a use of the defined surface the DC.com as an immediate precursor for DC.

Disclosed herein is a use of the pIL1-DsRed transgenic mouse line as a model for studying the DC.com population.

Disclosed herein is a use of the pIL1-DsRed transgenic mouse line as a tool for discovery of new drugs.

Other systems, methods, features, and advantages of the present invention will be or will become apparent to one with skill in the art upon examination of the following drawings and detailed description. It is intended that all such additional systems, methods, features, and advantages be included within this description, be within the scope of the present invention, and be protected by the accompanying claims.

### BRIEF DESCRIPTION OF THE DRAWINGS

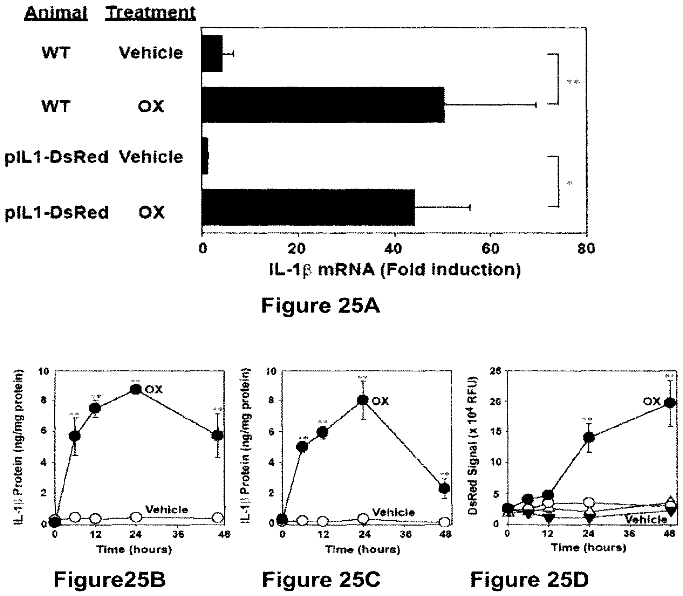

The patent or application file may contain one or more drawings executed in color and/or one or more photographs. Copies of this patent or patent application publication with color drawing(s) and/or photograph(s) will be provided by the Patent Office upon request and payment of the necessary fee.

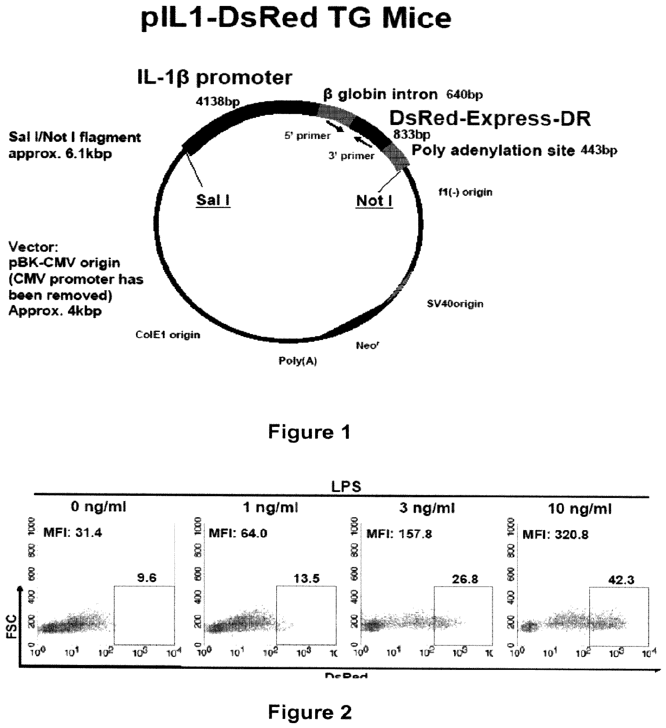

**Figure 1****.** Construct for pIL1-DsRed transgenic mouse line: This construct was used to generate the IL-β□ promoter-driven DsRed transgenic mouse strain.
**Figure 2****.** DsRed expression by DCs derived from pIL1-DsRed transgenic mice: CD11c+ DC cultures generated from the pIL1-DsRed transgenic mice were examined for DsRed expression 24 hours after stimulation with LPS at the indicated concentrations. Note that DCs express DsRed fluorescent signals upon stimulation with LPS.

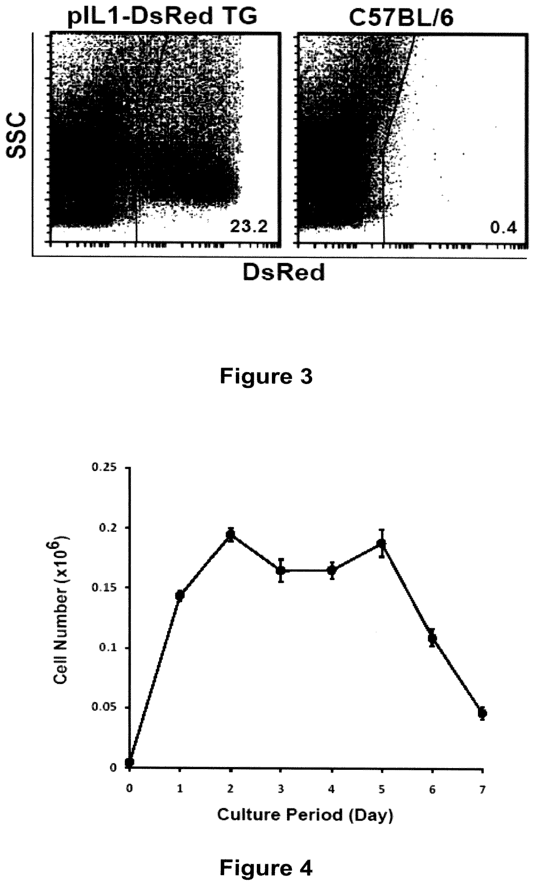

**Figure 3**. Detection of DsRed fluorescence signals in BM cell cultures from pIL1-DsRed transgenic mice: Bone marrow (BM) cells isolated from pIL1-DsRed transgenic or wild-type C57BL/6 mice were cultured for 2 days in the presence of 10 ng/ml GM-CSF and then examined for DsRed expression. Note that only the BM cultures generated from the pIL1-DsRed transgenic mice exhibit significant DsRed signals even in the absence of LPS stimulation.
**Figure 4****.** Kinetics for DsRed expression in BM culture: BM cells isolated from pIL1-DsRed transgenic were cultured for the indicated period in the presence of 10 ng/ml GM-CSF and then examined for DsRed expression. Data shown are the numbers of DsRed-positive cells per 10⁶ starting BM cells (means ± SD from triplicate cultures). Note almost complete absent DsRed+ cells in the starting BM cell populations and rapid expansion of DsRed+ cells in the subsequent cultures.

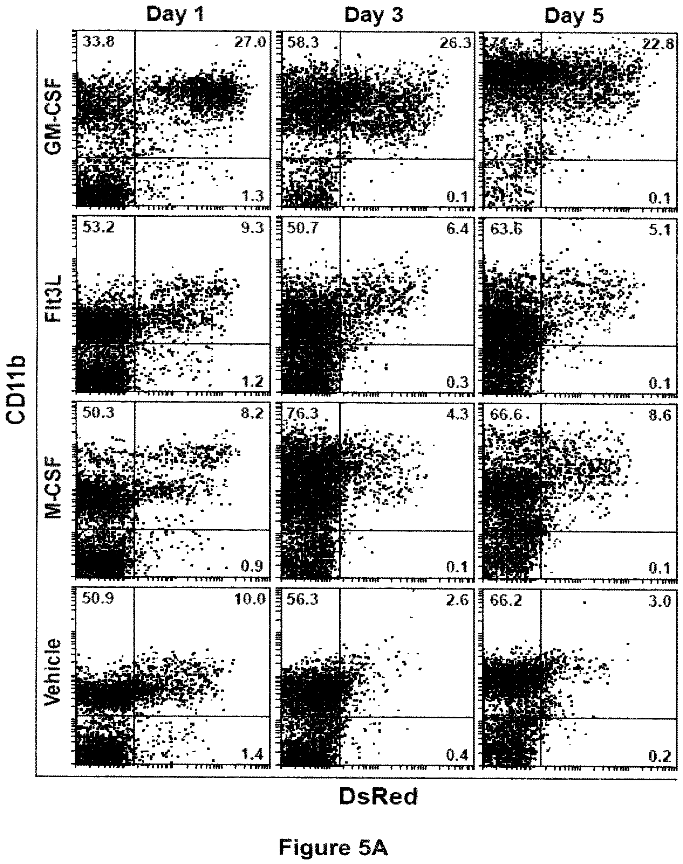

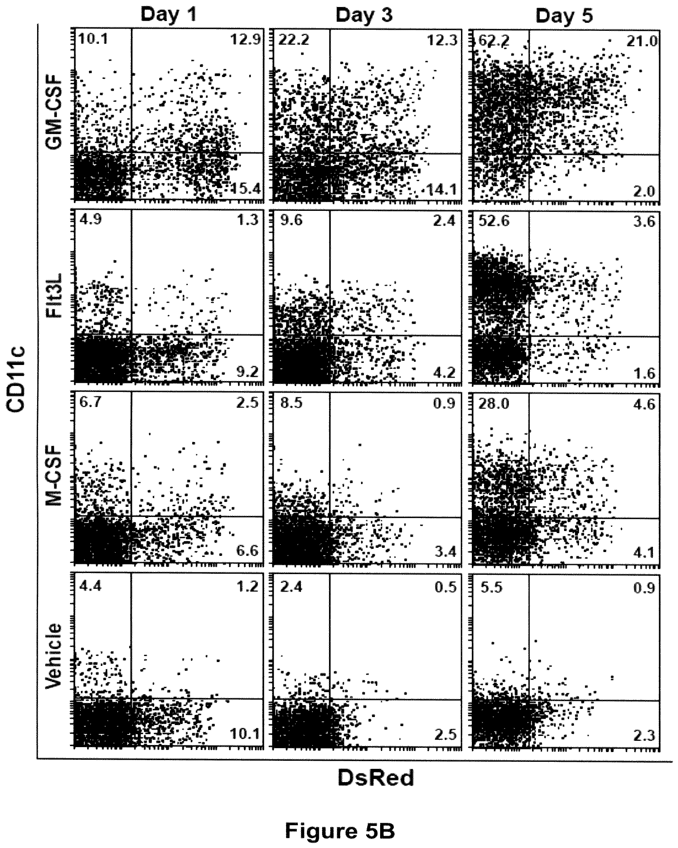

**Figures 5A**-**5B**. CD11b and CD11c expression by DsRed+ cells in BM culture: BM cells isolated from pIL1-DsRed transgenic were cultured for the indicated period in the presence or absence of 10 ng/ml GM-CSF, 200 ng/ml Flt3 ligand, or 10 ng/ml M-CSF and then examined for DsRed expression, as well as for surface expression of CD11b **(****Figure 5A****)** and CD11c **(****Figure 5B****).** Note that GM-CSF promotes the expansion of DsRed+/CD11b+ cells in the early phase of BM culture, as well as the increase in the numbers of DsRed+/CD11b+ and DsRed-/CD11c+ DCs in the late phase.

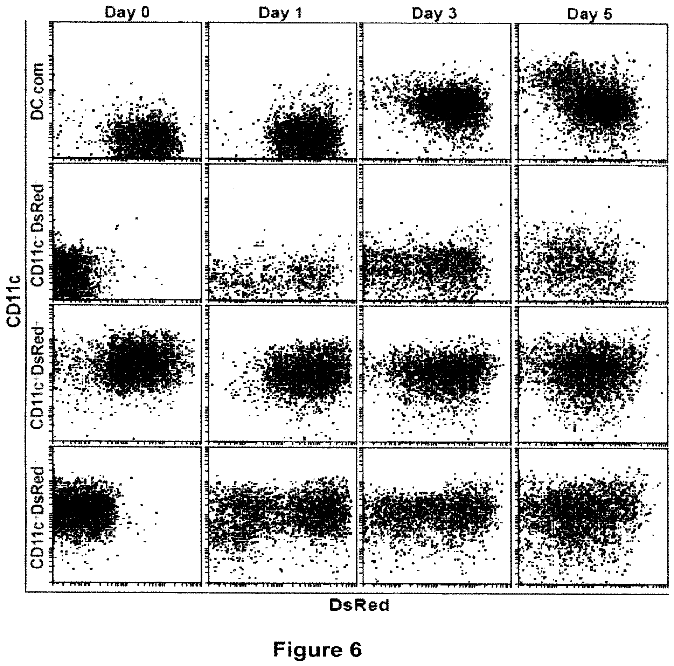

**Figure 6****.** Potential of DsRed+/CD11b+/CD11c- population to different into CD11c+ DCs: The CD11b+ cells in GM-CSF-supplemented BM cultures (day 3) of pIL1-DsRed transgenic mice were sorted into four fractions based on DsRed expression and surface expression of CD11c. Each fraction was placed back to culture in the presence of GM-CSF and then examined for DsRed and CD11c expression at the indicated time points. The profiles on day 0 represent the purity of individual sorted populations. Note that the DsRed+/CD11b+/CD11c- population (labeled as "DC.com") begin to express a DC marker CD11c when placed in culture with GM-CSF.

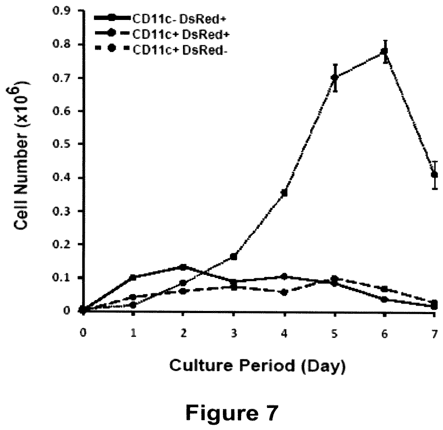

**Figure 7****.** Kinetics for the emergence of DC.com in BM culture: BM cells isolated from pIL1-DsRed transgenic were cultured for the indicated period in the presence of 10 ng/ml GM-CSF and then examined for the numbers of DC.com and DC populations. Data shown are the numbers of DsRed+/CD11c- DC.com cells (solid line), DsRed+/CD11c+ DC (broken line), and DsRed-/CD11c+ DC (dotted line) per 10⁶ starting BM cells. Note rapid and profound expansion of DsRed+/CD11c- cells within the first 24 hours in culture.

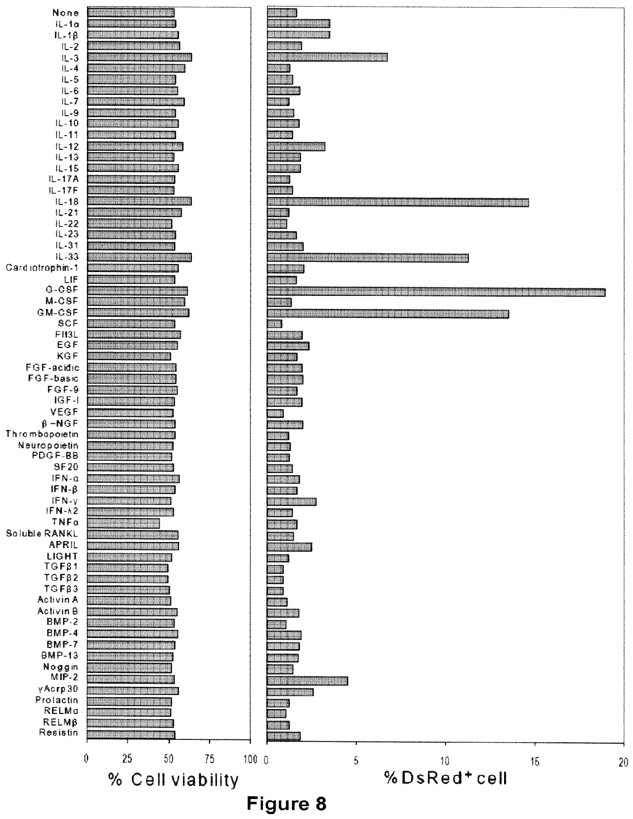

**Figure 8****.** Differential effects of cytokines on the expansion of DsRed+ cells in BM culture: BM cells isolated from pIL1-DsRed transgenic were cultured for 2 days in the presence of each cytokine (10 ng/ml) and then examined for cell viability and the % of cells expressing DsRed fluorescent signals. Note that only selected cytokines (including GM-CSF) promote the expansion of DsRed+ cells in BM culture.

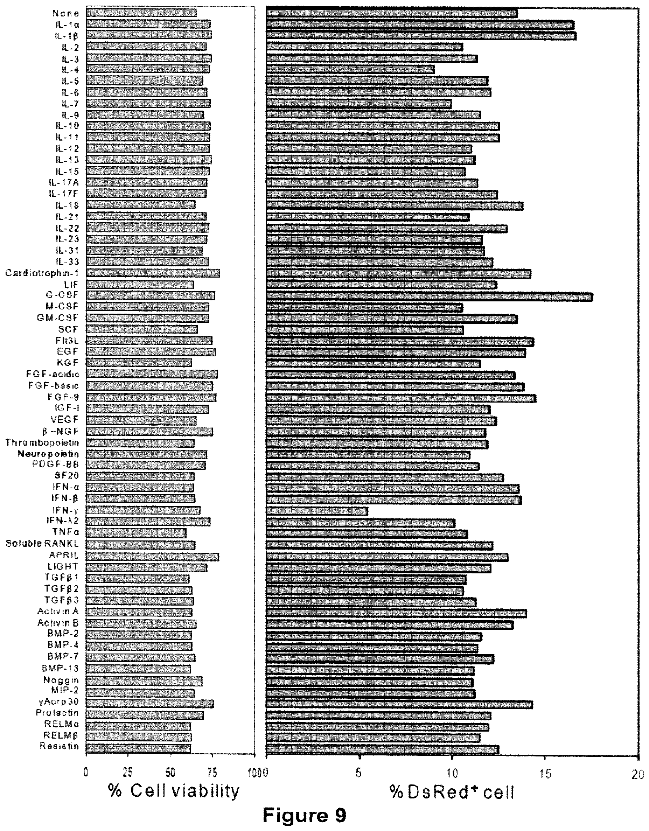

**Figure 9****.** Differential effects of cytokines on GM-CSF-dependent expansion of DsRed+ cells in BM culture: Each of the indicated cytokine was added at 10 ng/ml to the BM cell cultures from pIL1-DsRed transgenic mice supplemented with GM-CSF (10 ng/ml). Two days later, the samples were examined for cell viability and the % of cells expressing DsRed fluorescent signals. Note that only selected cytokines (including interferon-γ) inhibit GM-CSF-dependent expansion of DsRed+ cells in BM culture.

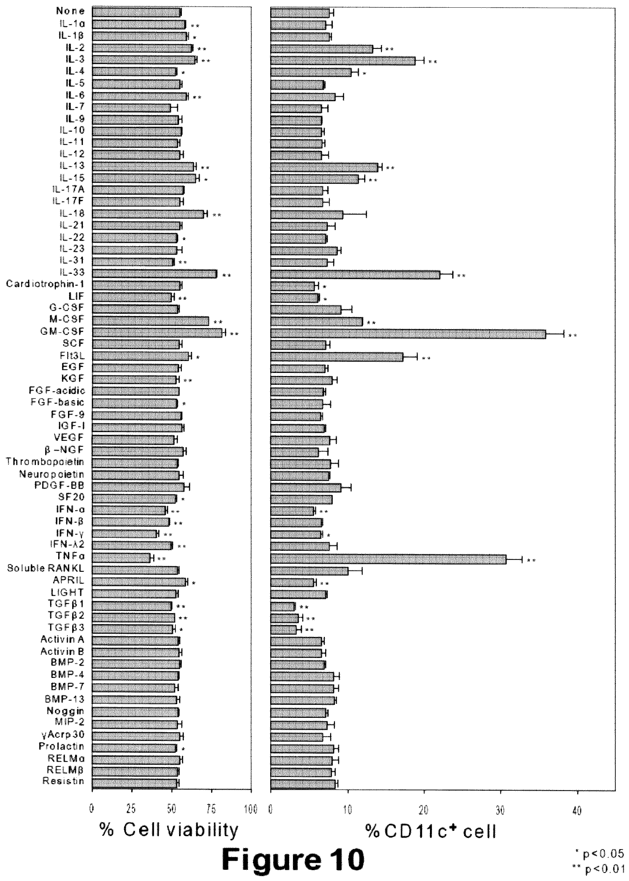

**Figure 10****.** Differential effects of cytokines on the expansion of CD11c+ DCs in BM culture: BM cells isolated from pIL1-DsRed transgenic were cultured for 2 days in the presence of each cytokine (10 ng/ml) and then examined for cell viability and the % of cells expressing a DC marker CD11c. Data shown are the means ± SD from triplicate cultures (*P<0.05, **P<0.01, ***P<0.001). Note that only selected cytokines (including GM-CSF) promote DC expansion in BM culture.

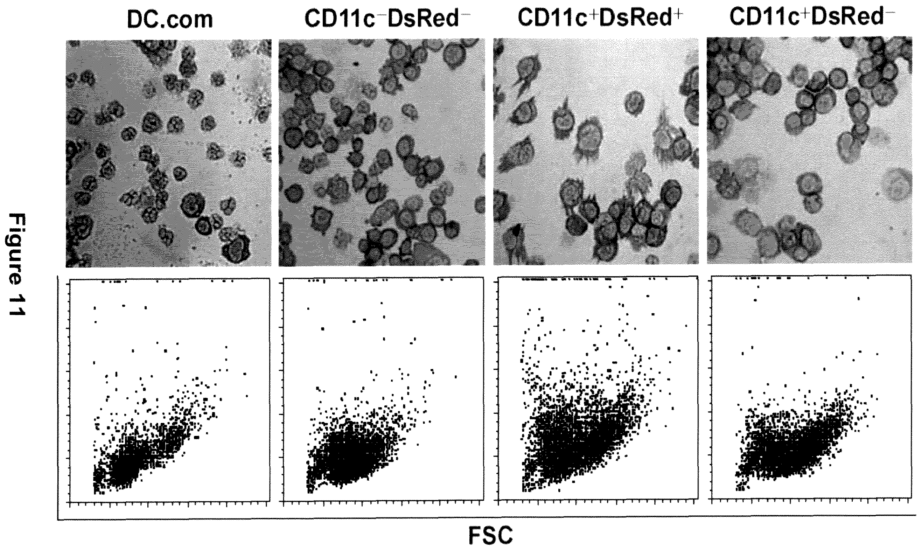

**Figure 11****.** Morphological characteristics of the DsRed+/CD11b+/CD11c-population: The CD11b+ cells in GM-CSF-supplemented BM cultures (day 3) of pIL1-DsRed transgenic mice were sorted into four fractions based on DsRed expression and surface expression of CD11c. Cytospin preparation of each fraction was examined for cellular morphology after Giemsa staining (top panels). Each fraction was also examined for cell size (FSC) and granularity (SSC) by flow cytometry. Note that the FACS-purified DsRed+/CD11b+/CD11c- cells (i.e., DC.com) uniformly exhibit a characteristic morphology, relatively small cell size (FSC), and limited granularity (SSC).

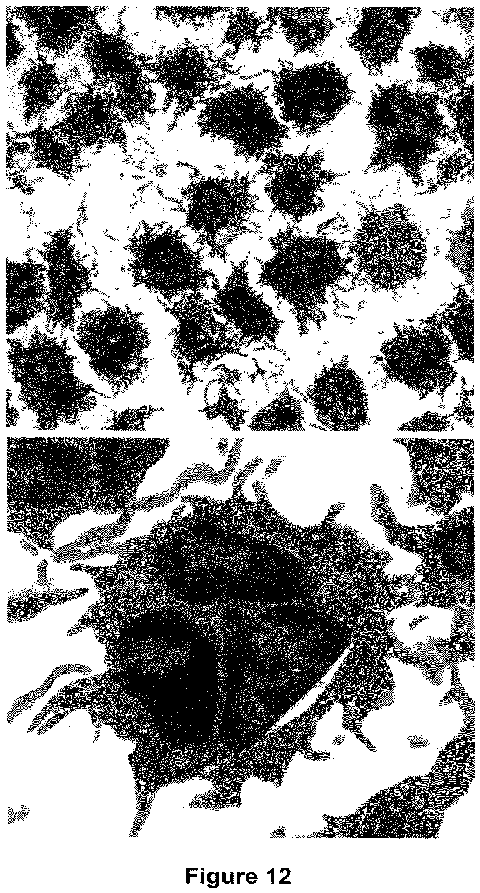

**Figure 12****.** Ultrastructural features of the DsRed+/CD11b+/CD11c- population: The CD11b+/DsRed+/CD11c- cells were FACS purified from GM-CSF-supplemented BM cultures (day 3) of pIL1-DsRed transgenic mice. The samples were then examined under electron microscopy. Note that the FACS-purified DsRed+/CD11b+/CD11c- cells (i.e., DC.com) uniformly exhibit short processes and lobulated nuclei.

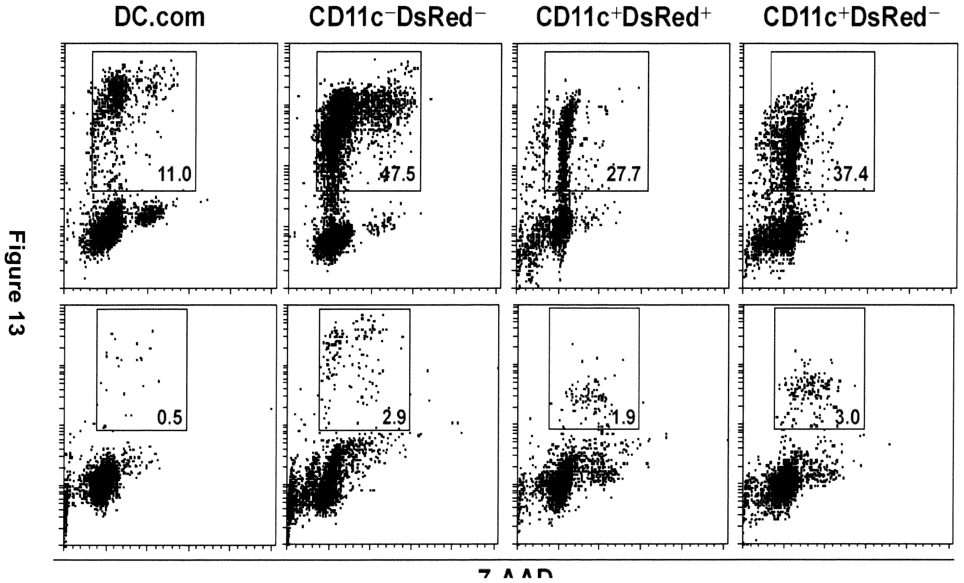

**Figure 13****.** Mitotic potentials of DC.com: The CD11b+ cells in GM-CSF-supplemented BM cultures (day 3) of pIL1-DsRed transgenic mice were sorted into four fractions based on DsRed expression and surface expression of CD11c after 18 hours labeling with BrdU (top panels). Alternatively, the sorted populations were cultured for additional 18 hours in the presence of BrdU (bottom panels). The samples were then examined for BrdU incorporation and DNA contents (by 7-AAD staining). Cells in the indicated squares represent those with high mitotic potentials. Note relatively limited mitotic potentials of DsRed+/CD11b+/CD11c- cells (i.e., DC.com).

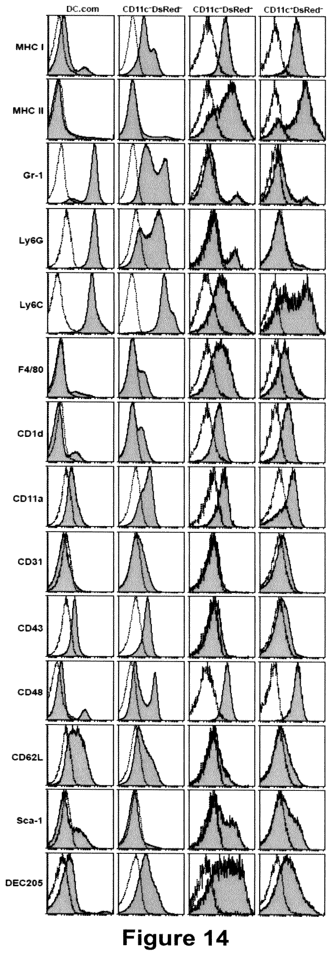

**Figure 14****.** Surface phenotype of DC.com: The DsRed+/CD11b+/CD11c- DC.com fraction and three other populations in GM-CSF-supplemented BM cultures (day 3) of pIL1-DsRed transgenic mice were examined for the surface expression of the indicated molecules. Staining profiles with isotype-matched control IgG are shown with solid lines. Note that the DC.com population can be defined by the characteristic surface phenotype, including MHC class I-negative, Gr-1-positive, Ly6G-positive, Ly6C-positive, and CD48-negative.

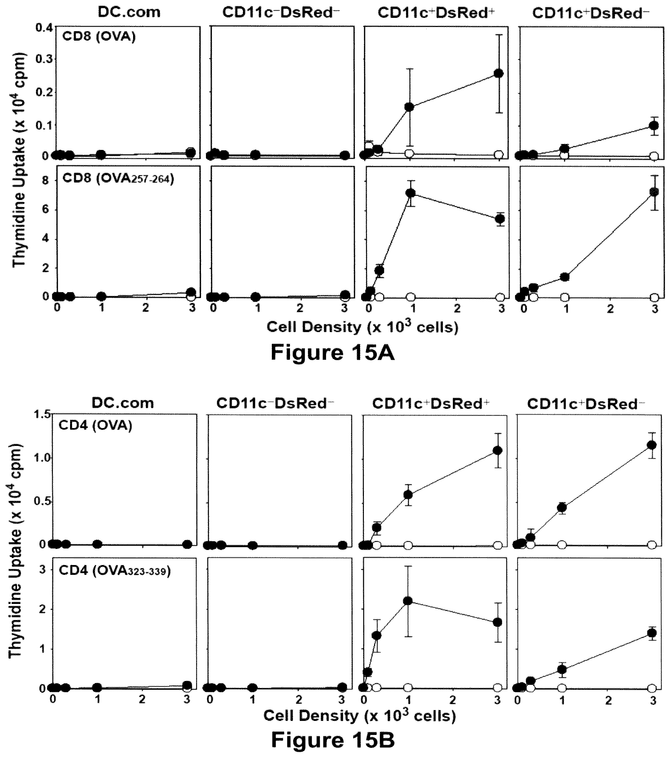

**Figures 15A-15B****.** Antigen presentation capacity of DC.com: The CD11b+ cells in GM-CSF-supplemented BM cultures (day 3) of pIL1-DsRed transgenic mice were sorted into four fractions based on DsRed expression and surface expression of CD11c. Each fraction was pulsed with OVA protein or OVA peptide and then co-cultured with CD8 T cells purified from the OT-I transgenic mice **(****Figure 15A****)** or CD4 T cells purified from the OT-II transgenic mice **(****Figure 15B****).** The numbers of added DC.com or other populations are plotted in the X-axis. Circles indicate control co-cultures in the absence of antigen pulsing. Data shown are 3H-thymidine uptake on day 4 (means ± SD from triplicate cultures). Note that the DC.com population exhibits no detectable antigen presenting capacity, whereas two DC populations (CD11c+/DsRed+ cells and CD11c+/DsRed- cells) efficiently present protein and peptide antigens to both CD8 and CD4 T cells.

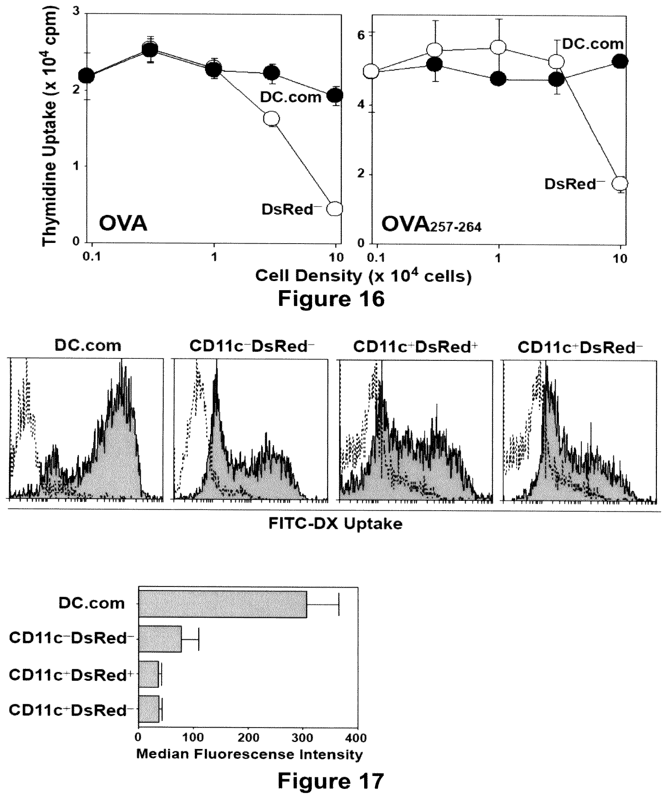

**Figure 16****.** Lack of myeloid-derived suppressor cell function in DC.com: The CD11b+/CD11c- cells in GM-CSF-supplemented BM cultures (day 3) of pIL1-DsRed transgenic mice were sorted into two fractions based on DsRed expression. The DsRed+ cells (DC.com) (filled circles) and the DsRed- cells (open circles) were co-cultured with spleen cells isolated from OT-1 transgenic mice in the presence of OVA protein (left panel) or OVA peptide (right panel). Data shown are 3H-thymidine uptake on day 3 (means ± SD from triplicate cultures). Note that the CD11b+/CD11c-/DsRed- cells inhibit antigen-specific T cell proliferation in a cell number-dependent manner, whereas the CD11b+/CD11c-/DsRed+ cells (DC.com) show no myeloid-derived suppressor cell function.
**Figure 17****.** Endocytotic potential of DC.com: The CD11b+ cells in GM-CSF-supplemented BM cultures (day 3) of pIL1-DsRed transgenic mice were sorted into four fractions based on DsRed expression and surface expression of CD11c. Each fraction was incubated with FITC-conjugated dextran for 10 min. Data shown are FACS profiles for FITC-dextran uptakes (top panels) and the means ± SD of the median fluorescence intensity from triplicate samples (bottom panels). Note that the DC.com population is much more efficient than other populations in their ability to uptake exogenous molecules.

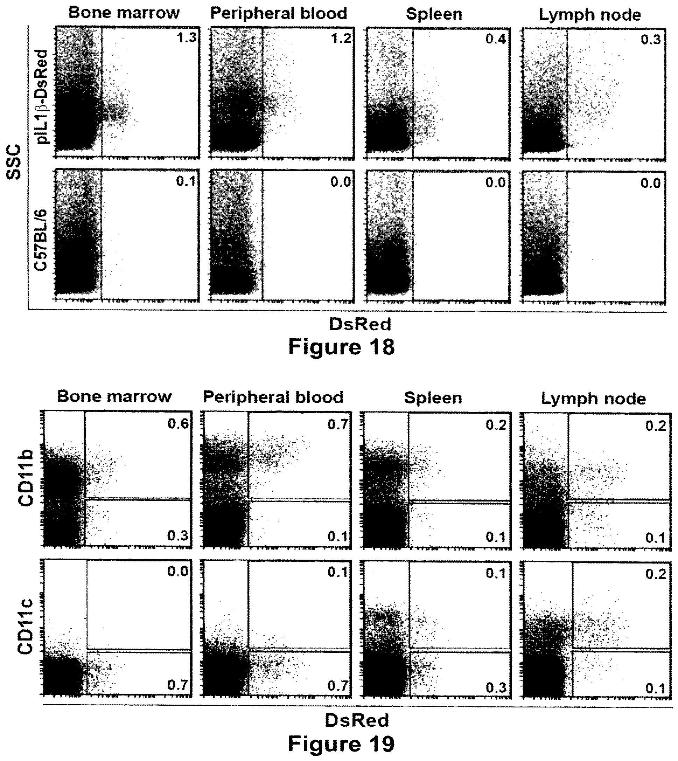

**Figure 18****.** DsRed+ cells identified in lymphoid tissues of pIL1-DsRed transgenic mice: Cell suspensions freshly prepared from the BM, peripheral blood, spleen, and lymph nodes of pIL1-DsRed transgenic mice (top) or wild-type control mice (bottom) were examined for DsRed expression (X-axis) versus granularity (Y-axis). Note the presence of DsRed+ cells in all tested tissues, but only from pIL1-DsRed transgenic mice.
**Figure 19****.** Identity of DsRed+ cells in lymphoid tissues: Cell suspensions freshly prepared from the BM, peripheral blood, spleen, and lymph nodes of pIL1-DsRed transgenic mice were examined for DsRed expression (X-axis) versus surface expression for CD11b or CD11c (Y-axis). Note that CD11b is expressed by a majority of the tissue-resident DsRed+ cells, consistent with our finding with DsRed+ cells in BM cultures.

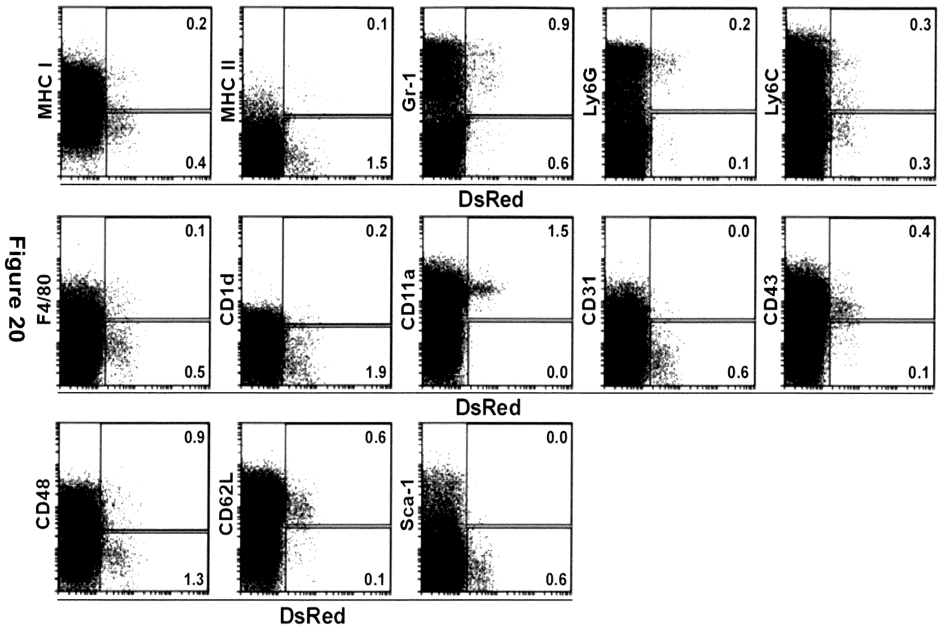

**Figure 20****.** Surface Phenotype of resident DC.com in BM: Cell suspensions freshly prepared from the BM of pIL1-DsRed transgenic mice were examined for DsRed expression (X-axis) versus surface expression for the indicated molecules (Y-axis). Note that the BM-resident DsRed+ population is indistinguishable from the DC.com identified in the BM cultures.

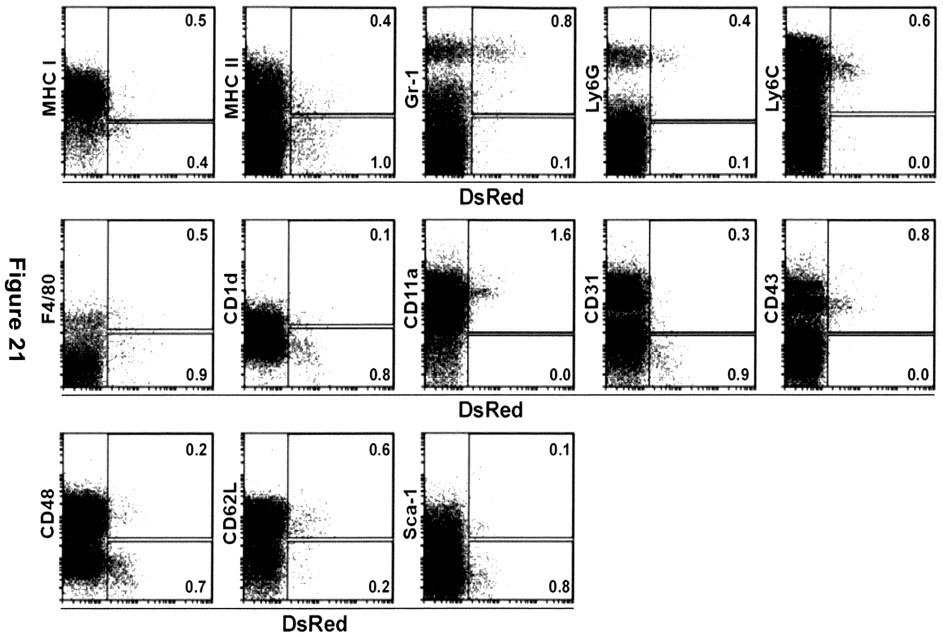

**Figure 21****.** Surface Phenotype of resident DC.com in peripheral blood: Peripheral blood samples freshly prepared from pIL1- DsRed transgenic mice were examined for DsRed expression (X-axis) versus surface expression for the indicated molecules (Y-axis). Note that the DsRed+ population in the peripheral blood is indistinguishable from the DC.com identified in the BM cultures.

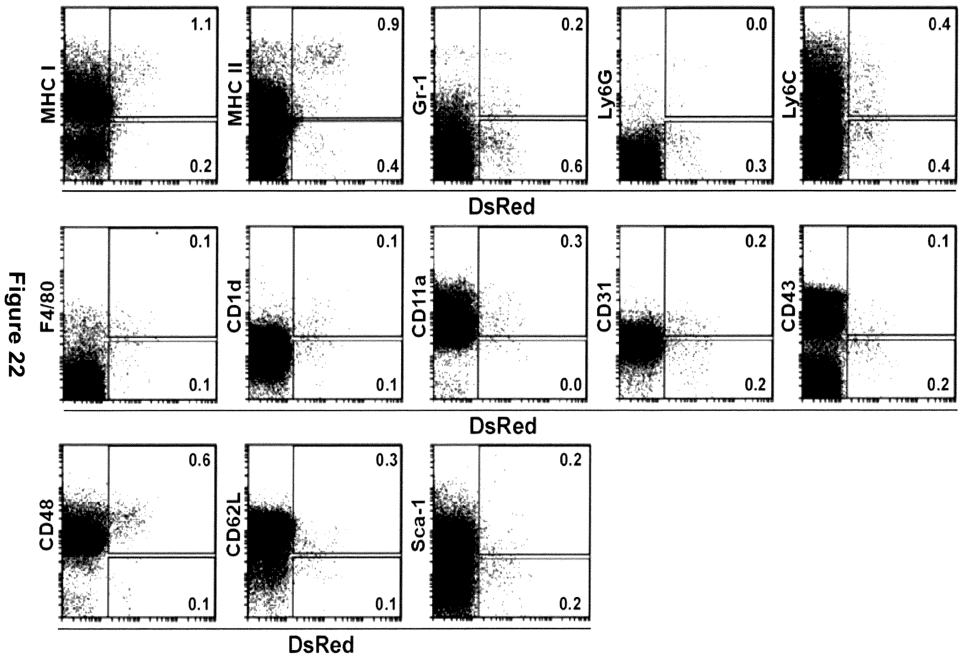

**Figure 22****.** Surface Phenotype of resident DC.com in lymph nodes: Cell suspensions freshly prepared from the inguinal lymph nodes of pIL1-DsRed transgenic mice were examined for DsRed expression (X-axis) versus surface expression for the indicated molecules (Y-axis). Note that the DsRed+ population in the lymph nodes is indistinguishable from the DC.com identified in the BM cultures.

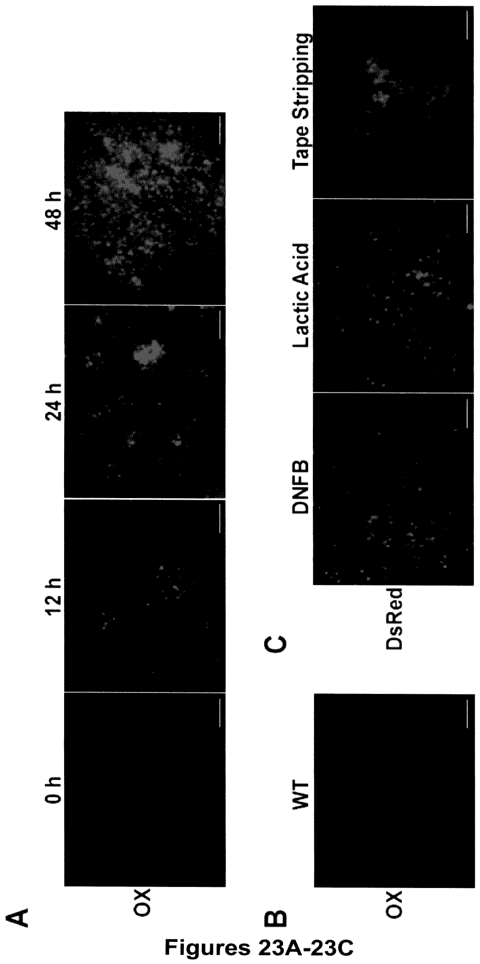

**Figures 23A-23B****.** Emergence of DsRed+ cells in the skin under inflammatory conditions: pIL1-DsRed transgenic mice were examined for DsRed expression on the ear skin by confocal microscopy at different time points after topical application of oxazolone (OX) **(****Figure 23A****).** The ear skin of wild-type mice was examined 24 hours after OX application **(****Figure 23B****).** The ear skin of pIL1-DsRed transgenic mice was examined 24 hours after topical application of dinitrofluorobenzene (DNFB) or lactic acid, or after tape stripping (Figure 23C). Scale bar: 50 µm. Note that DsRed+ cells are rarely found in the normal healthy skin, but they emerge rapidly under all tested inflammatory conditions.

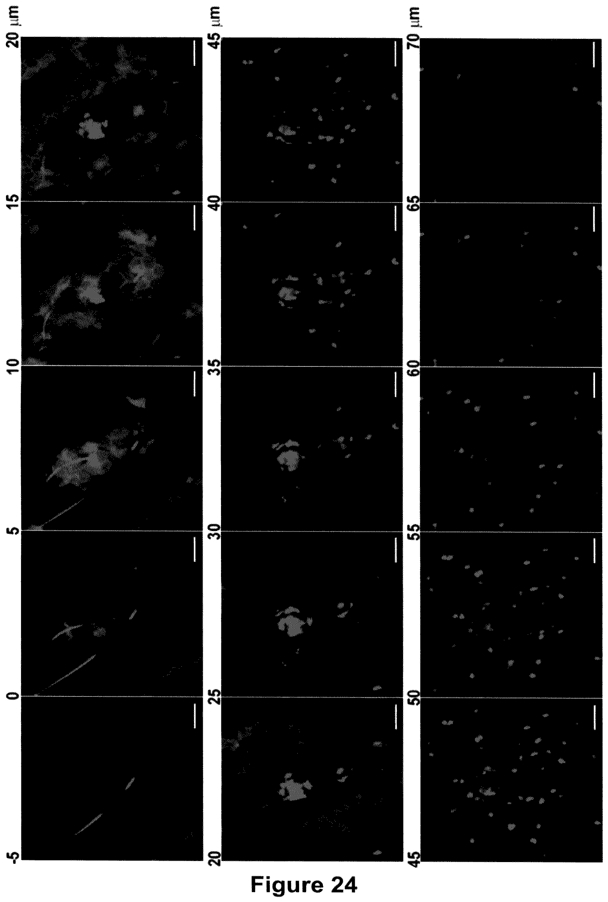

**Figure 24****.** Location of DsRed+ cells emerging in inflammatory skin lesions: pIL1-DsRed transgenic mice were examined for DsRed expression on the ear skin by confocal microscopy 24 hours after topical OX application. The data shown are the z-axis localization of DsRed+ cells at the indicated depth from the skin surface. Scale bar: 50 µm. Note that although a few DsRed+ cells are found in the epidermal compartment (from 0 to 30 µm ranges), a majority of DsRed+ cells are located in the dermal compartment (from 30 to 70 µm ranges) preferentially around hair follicles. Fluorescence signals observed at the skin surface represent autofluorescence associated with hair follicles and the *Stratum corneum* (the outermost layer composed of dead keratunocytes).
[000138] **Figures 25A-25D****.** Correlation between DsRed fluorescence signals and IL-β production: Wild-type mice and pIL1-DsRed transgenic mice received topical application of OX or vehicle alone on the ear skin. IL-β mRNA expression in the ear skin samples was examined by real-time PCR 24 hours after topical application of OX or vehicle alone **(****Figure 25A****).** At the indicated time points after application of OX or vehicle alone on the ear skin, we examined IL-β protein levels in ear skin extracts by ELISA in wild-type mice **(****Figure 25B****)** and pIL1-DsRed transgenic mice **(****Figure 25C****).** In **Figure 25D****,** we also measured DsRed fluorescent signals in the skin extracts from OX-treated (closed symbols) or vehicle-treated (open symbols) wild-type mice (triangles) and pIL1-DsRed transgenic mice (circles). Data shown are the means ± SD from triplicate mice per group (*P<0.05, **P<0.01). Note that DsRed fluorescence signals well correlate with IL-β production in the tissue.

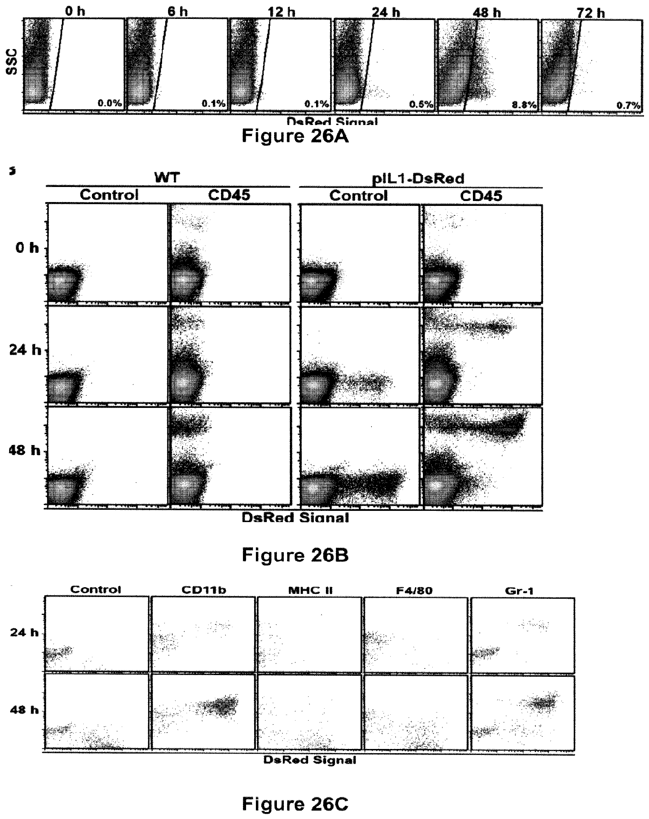

**Figures 26A-26C****.** Surface phenotype of DsRed+ cells emerging in the epidermal compartment: Epidermal cell suspensions were prepared from the ear skin of pIL1-DsRed transgenic mice at the indicated time points after topical OX treatment and examined for DsRed expression **(****Figure 26A****).** Epidermal cell suspensions from wild-type mice or pIL1-DsRed transgenic mice were also examined for DsRed expression and CD45 expression **(****Figure 26B****).** The CD45+ leukocyte populations in the above experiments were examined for the expression of the indicated marker **(****Figure 26C****).** Note that OX treatment induce time-dependent increase in the number of DsRed epidermal cells, which are CD45+, CD11b+, and Gr-1+.

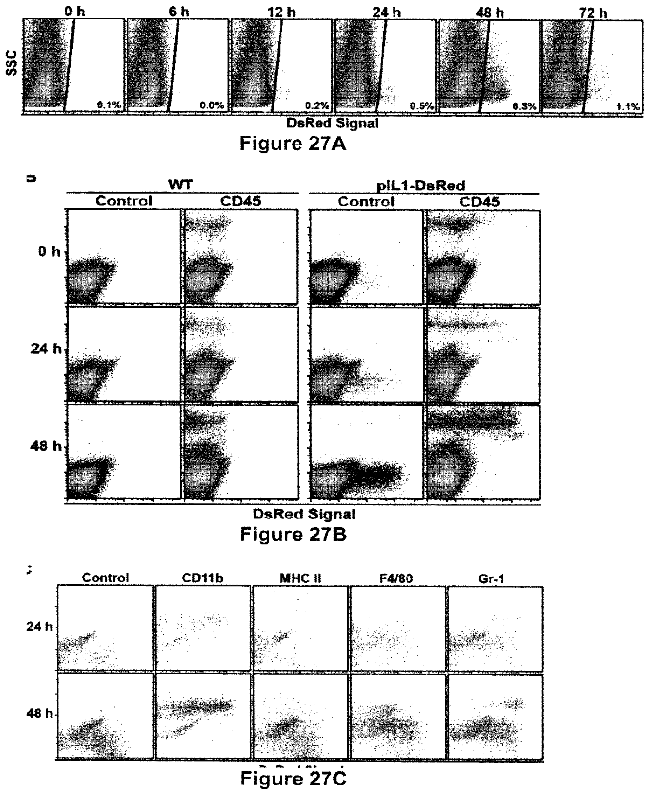

**Figures 27A-27C****.** Surface phenotype of DsRed+ cells emerging in the dermal compartment: Dermal cell suspensions were prepared from the ear skin of pIL1-DsRed transgenic mice at the indicated time points after topical OX treatment and examined for DsRed expression (**Figure 27A****).** Dermal cell suspensions from wild-type mice or pIL1-DsRed transgenic mice were also examined for DsRed expression and CD45 expression **(****Figure 27B****).** The CD45+ leukocyte populations in the above experiments were examined for the expression of the indicated marker **(****Figure 27C****).** Note that OX treatment induces time-dependent increase in the number of DsRed dermal cells, which are CD45+, CD11b+, and Gr-1+.

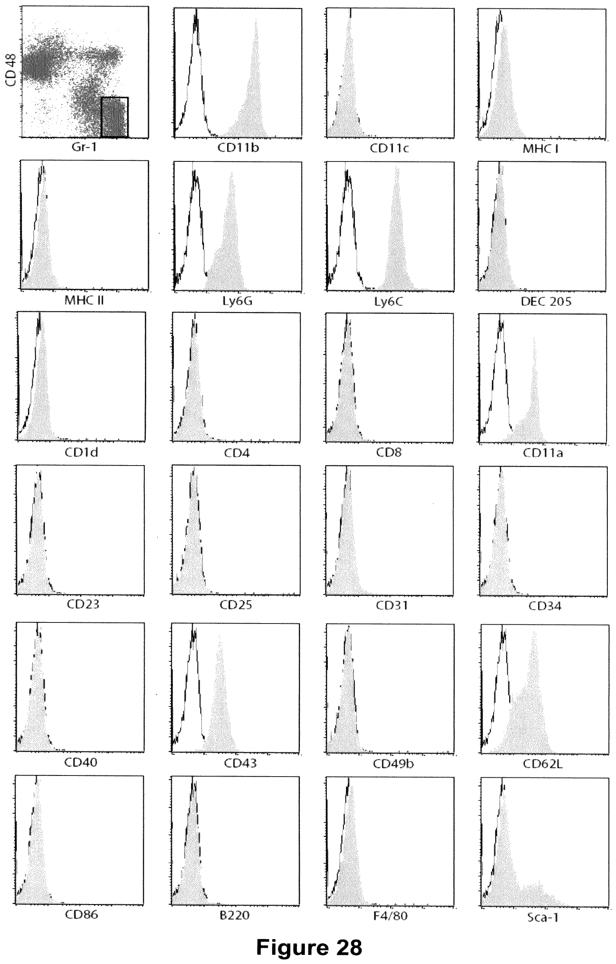

**Figure 28****.** Identification of DC.com in wild-type mice: GM-CSF-supplemented BM cultures (day 1) generated from wild-type C57BL/6 mice were examined for Gr-1 and CD48 expression. The CD48-negative/Gr-1-high population was then examined for the expression of the indicated surface marker. Note that the DC.com population identified in wild-type mice is indistinguishable from the CD11b+/DsRed+/CD11c- DC.com population originally identified using the pIL1-DsRed transgenic mice.

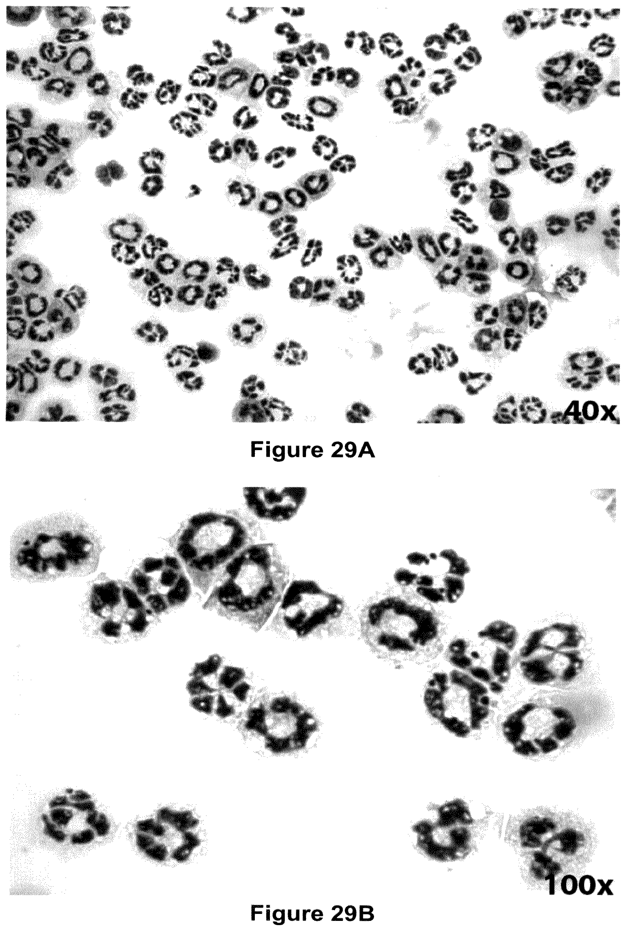

**Figures 29A-29B****.** Morphological characteristics of the DC.com population purified from wild-type mice: The CD48-negative/Gr-1-high population was FACS purified from GM-CSF-supplemented BM culture of wild-type C57BL/6 mice. The samples were then processed for H&E staining. Note that virtually all cells exhibit band-shaped lobulated nuclei, thus, resembling the DC.com cells purified from the pIL1-DsRed transgenic mice.

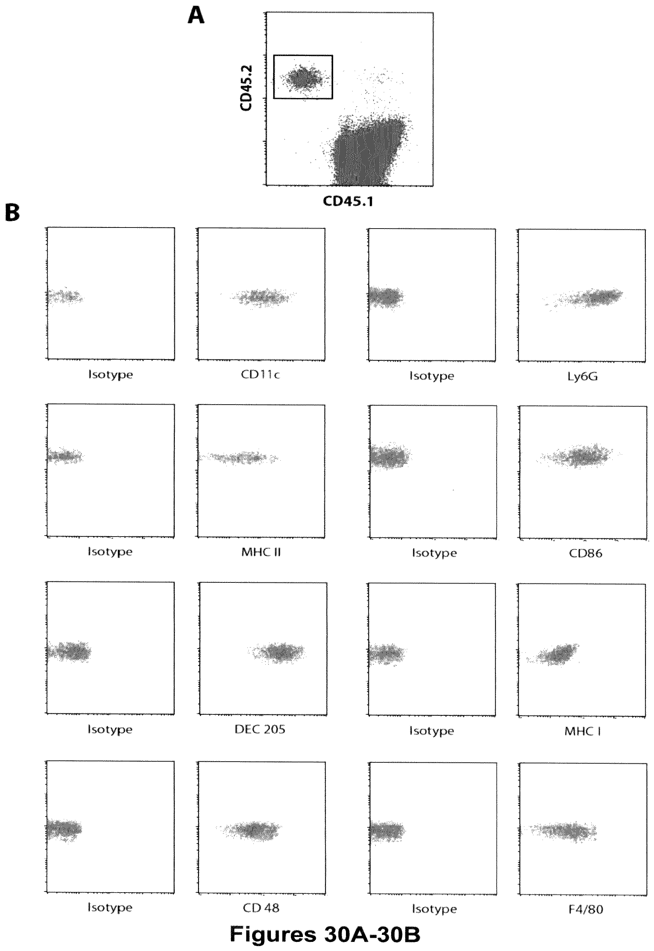

**Figures 30A-30B****.** Differentiation of DC.com during co-cultured with BM feeder cells: The CD48-negative/Gr-1-high DC.com population was FACS purified from BM cultures of C57BL/6 mice (which are CD45.2+), then co-cultured with freshly isolated BM cells from B6/SJL mice (which are CD45.1+) in the presence of GM-CSF. The cells derived from the DC.com population can be distinguished from the feeders by differential staining with anti-CD45.2 and anti-CD45.1 antibodies **(****Figure 30A****).** After 6 day co-culturing in this system, the CD45.2+ cells were analyzed for the surface expression of the indicated markers **(****Figure 30B****).** Note that DC.com cells acquire the expression of CD11c, MHC class II, DEC205 and other DC markers, while maintaining surface expression of Ly6G.

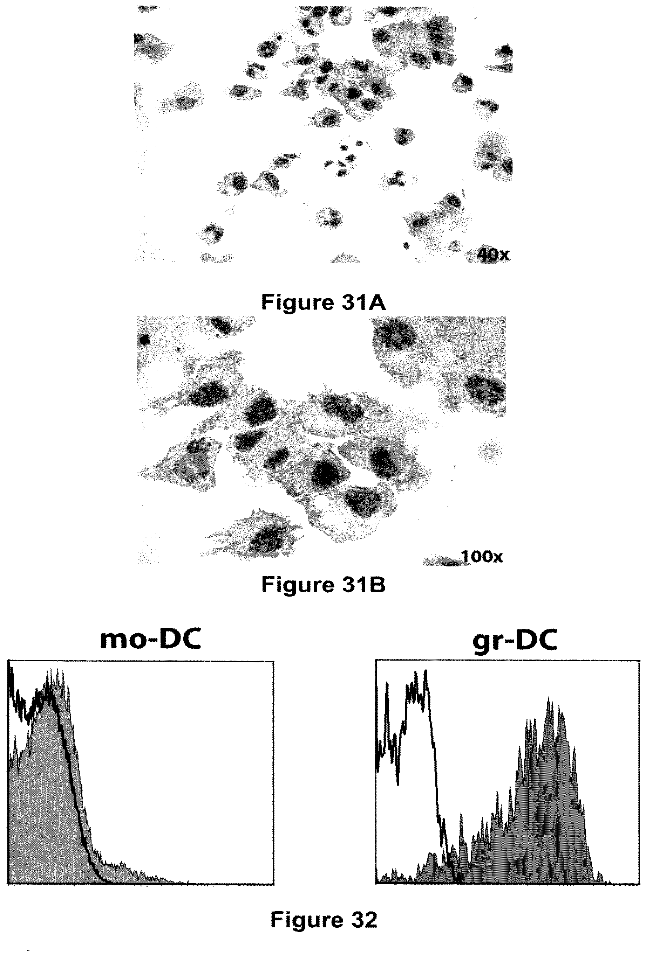

**Figures 31A-31B****.** Morphology of DC.com-derived DCs: The CD48-negative/Gr-1-high DC.com population was FACS purified from BM cultures of C57BL/6 mice (which are CD45.2+), then co-cultured with freshly isolated BM cells from B6/SJL mice (which are CD45.1+) in the presence of GM-CSF. After 6 day co-culturing in this system, the CD45.2+ cells were FACS-purified and processed for H&E staining. Note that a majority of the cells exhibit characteristic morphology of DCs, while a small number of cells shows numerous cytoplasmic granules.
**Figure 32****.** Differential expression of Ly6G by gr-DCs versus mo-DCs: CD11b+/CD11c-/Ly6G- monocytes freshly isolated from BM of wild-type mice were cultured for 6 days in the presence of GM-CSF. The CD48-negative/Gr-1-high DC.com population was FACS purified from BM cultures of C57BL/6 mice (which are CD45.2+), co-cultured for 6 days with freshly isolated BM cells from B6/SJL mice (which are CD45.1+) in the presence of GM-CSF. The resulting monocyte-derived DCs (mo-DCs) and DC.com-derived DCs (i.e., CD45.2+ cells in the co-culture and labeled as "gr-DCs") were then compared for surface expression of Ly6G (filled histograms). Open histograms indicate staining patterns with isotype-matched control IgG. Note that Ly6G expression can be used to distinguish the two DC subsets.

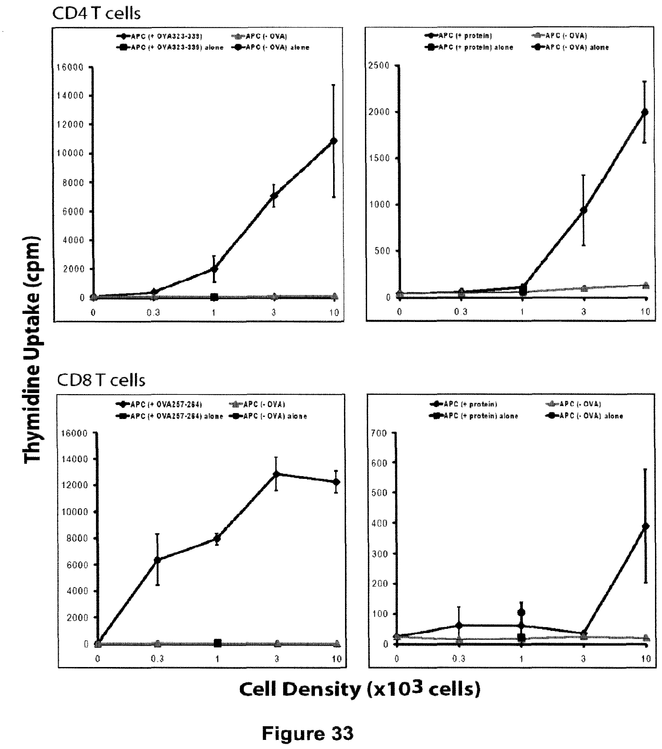

**Figure 33****.** Antigen presenting capacity of DC.com-derived DCs: The CD48-negative/Gr-1-high DC.com population was FACS purified from BM cultures of C57BL/6 mice (which are CD45.2+), co-cultured for 6 days with freshly isolated BM cells from B6/SJL mice (which are CD45.1+) in the presence of GM-CSF. The CD45.2+ cells were FACS-purified, pulsed with OVA protein (left panels) or OVA peptides (right panels) and then co-cultured with CD4 T cells purified from the OT-II transgenic mice (upper panels) or CD8 T cells purified from the OT-I transgenic mice (lower panels). The numbers of added gr-DCs/well are plotted in the X-axis. Triangles indicate control co-cultures of gr-DCs plus T cells in the absence of antigen. Circles indicate control cultures of gr-DCs alone without T cells. Data shown are 3H-thymidine uptake on day 4 (means ± SD from triplicate cultures). Note that DCs derived from the DC.com (i.e., gr-DCs) efficiently present OVA antigens to both CD4 and CD8 T cells.

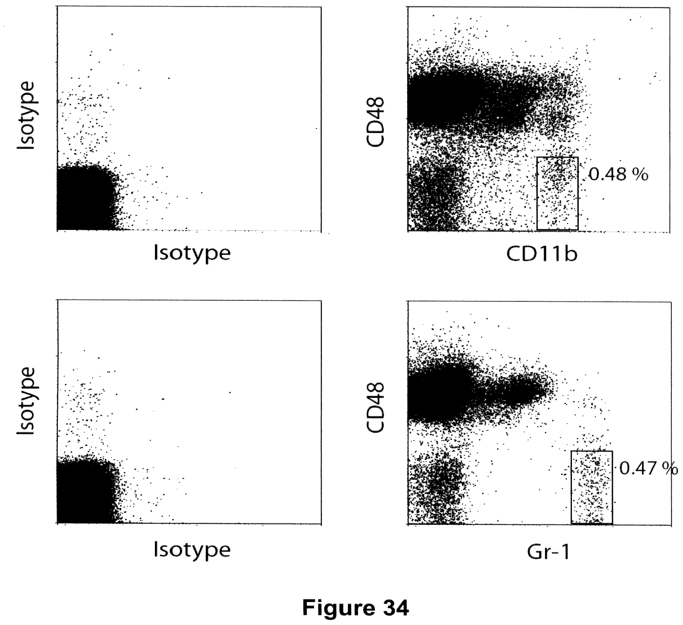

**Figure 34****.** Identification of the DC.com population in the spleen of wild-type mice: Crude spleen cells freshly harvested from wild-type C57BL/6 mice were examined for the presence of CD48-negative/CD11b-positive cells and CD48-negative/Gr-1-high cells. Note that spleen cells contain a small fraction (0.5%) of the cells exhibiting the characteristic phenotype of DC.com.

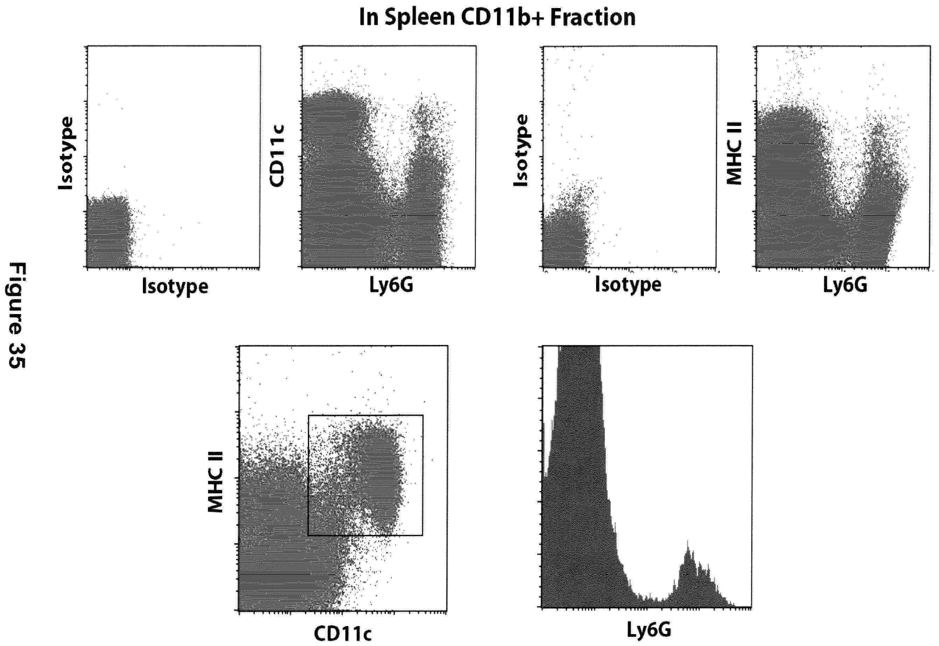

**Figure 35****.** Identification of granulocyte-derived DCs in the spleen of wild-type mice: Crude spleen cells freshly harvested from wild-type C57BL/6 mice were examined for the presence of CD11c+/MHC class II+/Ly6G+ cells. The panel in the right bottom corner indicates Ly6G expression within the CD11c+/MHC class II+ population. Note that a significant fraction (2.5%) of the CD11c+/MHC class II+ splenic DCs expresses Ly6G, a marker for granulocyte-derived DCs.

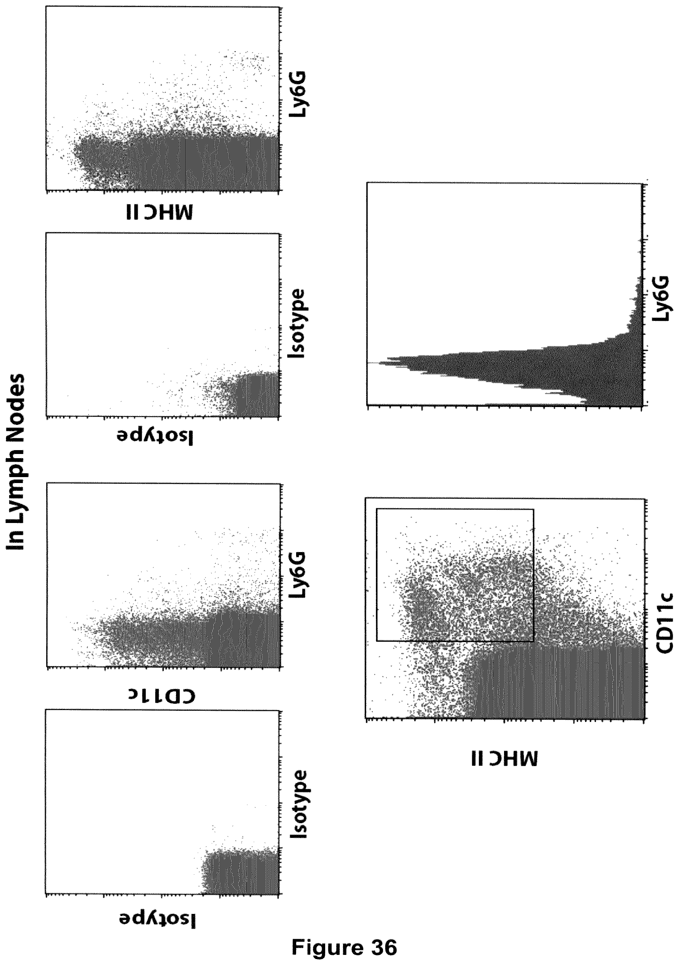

**Figure 36****.** Absence of granulocyte-derived DCs in the lymph nodes of wild-type mice: Crude lymph node cells freshly isolated from wild-type C57BL/6 mice were examined for the expression of CD11c+/MHC class II+/Ly6G+ cells. Note that granulocyte-derived DCs are almost undetectable in the lymph nodes in the steady state.

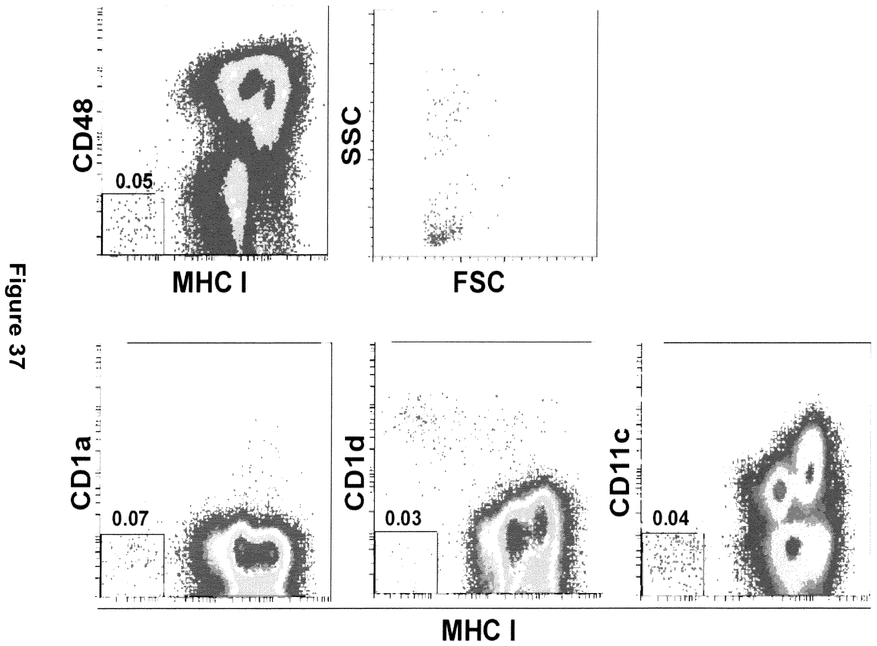

**Figure 37****:** Identification of the DC.com population in human peripheral blood: Human peripheral blood samples were examined for CD48 and MHC class I expression. The CD48- and MHC class I-double negative and small-sized population exhibits a uniform surface phenotype indistinguishable from the murine DC.com identified in the pIL1-DsRed transgenic mice.

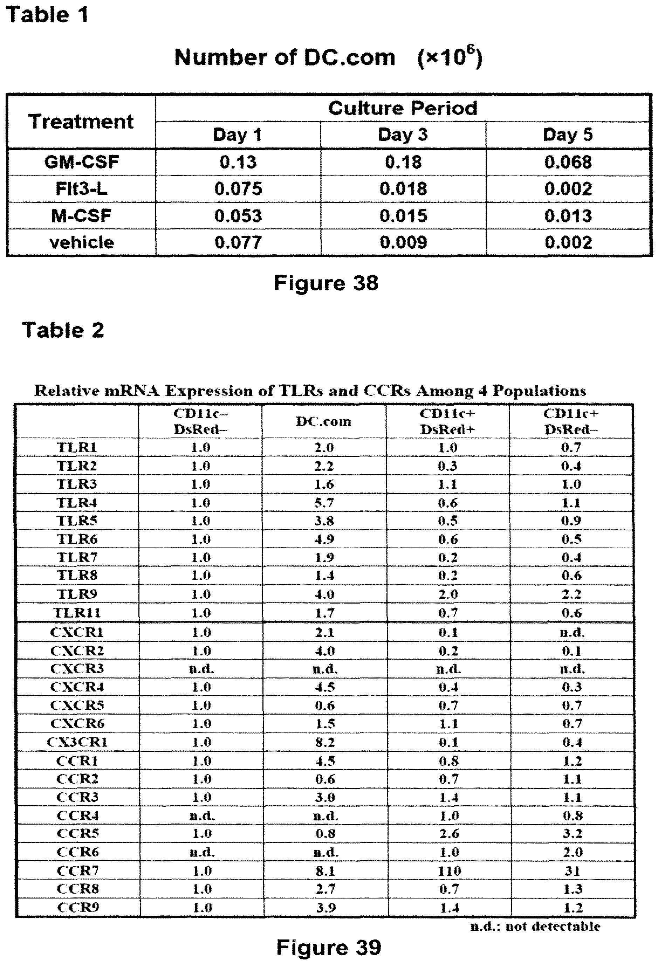

**Figure 38****:** Table 1 - Number of DC.com cells x 10⁶. The BM cells isolated from IL-1β-DsRed mice were cultured with GM-CSF (10ng/ml), Flt3L (200 ng/ml), M-CSF (100ng/ml) or vehicle alone for 5 days. The number of DC.com was analyzed by flow cytometry every 2 days.
**Figure 39****:** Table 2 - Relative mRNA expression of Toll like receptors and CC chemokine receptors among four (4) populations. The BM cells cultured with GM-CSF (10ng/ml) for 3 days were FACS purified into 4 fractions based on CD11c and DsRed expression. After purification, total RNA was extracted and real time RT-PCR was performed to examine the relative expression levels of the indicated genes. Note that the DC.com fraction is distinguishable from other fractions by a unique gene expression profile.

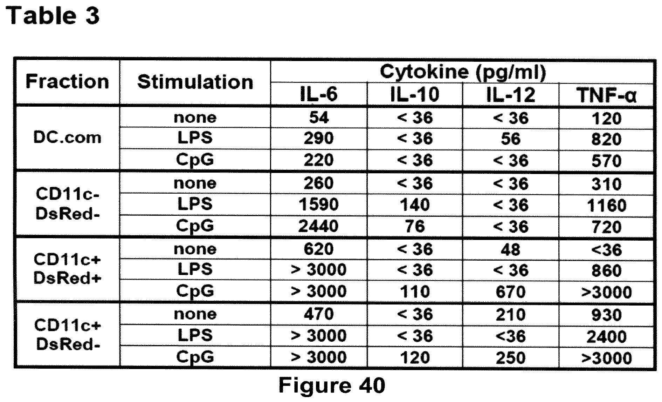

**Figure 40****:** Table 3 - Cytokine profiles among four (4) populations. The BM cells cultured with GM-CSF (10ng/ml) for 3 days were FACS purified into 4 fractions based on CD11c and DsRed expression. After purification, the 4 fractions were cultured with lipopolysaccharide (LPS) (100 ng/ml), CpG (1000 nM/ml) or no stimulation for 24 hours and the culture supernatant was examined for the indicated cytokine by Excell Array™.

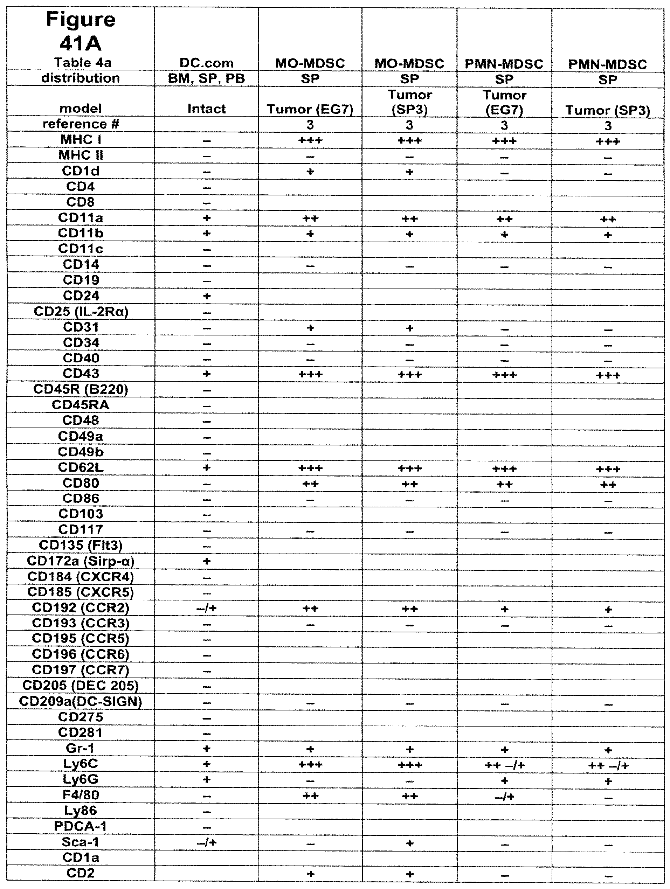

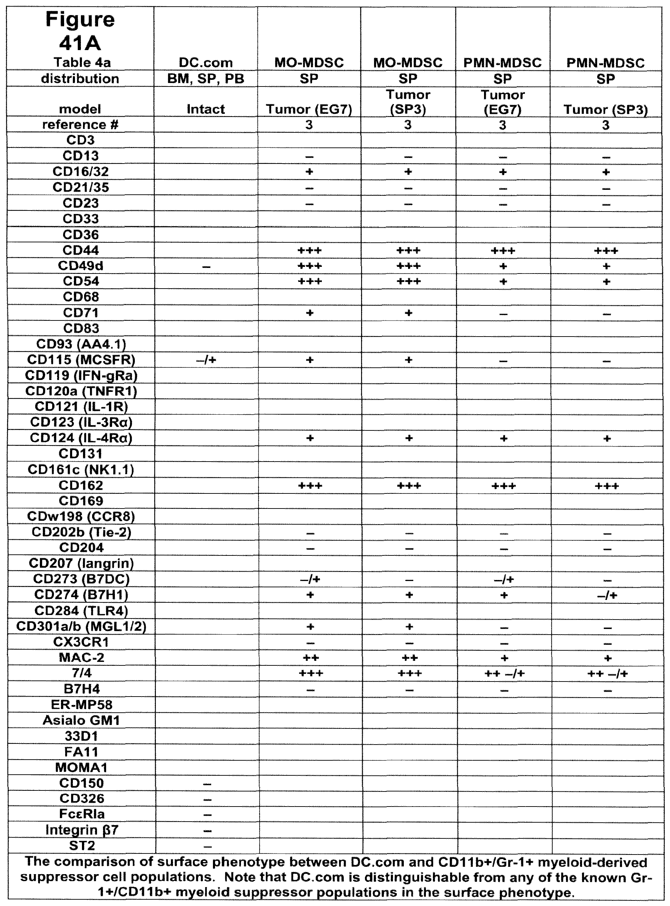

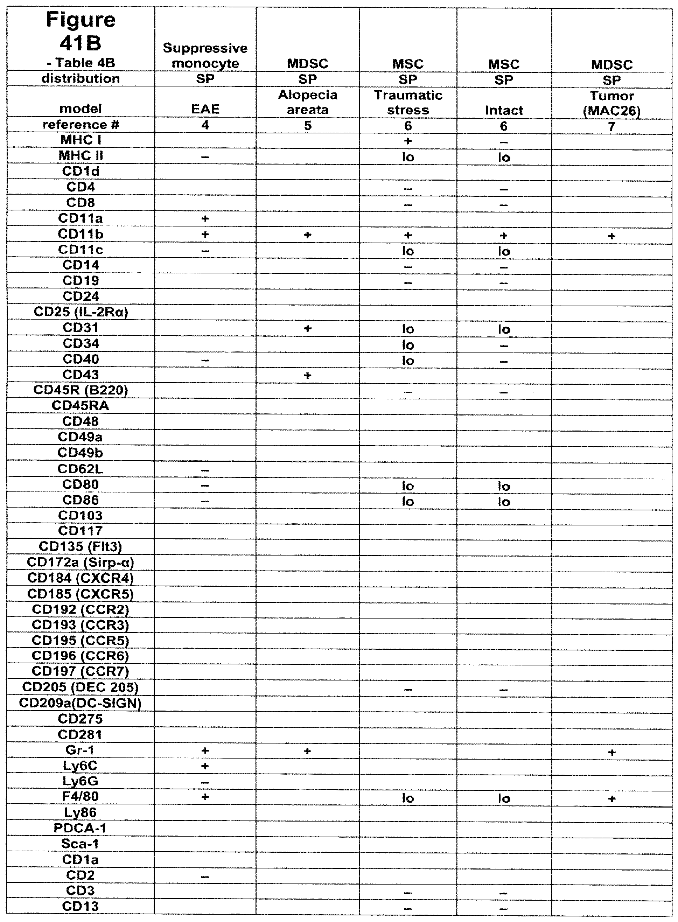

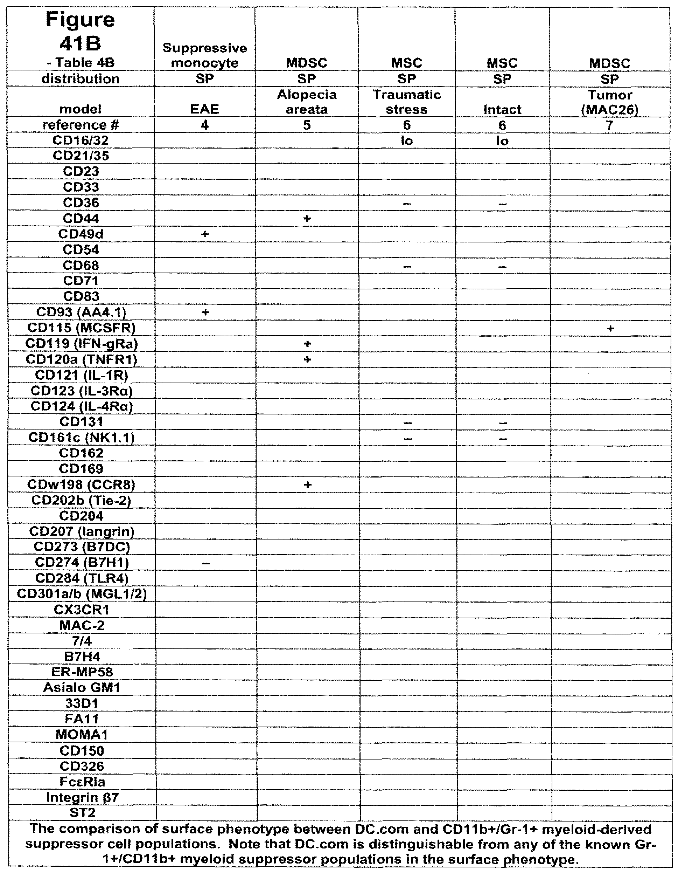

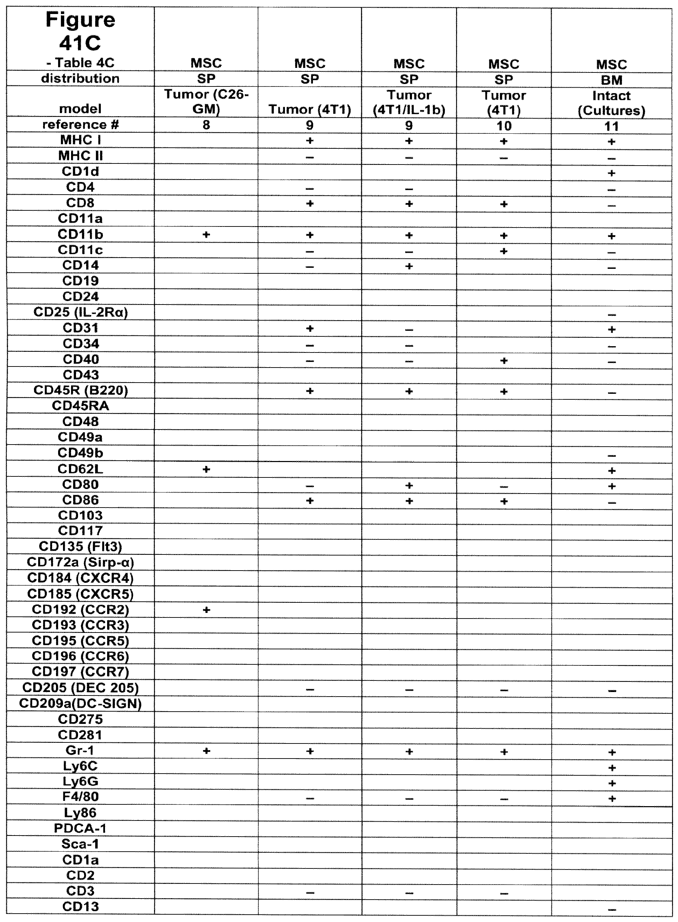

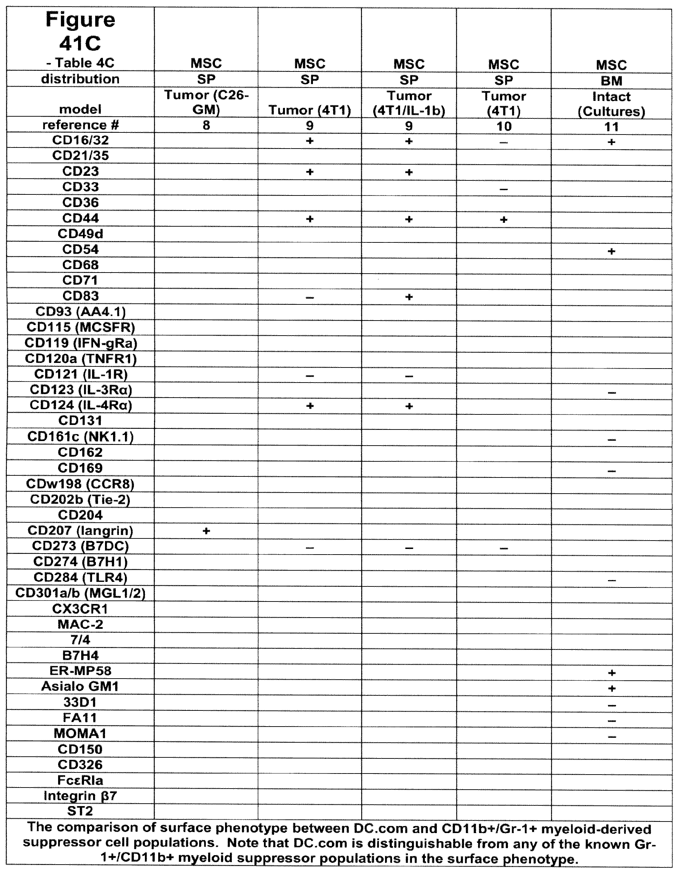

**Figures 41A-41C****:** Tables 4A-4C - The surface phenotype of DC.com was compared with CD11b+/Gr-1+myeloid-derived suppressor cell populations. The DC.com is distinguishable from any of the known CD11b+/Gr-1+ myeloid suppressor cell populations in the surface phenotype.

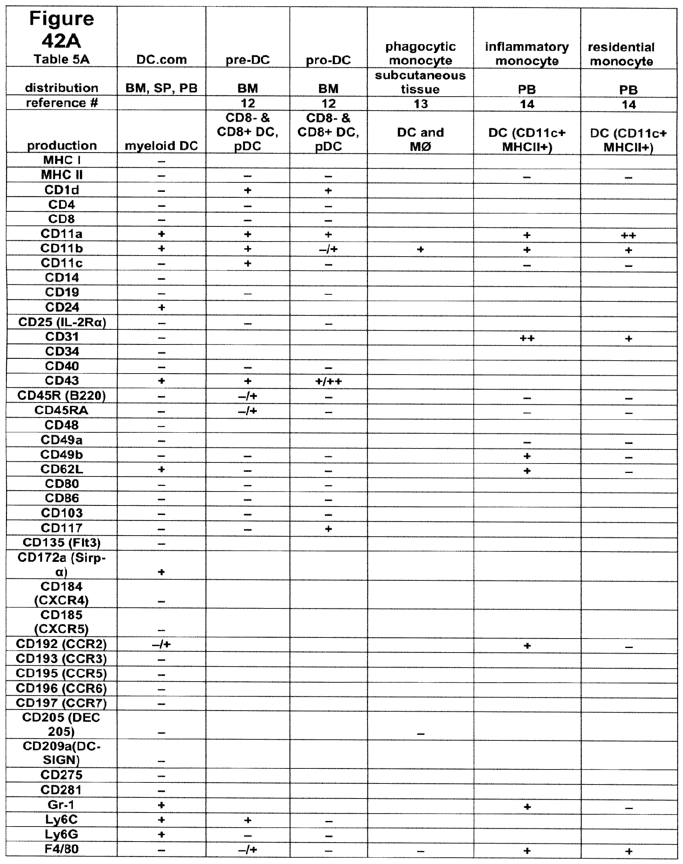

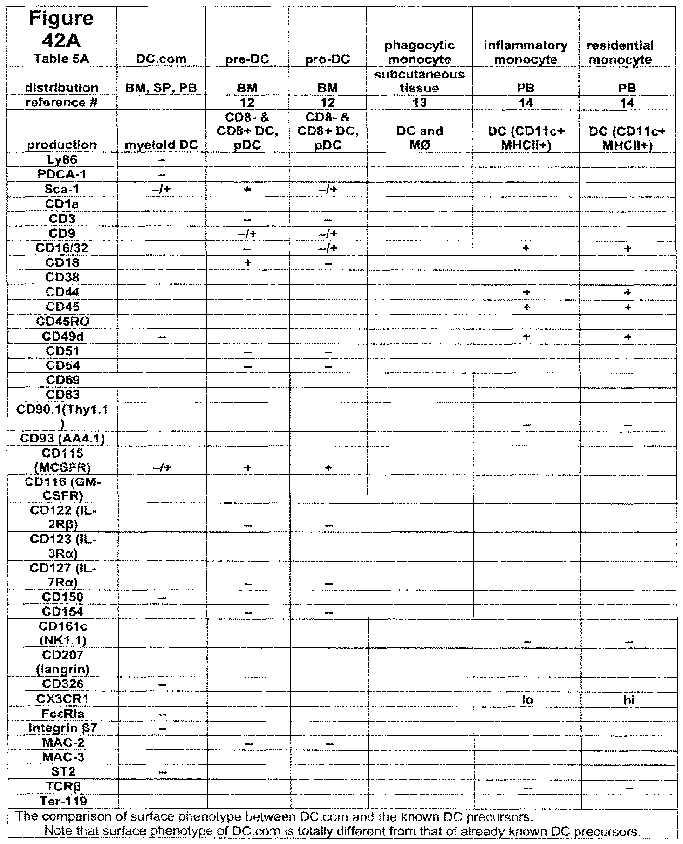

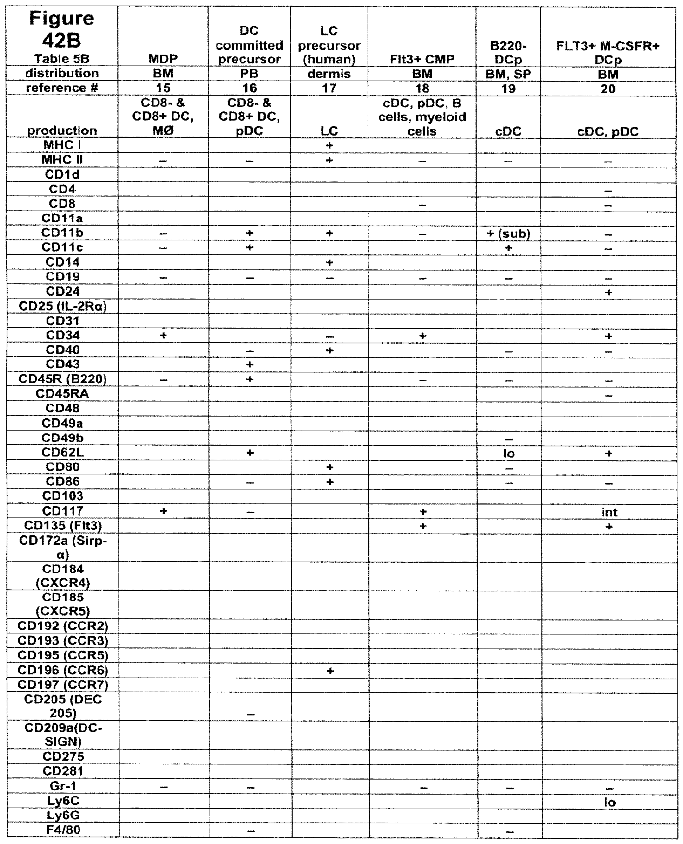

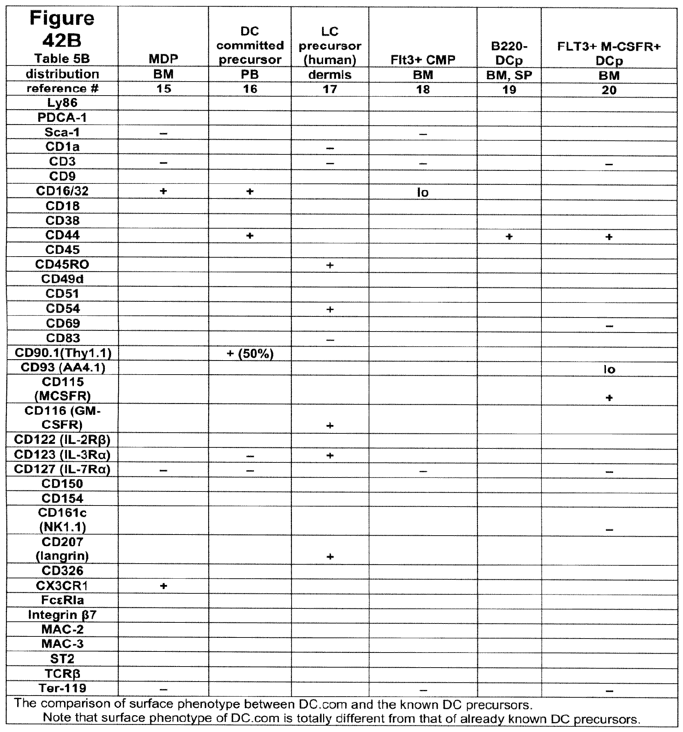

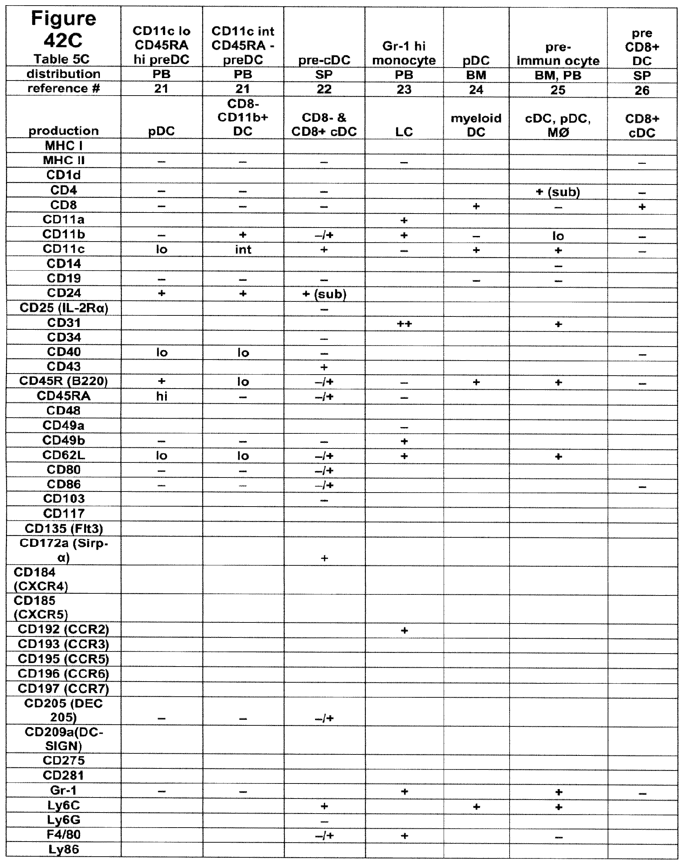

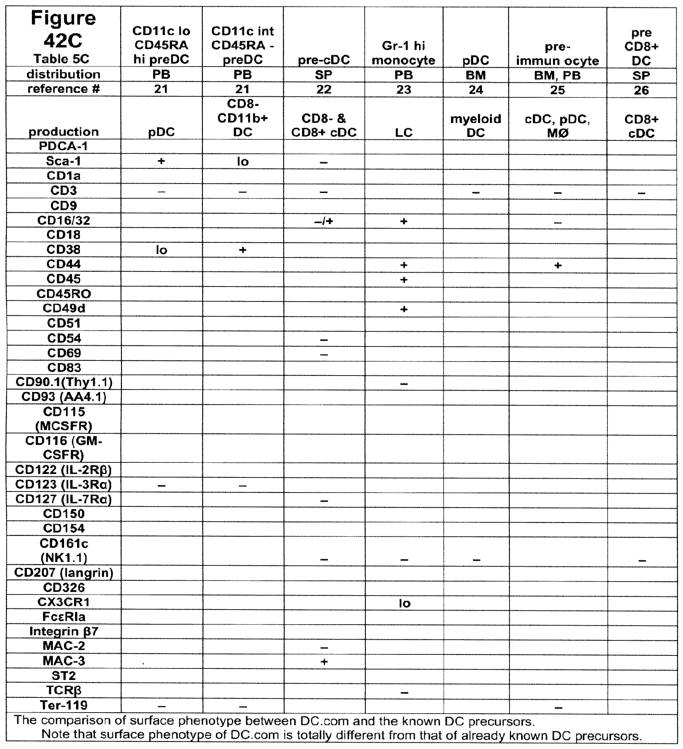

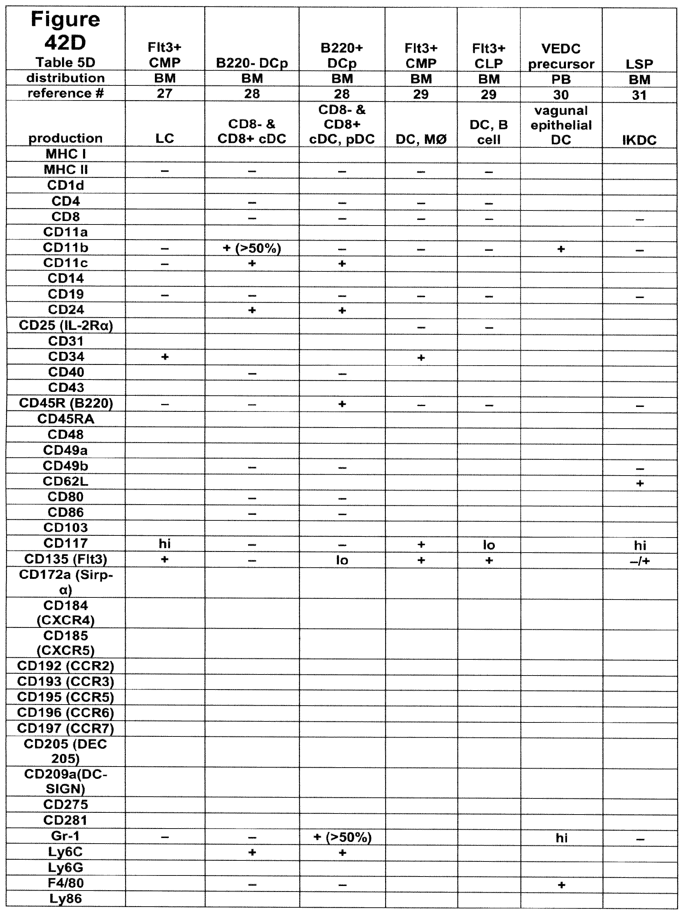

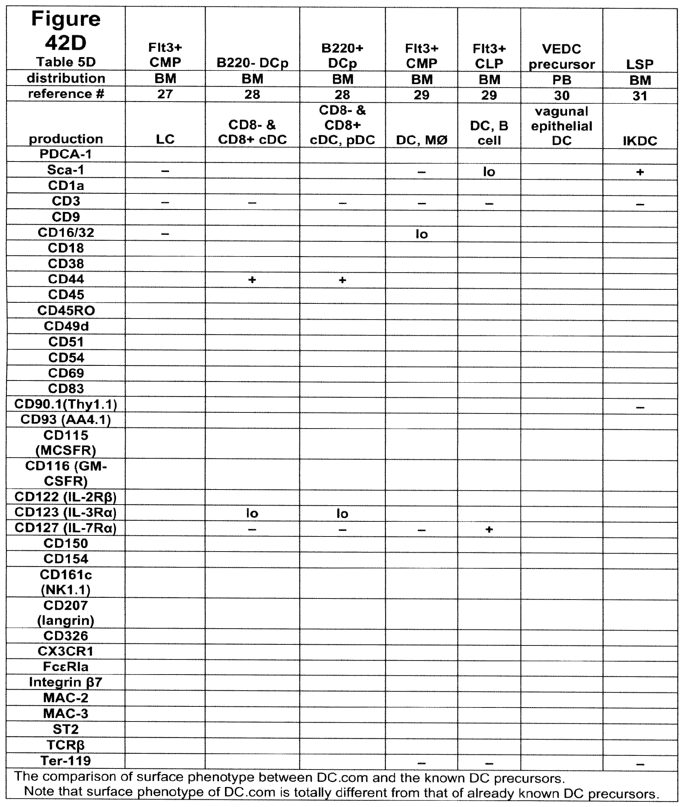

**Figures 42A-42D****:** Tables 5A-5D - The surface phenotype of DC.com was compared with reported DC precursors. DC.com is distinct from any other DC precursor.

### DETAILED DESCRIPTION OF THE PREFERRED EMBODIMENT(S)

Dendritic cells (DC) function as major antigen presenting cells that play protective roles in the induction of both innate and adaptive immune responses against infectious microorganisms, viruses, cancer cells, autoimmune diseases, graft rejection, graft versus host rejection disease, and other potentially harmful antigens. DCs in the steady state play equally important roles in the maintenance of immunological tolerance against self-antigens and harmless environmental antigens. However, the pathways for DC development still remain relatively unclear.

Described herein is a new DC developmental pathway in which a DC-committed precursor population, termed DC.com, differentiates into a previously unrecognized subset of DCs. The DC.com was discovered using a transgenic mouse strain expressing the DsRed gene under the control of murine IL-1β promoter. The same population was also discovered in wild-type mice and in healthy humans based on the characteristic surface phenotype, i.e., CD11b+/CD11c-/Ly6G+/CD48-/MHC I-/MHC II-.

When the DC.com population was cultured in the presence of GM-CSF, it differentiated into DCs showing characteristic dendritic morphology, DC markers (CD11c, DEC205, CD86, and MHC II), and potent abilities to present foreign protein and peptide antigens to both CD4 and CD8 T cells.

The DCs derived from the DC.com population differ from conventional DCs generated from monocytes by surface expression of Ly6G, which is generally considered as a marker of granulocytes. Indeed, the DC.com resembles to the "band cells," an immediate precursor population of neutrophils, not only by uniform surface expression of Ly6G, but also in the morphological features at both light and electron microscopic levels.

Also described herein is a new DC developmental pathway in which neutrophil precursors give rise to a unique DC subset, termed the granulocyte-derived DCs (gr-DCs).

We have identified a new DC developmental pathway in which a unique DC-committed population (termed the DC.com), which resembles a neutrophil progenitor population (known as band cells), differentiates into a DC subset (termed the gr-DC) that is distinct from any of the previously identified DC subpopulations. Herein, we describe the methods for identification, purification, expansion, and use of the DC.com and the gr-DC populations.

Based on the observation that rapid and profound IL-1β mRNA expression is a hallmark of DC activation (Mizumoto *et al.,* 2005b), we recently developed a DC biosensor clone by engineering our stable DC line XS106 to express the yellow fluorescence protein (YFP) gene under the control of a murine IL-1 β promoter **(****Figure 1****).**

The resulting XS106-pIL1-YFP bio sensor clone expressed YFP fluorescence signals upon stimulation with LPS, as well as other pharmacological agents known to deliver DC-stimulatory signals (Mizumoto *et al.,* 2005a). A major limitation of this system was the use of a long-term DC line, which may not fully represent bona fide DC populations in the body. To overcome this, we constructed the transgenic mouse line expressing the DsRed gene under the control of the same IL-1 β promoter. DC cultures were then generated from the pIL1-DsRed transgenic mice by culturing bone marrow (BM) cells for 5-6 days in the presence of exogenous GM-CSF. Corroborating with our observation with the XS106-pIL1-YFP DC biosensor clone, CD11c+ DC populations in Day 6 BM cultures showed markedly elevated DsRed signals in response to LPS treatment **(****Figure 2****).**

Totally unexpected was our finding that robust DsRed expression became detectable in GM-CSF-supplemented BM cultures in the absence of LPS stimulation **(****Figure 3****).** Time-kinetics experiments revealed rapid (within 24-48 hours) and striking (>200-fold) expansion of DsRed⁺ cells in BM culture **(****Figure 4****).**

Surface phenotype analyses revealed that an overwhelming majority (>95%) of the DsRed+ cells express CD11b (a marker of myeloid cells) throughout the 5 day-culture period in the presence of exogenous granulocyte-macrophage colony-stimulating factor GM-CSF **(****Figure 5A****).** By contrast, CD11c (a most reliable DC marker) was detected in about 50% of the DsRed+ cells on day 1 and the fraction of CD11c+ cells in the DsRed+ population increased up to 90% on day 5 **(****Figure 5B****).**

The observed time-course of CD11c expression within the CD11b+/DsRed+ populations suggested that DsRed+/CD11b+/CD11c- cells may represent a precursor for CD11c+ DCs. To test directly, we purified four distinct cell populations from day 3 BM cultures based on DsRed and CD11c expression using flow cytometry and placed them back in culture in the presence of added GM-CSF **(****Figure 6****).** The DsRed+/CD11c- fraction (labeled as DC.com) acquired uniform CD11c expression within 3 days of subsequent culture. Some of the DsRed-/CD11c- cells began to express DsRed as early as in 24 hours. Both DsRed+/CD11c+ and DsRed-/CD11c+ DC populations maintained CD11c expression, although DsRed expression became undetectable (or detectable) in the former (or the latter) population. These observations show that the CD11b+/DsRed+/CD11c- population represents an immediate precursor of DCs. We, thus, designated this DC-committed population as the "DC.com."

When BM cells were cultured in the presence of GM-CSF, the number of CD11b+/DsRed+/CD11c- DC.com cells increased dramatically (40-fold) in the first 24 hours **(****Figure 7****).** By contrast, the number of DsRed-/CD11c+ DCs increased progressively during the first 6 days in culture.

The emergence of CD11b+/DsRed+/CD11c-/ cells was found to be dependent upon the BM culture conditions **(****Figure 5** and **Figure 38** **- Table 1).** When BM cells were cultured in the presence of Flt3 ligand or M-CSF, which are also known to promote the survival and growth of DCs in general, only small numbers of the cells exhibited the phenotype of DC.com (CD11b+/DsRed+/CD11c-).

We next tested 65 different cytokines individually for the effects on the emergence of DsRed+ cells in BM culture **(****Figure 8****).** In addition to GM-CSF, selected cytokines appeared to support the emergence or generation of DsRed+ cells. When the same 65 cytokines were tested in combination with GM- CSF, selected cytokines, including interferon-γ, were found to inhibit GM-CSF-dependent development of DsRed+ cells **(****Figure 9****).** Interestingly, the same cytokines that promoted DsRed expression in BM culture (IL-3, IL-18, and IL-33) in **(****Figure 10****)** also supported the development of CD11c+ DCs.

These data demonstrate that development of the DC.com population and its subsequent differentiation into DCs are both regulated by cytokines. At the same time, the data show that similar assay systems can be used to identify compounds that enhance and/or inhibit the generation and differentiation of DC.com cells.

Based on the expression of DsRed and CD11c, we purified four CD11b+ populations from GM-CSF-supplemented BM culture. The DsRed+/CD11c- DC.com population differed from CD11c+ DC fractions in the morphology and the cell size **(****Figure 11****).** At electron microscopic levels, the DC.com fraction further exhibited a unique morphology characterized by lobulated nuclei and inclusion of modest numbers of cytoplasmic granules **(****Figure 12****).** Importantly, the DC.com population was extremely homogeneous in terms of surface phenotype, cell size, granularity, and morphology, indicating the efficiency of our purification strategy.

We tested mitotic potentials of the above four fractions by BrdU uptake and 7-AAD staining **(****Figure 13****).** The DC.com population exhibited relatively limited mitotic potentials as compared to the CD11b+/DsRed-/CD11c- population in BM culture.

The DC.com population is distinguishable from CD11c+ DCs based on the surface phenotype **(****Figure 14****).** CD11c, CD48, and MHC class I and class II molecules, which are expressed at relatively high levels on DCs, are not detectable in the DC.com population. Conversely, only the DC.com population uniformly expresses Gr-1 and Ly6G at high levels. It is also to be noted that anti-Gr-1 antibody recognizes two distinct antigens, Ly6G and Ly6C. Although Ly6C has been shown to be expressed by selected monocyte and DC subsets, there is no report documenting Ly6G expression by monocytes or DCs. Thus, Ly6G is generally considered to be a highly specific marker of granulocytes.

The DC.com population showed no detectable antigen presenting capacity as measured by the ability to present OVA protein and peptide to CD8 and CD4 T cells isolated from OT-I and OT-II T cell receptor transgenic mice (in which virtually all CD8 and CD4 T cells recognize OVA peptides). By marked contrast, both DsRed+/CD11c+ DCs and DsRed-/CD11c+ DCs exhibited potent capacities to present OVA protein and peptide to both CD8 cells **(****Figure 15A****)** and CD4 T cells **(****Figure 15B****).**

Surface expression of CD11b and Gr-1 by the DC.com cells may suggest their functional resemblance to the recently identified leukocyte population known as "myeloid-derived suppressor cells" (Zhu *et al.,* 2007) (Marhaba *et al.,* 2007) (Makarenkova *et al.,* 2006) (Gallina *et al.,* 2006) (Bunt *et al.,* 2006) (Sinha *et al.,* 2005). Importantly, the CD11b+/DsRed-/CD11c- fraction from the BM culture, but not the CD11b+/DsRed+/CD11c-fraction, exhibited the ability to inhibit antigen-specific proliferation of OT-1 CD8 T cells. Thus, the newly identified DC.com population differs functionally from the myeloid-derived suppressor cells **(****Figure 16****).**

GeneChip analyses revealed striking diversity among the four fractions in terms of gene expression profiles. Indeed, real-time PCR analyses confirmed some of these findings that the DC.com differs from other populations in the Toll like receptor and chemokine receptor expression profiles **(****Figure 40** **- Table 2).**

CD11c+ DCs elaborated large amounts of cytokines, such as IL-6 and TNFα, after stimulation with LPS or CpG oligonucleotides. By contract, the DC.com fraction released only modest amounts of these pro-inflammatory cytokines.

The most striking feature of the DC.com fraction is its potent ability to internalize exogenous molecules as examined by a standard FITC-dextran uptake assay **(****Figure 17****).**

In addition to isolating the DC.com fraction from BM culture, we determined that similar populations can be identified in lymphoid tissues. In the pIL1-DsRed transgenic mice, DsRed expression was detectable in various lymphoid tissues, including BM, peripheral blood, spleen, and lymph nodes **(****Figure 18****).** Moreover, those tissue-resident DsRed+ cells were virtually indistinguishable from the DC.com population originally identified in the BM cultures in terms of the cell size and surface phenotype **(****Figures 19-22****).**

Having confirmed the presence of DC.com cells in lymphoid tissues, we next examined their presence in epithelial tissues. No DsRed+ cells were found in the ear skin of the pIL1-DsRed transgenic mice in the steady state **(****Figure 23****).** However, we observed rapid emergence of DsRed+ cells after topical application of skin sensitizing chemicals (oxazolone and DNFB) or skin irritating chemicals (lactic acid). Skin inflammation caused by mechanical treatment (tape stripping) also induced the emergence of DsRed+ cells in the skin. Thus, we conclude that DsRed+ cells emerge in epithelial tissues only under inflammatory conditions.

In the skin, DsRed+ cells were primarily found in the dermal compartment, preferentially around hair follicles **(****Figure 24****).** Using the skin as a model tissue, we also confirmed that DsRed fluorescence signals indeed correlate with IL-1β □ production in the transgenic mice **(****Figure 25****).**

FACS analyses of these DsRed+ skin cells revealed their phenotypic resemblance to the DC.com population we identified in the BM culture system **(****Figure 26** and **Figure 27****).**

We also determined that similar populations can be identified in the absence of DsRed fluorescence signals in wild-type mice. We found that the DC.com population can be identified and purified by using only two surface markers, CD48 and Gr-1 (or Ly6G). The CD48-negative/Gr-1-high population in the BM culture from wild-type mice showed the same phenotype of the DC.com originally identified using the pIL1-DsRed mouse system **(****Figure 28****).**

Moreover, the CD48-negative/Gr-1-high population purified from the wild-type BM culture exhibited the same morphological features of DC.com cells **(****Figure 29****).** The H&E staining patterns of this population were almost indistinguishable from those reported for "band cells", which are immediate precursors of neutrophils. Surface expression of a granulocyte marker (Ly6G) by the DC.com further supports the notion that the DC.com population closely resembles the band cells or immature granulocytes.

The same CD48-negative/Gr-1-high population also differentiated into DCs expressing CD11c, MHC class II, CD86, and DEC205 **(****Figure 30****).** Importantly, Ly6G (which is expressed by the DC.com) remained on the surface of those DCs derived from the DC.com. Moreover, when the DC.com was cultured with GM-CSF, a majority of the cells began to express the characteristic dendritic morphology **(****Figure 31****).** On the other hand, a small number of cells differentiated into cells resembling typical granulocytes. These data show that the DC.com gives rise to a previously unrecognized subset of DCs, which we have termed granulocyte-derived DCs or gr-DCs.

Conventional DCs derived from monocytes showed no detectable Ly6G expression **(****Figure 32****).** By marked contract, DC.com-derived gr-DCs uniformly expressed Ly6G at high levels. Thus, Ly6G can be used as a unique marker distinguishing the gr-DC subset from other DC subsets.

The gr-DC preparation generated from the DC.com exhibited a potent ability to present foreign antigens to both CD8 and CD4 T cells **(****Figure 33****).**

Using the same markers (i.e., CD11b, CD48, and Gr-1), we have identified DC.com populations in the spleen of wild-type mice (Figure 34).

Moreover, using another set of markers (i.e., CD11c, MHC class II, and Ly6G), we have identified gr-DC populations in the spleen, but not lymph nodes, of wild-type mice **(****Figure 35** and **Figure 36****).**

We have also identified a DC.com population in human peripheral blood samples based on the cell size and the surface phenotype of CD48-/MHC class I-/CD1a-/CD1d-/CD11c- **(****Figure 37****).** It should be stated that although a human equivalent for murine Ly6G has not been identified, the above markers will enable identification and purification of human DC.com populations.

The DC.com population differs from any myeloid-derived suppressor cell populations **(****Figures 41A-C** **- Tables 4A-C)** and any DC precursor populations reported in the literature **(****Figures 42A-D** **- Table 5A-D).**

The DC.com population most closely resembles an immediate progenitor of granulocytes, known as the band cells.

The DC.com differentiates into a unique DC subset (termed gr-DCs), which differs from other DC subsets by the uniform expression of Ly6G at high levels.

Methods for screening a large number of test samples for their potential to promote or inhibit the development of DC.com cells are also within the contemplated scope of the present invention.

In addition, these methods can be used to identify molecules that promote or inhibit the differentiation of DC.com into gr-DCs.

The present invention is further explained in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the scope thereof, can make various changes and modifications of the invention to adapt it to various usages and conditions.

### EXAMPLE I

### Materials and Methods

### Animals

C57BL/6, OT-I, and OT-II mice were obtained from Taconic Farms, Inc. (Germantown, NY). All animals in this study were bred and maintained with the specific pathogen-free conditions in the Animal Research Center facilities at the University of Toledo (Toledo, OH). All of the animal experiments were approved by the Institutional Review Board and conducted according to guidelines of National Institutes of Health (Bethesda, MD).

### Generation of IL-1β-RFP mice

A 1.2kB rabbit β-globin gene containing noncoding intron/exon was obtained by digesting the pSG-1 expression vector with *Bam*HI and *Xho*I (reference 1, 2). The fragment was subcloned to the *Bam*HI/*Xho*I site of pBK-CMV (Stratagene, La Jolla, CA) to produce the plasmid pBK-CMV-SG. To generate a red fluorescent protein(RFP)-expressing vector, a PCR fragment was amplified from pDsRed-Express-DR plasmid (Clontech, Palo Alto, CA) using primer sets, 5'-GGGAATTCCGGTCGCCACCATGGCCTC-3' **[SEQ ID NO:1**] and 5'-GGAGATCTACACATTGATCCTAGCAGAAG-3' **[SEQ ID NO:2],** which was subsequently ligated to a TA-cloning vector, pCR4-TOPO (Invitrogen, Carlsbad, CA), and then subcloned between the *Eco*RI and *Bgl*II sites of pBK-CMV-SG.

The resulting vector, pBK-CMV-SG-Red, carried a CMV immediate early promoter upstream of the β-globin intron/exon-RFP fusion gene. The CMV promoter region was removed by digestion with *Vsp*I and *Nhe*I followed by blunting of both ends with Klenow fragment and self ligation. Then the 4,138-bp *Bam*HI fragment of the murine IL-1β promoter (reference 3) was inserted into the BamHI site to generate the plasmid pBK-SG-IL-1β-Red. The plasmid pBK-SG-IL-1β-Red was digested with *Sal*I and *Not*I to clear the vector sequences, and the transgene fragment was purified by Elutip-D (Schleicher & Schuell, Keene, NH). DNA was microinjected into fertilized eggs of C57B1/6 mice. Thirty-four founders were born and ten of them were confirmed to carry the transgene by PCR analysis using primers, 5'-TGCTGGTTGTTGTGCTGTCTCATC-3' **[SEQ ID NO:3]** and 5'-CACGTACACCTTGGAGCCGTACTG-3' [**SEQ ID NO:4].**

### Cell isolations and in vitro cultures

Blood was collected by lumbar puncture into heparinized tubes. Red blood cells (RBC) were eliminated with Red Blood Cell Lysing Buffer (Sigma-Aldrich, St Louis, MO), followed by staining with mAbs for flow cytometry analysis. Single cell suspensions for spleens and lymph nodes were prepared by mechanically disrupting the organs and lysing RBC. Bone marrow (BM) cells were isolated by flushing femurs with complete RPMI1640 (cRPMI); RPMI1640 (Cellgro, Herndon, VA) supplemented with heat-inactivated 10% FBS, 2 mM L-glutamine, 10 mM nonessential amino acids, 1x penicillin/streptomycin, 10 mM sodium pyruvate, 25 mM HEPES and 50 µM 2-ME (Sigma-Aldrich). After depleting RBC, the cells (2 x 10⁶ cells/ml) were cultured in 6-well cell culture plates (Corning, Lowell, MA) in cRPMI supplemented with 10 ng/ml GM-CSF, 10 ng/ml M-CSF (both from R & D Systems, Minneapolis, MN), or 200 ng/ml FLT-3L (Peprotech EC, London). The cells were harvested at different times from the cultures by removal of non-adherent cells and by treatment with 0.3% Trypsin/0.025% EDTA (Cellgro) to recover adherent cells. The cells were then pooled with the non-adherent cell fraction.

### Flow cytometry

Before incubation with mAbs, the cells were blocked at 4 degree for 15 min with FACS staining buffer (Hanks' balanced salt solution containing 2% FBS and 10 mM HEPES) supplemented with 5 µg/ml CD16/CD32 (2.4G2) anti-FcR mAb, 1% normal rat serum, 1% normal hamster serum. All mAbs were purchased from BD Biosciences (Palo Alto, CA) except where noted. In addition to isotype controls, the following mAbs were used: CD1d (1B1), CD4 (H129.19), CD8α (53-6.7), CD11b (M1/70), CD11c (HL3), CD19 (1D3), CD24 (M1/69), CD25 (7D4), CD40 (3/23), CD45RA (14.8), CD48 (HM48-1), CD49b (DX5), CD80 (16-10A1), CD172a (P84), CD209 (5H10/CIRE), CD275 (HK5.3), H-2K^{b} (AF6-88.5), Ia^{b} (AF6-120.1), B220 (RA3-6B2), Gr-1 (RB6-8C5), Ly6-C (AL-21), Ly6-G (1A8), Sca-1 (D7), and Ly86 (MD14). F4/80 (BM8), CD34 (RAM34), CD135 (A2F10), and PDCA-1 (BST2) were obtained from eBioscience (San Diego, CA). CD205 (NLDC-145) and CD49a (HMalphal) were purchased from Miltenybiotec (Auburn, CA) and AbD Serotec (Oxford, UK), respectively. To exclude dead cells from analysis, propidium iodide (Invitrogen, San Diego, CA) or DAPI (Pierce, Rockford, IL) were added in the last wash to exclude dead cells from analysis. Stained cells were acquired using FACSCalibur or FACSAria (both BD Biosciences) and events were analyzed with CellQuest, FACSDiva (both BD Biosciences), or Weasel software (The Walter and Eliza Hall Institute of Medical Research, Melbourne, Australia).

### Cell isolation and culture

Cells were sorted from BM cultures propagated from pIL1β-dsRed transgenic mice or wild-type mice by flow cytometry using FACSAria. Cells were stained in FACS staining buffer with anti-CD 11b-APC-Cy7 and -CD11c-APC mAbs and sorted into CD11b^{□□}CD11c^{□□}DsRed^{□}, CD11b^{□□}CD11c^{□□}DsRed^{□}, CD11b^{□□}CD11c^{□□}DsRed^{□}, and CD11b^{□□}CD11c^{□□}DsRed^{□} fractions. Alternatively, the DC.com population was FACS-purified based on the surface phenotype of CD11b+/CD48-/Ly6G+.

The sorted cell fractions were cultured again with cRPMI in the presence of 10 ng/ml GM-CSF followed by flow cytometry analysis. In some experiments, DC.com population was purified from BM cultures of C57BL/6 mice, which are CD45.2⁺, then co-cultured with freshly isolated BM cells from B6/SJL mice, which are CD45.1⁺. The cells derived from the DC.com were distinguished from the feeders by differential staining with anti-CD45.2 and anti-CD45.1 antibodies. In this co-culture system, the DC.com population remained viable and differentiated into CD11c+/MHCII+ DC.

### Phenotypic and functional analyses

For the morphological study, cytospin preparations from sorted cells (2 x 10⁵ cells/slide) were stained with Giemsa solution (Sigma-Aldrich). The proliferation activity for sorted cells was examined using BrdU Flow Kit (BD Biosciences), following by the manufacture's instruction. Briefly, cells were incubated with BrdU for 18 h at 37 degree, followed by fixation and permiabilization. Cells were stained with FITC-conjugated anti-BrdU antibody in combination with 7-AAD for flow cytometry analysis. To test the ability of antigen uptake, sorted cells were incubated for 10 min with 5 micro-g/ml of FITC-conjugated dextran (70,000 Da molecular weight; Sigma) at 4°Cor 37°C, washed extensively, and then examined for FITC signals within the cells using FACSCaliber.

Sorted cells from BM cultures were pulsed with ovalbumin (OVA), OVA₃₂₃₋₃₃₉, or OVA₃₂₃₋₃₃₉ peptide at indicated concentrations for 1 h before co-culture with T cells. Naive CD4⁺ and CD8⁺ T cells were enriched from spleen of OVA-specific T cell receptor (TCR)-transgenic OT-I or OT-II mice, respectively, by immunomagnetic cell separation using negative isolation kits for CD4⁺ or CD8⁺ T cells (Dynal, Lake Success, NY). The percentage of enriched T cells expressing transgenic TCR was determined by flow cytometry (>90%), using anti-CD4-APC or anti-CD8-APC and anti-Vα2-FITC mAb (BD PharMingen).

For proliferation assays, naive T cells at 2 x 10⁴ cells /well were seeded into 96-U-well cell culture plates (Corning) and cultured with varying numbers of protein- or peptide-pulsed BM cell populations in cRPMI (200 µl/well). Cultures were maintained for 72 h at 37°C. The proliferation was assayed by pulsing the cells with 1 µCi of [³H]-thymidine (ICN Biomedicals, Costa Mesa, CA) for 16 h. At the end of incubation, the cells were harvested onto glass-fiber filters (Packard, Meriden, CT), and radioactivity was counted in a TopCount NTX (PerkinElmer, Shelton, CT).

### Statistical analyses

Data are expressed as means ± □S.D. Differences in measured variables between the experimental and control groups were assessed with two-tailed student's *t* test, except for the analysis of dose response, which was done using an analysis of variance (ANOVA) and Dunnett's test. Comparisons among more than three groups were assessed by Student-Newman-Keuls (SNK) multiple comparison test. Each experiment was repeated at least once to assess reproducibility.

### Visualization of DsRed signals in the skin

To induce pathological conditions, pIL1β-dsRed transgenic mice were received by topical application of 1.25% oxazolone (Sigma-Aldrich) or vehicle (acetone/olive oil) alone on the ear. In some experiments, the pathological conditions were induced on the ear by tape stripping. On day 1, whole ear skin samples were harvested to examine DsRed expression using a Zeiss LSM 510 META 2P confocal microscope. Epidermal cell suspensions and dermal cell suspensions were prepared by enzymatic treatment after separating the two compartments at the dermo-epidermal junction.

### EXAMPLES of USES and DEFINITIONS THEREOF

The practice of the present invention will employ, unless otherwise indicated, conventional methods of pharmacology, chemistry, biochemistry, recombinant DNA techniques and immunology, within the skill of the art. Such techniques are explained fully in the literature. See, e.g., Handbook of Experimental Immunology, Vols. I-IV (D. M. Weir and C. C. Blackwell eds., Blackwell Scientific Publications); A. L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.).

As such, the definitions herein are provided for further explanation and are not to be construed as limiting.

The articles "a" and "an" are used herein to refer to one or to more than one (i.e., to at least one) of the grammatical object of the article. By way of example, "an element" means one element or more than one element.

A "marker" and "biomarker" is molecule and/or functional variants thereof whose altered level of expression in a tissue or cell from its expression level in normal or healthy tissue or cell is associated with a disorder and/or disease state.

The "normal" level of expression of a marker is the level of expression of the marker in cells of a human subject or patient not afflicted with a disorder and/or disease state.

An "over-expression" or "significantly higher level of expression" of a marker refers to an expression level in a test sample that is greater than the standard error of the assay employed to assess expression, and in certain embodiments, at least twice, and in other embodiments, three, four, five or ten times the expression level of the marker in a control sample (e.g., sample from a healthy subject not having the marker associated disorder and/or disease state) and in certain embodiments, the average expression level of the marker in several control samples.

A "significantly lower level of expression" of a marker refers to an expression level in a test sample that is at least twice, and in certain embodiments, three, four, five or ten times lower than the expression level of the marker in a control sample (e.g., sample from a healthy subject not having the marker associated disorder and/or disease state) and in certain embodiments, the average expression level of the marker in several control samples.

### Examples of Uses

The compositions, kits and methods described herein have the following non-limiting uses, among others:
assessing whether a subject is afflicted with a disorder and/or disease state;
assessing the stage of a disorder and/or disease state in a subject;
assessing the grade of a disorder and/or disease state in a subject;
assessing the nature of a disorder and/or disease state in a subject;
assessing the potential to develop a disorder and/or disease state in a subject;
assessing the histological type of cells associated with a disorder and/or disease state in a subject;
making antibodies, antibody fragments or antibody derivatives that are useful for treating a disorder and/or disease state in a subject;
assessing the presence of a disorder and/or disease state in a subject's cells;
assessing the efficacy of one or more test compounds for inhibiting a disorder and/or disease state in a subject;
assessing the efficacy of a therapy for inhibiting a disorder and/or disease state in a subject;
monitoring the progression of a disorder and/or disease state in a subject;
selecting a composition or therapy for inhibiting a disorder and/or disease state in a subject;
treating a subject afflicted with a disorder and/or disease state;
inhibiting a disorder and/or disease state in a subject;
assessing the harmful potential of a test compound; and
preventing the onset of a disorder and/or disease state in a subject at risk therefor.

### Screening Methods

Screening methods are also within the contemplated scope of the present invention. In one non-limiting example, the method of screening for a therapeutic agent for a disorder and/or disease can be carried out either *in vivo* or *in vitro.* This screening method can be performed, for example, by:
administering a candidate compound to an animal subject;
measuring the expression level of at least one marker in a biological sample from the animal subject; or
selecting a compound that increases or decreases the expression level of the marker as compared to that in a control with which the candidate compound has not been contacted.

In still another aspect, there is provided herein a method to assess the efficacy of a candidate compound for a pharmaceutical agent on the expression level of at least one marker by contacting an animal subject with the candidate compound and monitoring the effect of the compound on the expression level of the marker in a biological sample derived from the animal subject. The variation in the expression level of the marker in a biological sample derived from the animal subject can be monitored using the same technique as used in the testing method described above. Furthermore, based on the evaluation, a candidate compound for a pharmaceutical agent can be selected by screening.

Animal models can be created to enable screening of therapeutic agents useful for treating or preventing a disorder and/or disease state in a subject. Accordingly, the methods are useful for identifying therapeutic agents for treating or preventing a disorder and/or disease state in a subject. The methods comprise administering a candidate agent to an animal model made by the methods described herein, and assessing at least one response in the animal model as compared to a control animal model to which the candidate agent has not been administered. If at least one response is reduced in symptoms or delayed in onset, the candidate agent is an agent for treating or preventing the disease.

The candidate agents may be pharmacologic agents already known in the art or may be agents previously unknown to have any pharmacological activity. The agents may be naturally arising or designed in the laboratory. They may be isolated from microorganisms, animals or plants, or may be produced recombinantly, or synthesized by any suitable chemical method. They may be small molecules, nucleic acids, proteins, peptides or peptidomimetics. In certain embodiments, candidate agents are small organic compounds having a molecular weight of more than 50 and less than about 2,500 daltons. Candidate agents comprise functional groups necessary for structural interaction with proteins. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. There are, for example, numerous means available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. In certain embodiments, the candidate agents can be obtained using any of the numerous approaches in combinatorial library methods art, including, by non-limiting example: biological libraries; spatially addressable parallel solid phase or solution phase libraries; synthetic library methods requiring deconvolution; the "one-bead one-compound" library method; and synthetic library methods using affinity chromatography selection.

In certain further embodiments, certain pharmacological agents may be subjected to directed or random chemical modifications, such as acylation, alkylation, esterification, amidification, etc. to produce structural analogs.

The same methods for identifying therapeutic agents for treating a disorder and/or disease state in a subject can also be used to validate lead compounds/agents generated from *in vitro* studies.

The candidate agent may be an agent that up- or down-regulates one or more of a disorder and/or disease state in a subject response pathway. In certain embodiments, the candidate agent may be an antagonist that affects such pathway.

Methods for Treating a Disorder and/or Disease State

Methods for treating, inhibiting, relieving or reversing a disorder and/or disease state response is also within the contemplated scope of the present invention. In one non-limiting example, an agent that interferes with a signaling cascade is administered to an individual in need thereof, such as, but not limited to, subjects in whom such complications are not yet evident and those who already have at least one such response.

In the former instance, such treatment is useful to prevent the occurrence of such response and/or reduce the extent to which they occur. In the latter instance, such treatment is useful to reduce the extent to which such response occurs, prevent their further development or reverse the response.

In certain embodiments, the agent that interferes with the response cascade may be an antibody specific for such response.

### Expression of Marker(s)

Expression of a marker can be inhibited in a number of ways, including, by way of a non-limiting example, an antisense oligonucleotide can be provided to cells in order to inhibit transcription, translation, or both, of the marker(s). Alternately, a polynucleotide encoding an antibody, an antibody derivative, or an antibody fragment which specifically binds a marker protein, and operably linked with an appropriate promoter/regulator region, can be provided to the cell in order to generate intracellular antibodies which will inhibit the function or activity of the protein. The expression and/or function of a marker may also be inhibited by treating the cell with an antibody, antibody derivative or antibody fragment that specifically binds a marker protein. Using the methods described herein, a variety of molecules, particularly including molecules sufficiently small that they are able to cross the cell membrane, can be screened in order to identify molecules which inhibit expression of a marker or inhibit the function of a marker protein. The compound so identified can be provided to the subject in order to inhibit the disorder and/or disease state of the subject.

Any marker or combination of markers, as well as any certain markers in combination with the markers, may be used in the compositions, kits and methods described herein. In general, it is desirable to use markers for which the difference between the level of expression of the marker in target cells and the level of expression of the same marker in normal cells is as great as possible. Although this difference can be as small as the limit of detection of the method for assessing expression of the marker, it is desirable that the difference be at least greater than the standard error of the assessment method, and, in certain embodiments, a difference of at least 2-, 3-, 4-, 5-, 6-, 7-, 8-, 9-, 10-, 15-, 20-, 100-, 500-, 1000-fold or greater than the level of expression of the same marker in normal tissues/cells.

Because the compositions, kits, and methods rely on detection of a difference in expression levels of one or more markers, it is desired that the level of expression of the marker is significantly greater than the minimum detection limit of the method used to assess expression in at least one of normal cells and target cells.

It is understood that by routine screening of additional subject samples using one or more of the markers, it will be realized that certain of the markers are over-expressed in cells of various types, including a specific disorder and/or disease state in a subject.

In addition, as a greater number of subject samples are assessed for expression of the markers and the outcomes of the individual subjects from whom the samples were obtained are correlated, it will also be confirmed that altered expression of certain of the markers are strongly correlated with a disorder and/or disease state in a subject and that altered expression of other markers are strongly correlated with other diseases. The compositions, kits, and methods are thus useful for characterizing one or more of the stage, grade, histological type, and nature of a disorder and/or disease state in a subject.

When the compositions, kits, and methods are used for characterizing one or more of the stage, grade, histological type, and nature of a disorder and/or disease state in a subject, it is desired that the marker or panel of markers is selected such that a positive result is obtained in at least about 20%, and in certain embodiments, at least about 40%, 60%, or 80%, and in substantially all subjects afflicted with a disorder and/or disease state of the corresponding stage, grade, histological type, or nature. The marker or panel of markers invention can be selected such that a positive predictive value of greater than about 10% is obtained for the general population (in a non-limiting example, coupled with an assay specificity greater than 80%).

When a plurality of markers is used in the compositions, kits, and methods, the level of expression of each marker in a subject sample can be compared with the normal level of expression of each of the plurality of markers in non-disorder and/or non-disease samples of the same type, either in a single reaction mixture (i.e. using reagents, such as different fluorescent probes, for each marker) or in individual reaction mixtures corresponding to one or more of the markers.

In order to maximize the sensitivity of the compositions, kits, and methods (i.e. by interference attributable to cells of system origin in a subject sample), it is desirable that the marker used therein be a marker which has a restricted tissue distribution, e.g., normally not expressed in a non-system tissue.

It is recognized that the compositions, kits, and methods will be of particular utility to subjects having an enhanced risk of developing a disorder and/or disease state in a subject and their medical advisors. Subjects recognized as having an enhanced risk of developing a disorder and/or disease include, for example, subjects having a familial history of such disorder or disease.

The level of expression of a marker in normal human system tissue can be assessed in a variety of ways. In one embodiment, this normal level of expression is assessed by assessing the level of expression of the marker in a portion of system cells which appears to be normal and by comparing this normal level of expression with the level of expression in a portion of the system cells which is suspected of being abnormal. Alternately, and particularly as further information becomes available as a result of routine performance of the methods described herein, population-average values for normal expression of the markers may be used. In other embodiments, the 'normal' level of expression of a marker may be determined by assessing expression of the marker in a subject sample obtained from a non-afflicted subject, from a subject sample obtained from a subject before the suspected onset of a disorder and/or disease state in the subject, from archived subject samples, and the like.

There is also provided herein compositions, kits, and methods for assessing the presence of disorder and/or disease state cells in a sample (e.g., an archived tissue sample or a sample obtained from a subject). These compositions, kits, and methods are substantially the same as those described above, except that, where necessary, the compositions, kits, and methods are adapted for use with samples other than subject samples. For example, when the sample to be used is a parafinized, archived human tissue sample, it can be necessary to adjust the ratio of compounds in the compositions, in the kits, or the methods used to assess levels of marker expression in the sample.

### Kits and Reagents

A kit can be any manufacture (e.g. a package or container) comprising at least one reagent, e.g., a probe, for specifically detecting the expression of a marker. The kit may be promoted, distributed or sold as a unit for performing the methods of the present invention.

The kits are useful for assessing the presence target cells (e.g., in a sample such as a subject sample). The kit comprises a plurality of reagents, each of which is capable of binding specifically with a marker nucleic acid or protein. Suitable reagents for binding with a marker protein include antibodies, antibody derivatives, antibody fragments, and the like. Suitable reagents for binding with a marker nucleic acid (e.g., a genomic DNA, an MRNA, a spliced MRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

The kits may optionally comprise additional components useful for performing the methods described herein. By way of example, the kit may comprise fluids (e.g. SSC buffer) suitable for annealing complementary nucleic acids or for binding an antibody with a protein with which it specifically binds, one or more sample compartments, an instructional material which describes performance of the method, a sample of normal system cells, a sample of affected cells, and the like.

### Methods of Producing Antibodies

Methods of producing an antibody useful for assessing whether a subject is afflicted with a disorder and/or disease state are also within the contemplated scope of the present invention. In one non-limiting example, a protein or peptide comprising the entirety or a segment of a marker protein is synthesized or isolated (e.g., by purification from a cell in which it is expressed or by transcription and translation of a nucleic acid encoding the protein or peptide *in vivo* or *in vitro*)*.* A vertebrate, for example, a mammal such as a mouse, rat, rabbit, or sheep, is immunized using the protein or peptide. The vertebrate may optionally (and preferably) be immunized at least one additional time with the protein or peptide, so that the vertebrate exhibits a robust immune response to the protein or peptide. Splenocytes are isolated from the immunized vertebrate and fused with an immortalized cell line to form hybridomas, using any of a variety of methods. Hybridomas formed in this manner are then screened using standard methods to identify one or more hybridomas which produce an antibody which specifically binds with the marker protein or a fragment thereof. There is also provided herein hybridomas made by this method and antibodies made using such hybridomas.

### Methods of Assessing Efficacy

Methods of assessing the efficacy of a test compound for inhibiting target cells are also within the contemplated scope of the present invention. In one non-limiting example, as described herein, differences in the level of expression of the markers correlate with the abnormal state of the subject's cells. Although it is recognized that changes in the levels of expression of certain of the markers likely result from the abnormal state of such cells, it is likewise recognized that changes in the levels of expression of other of the markers induce, maintain, and promote the abnormal state of those cells. Thus, compounds which inhibit a disorder and/or disease state in a subject will cause the level of expression of one or more of the markers to change to a level nearer the normal level of expression for that marker (i.e., the level of expression for the marker in normal cells).

The method can include comparing expression of a marker in a first cell sample and maintained in the presence of the test compound and expression of the marker in a second cell sample and maintained in the absence of the test compound. A significantly reduced expression of a marker in the presence of the test compound is an indication that the test compound inhibits a disorder or related disease state. The cell samples may, for example, be aliquots of a single sample of normal cells obtained from a subject, pooled samples of normal cells obtained from a subject, cells of a normal cell line, aliquots of a single sample of related disease cells obtained from a subject, pooled samples of related target cells obtained from a subject, cells of a disorder or disease cell line, or the like.

In one embodiment, the samples are affected cells obtained from a subject and a plurality of compounds believed to be effective for inhibiting various inflammatory-related disorders and/or diseases are tested in order to identify the compound which is likely to best inhibit the disorder and/or disease in the subject.

This method may likewise be used to assess the efficacy of a therapy for inhibiting a related disorder and/or disease in a subject. In this method, the level of expression of one or more markers in a pair of samples (one subjected to the therapy, the other not subjected to the therapy) is assessed. As with the method of assessing the efficacy of test compounds, if the therapy induces a significantly lower level of expression of a marker, then the therapy is efficacious for inhibiting a disorder and/or disease. As above, if samples from a selected subject are used in this method, then alternative therapies can be assessed *in vitro* in order to select a therapy most likely to be efficacious for inhibiting a disorder and/or disease in the subject.

As described herein, the abnormal state of human cells is correlated with changes in the levels of expression of the markers. There is also provided a method for assessing the harmful potential of a test compound. This method comprises maintaining separate aliquots of human cells in the presence and absence of the test compound. Expression of a marker in each of the aliquots is compared. A significantly higher level of expression of a marker in the aliquot maintained in the presence of the test compound (relative to the aliquot maintained in the absence of the test compound) is an indication that the test compound possesses a harmful potential. The relative harmful potential of various test compounds can be assessed by comparing the degree of enhancement or inhibition of the level of expression of the relevant markers, by comparing the number of markers for which the level of expression is enhanced or inhibited, or by comparing both. Various aspects are described in further detail in the following subsections.

### Isolated Proteins and Antibodies

Isolated marker proteins and biologically active portions thereof, as well as polypeptide fragments suitable for use as immunogens to raise antibodies directed against a marker protein or a fragment thereof, are also within the contemplated scope of the present invention.

In one non-limiting example, the native marker protein can be isolated from cells or tissue sources by an appropriate purification scheme using standard protein purification techniques. In another embodiment, a protein or peptide comprising the whole or a segment of the marker protein is produced by recombinant DNA techniques. Alternative to recombinant expression, such protein or peptide can be synthesized chemically using standard peptide synthesis techniques.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein").

When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or compounds other than the polypeptide of interest.

Biologically active portions of a marker protein include polypeptides comprising amino acid sequences sufficiently identical to or derived from the amino acid sequence of the marker protein, which include fewer amino acids than the full length protein, and exhibit at least one activity of the corresponding full-length protein. Typically, biologically active portions comprise a domain or motif with at least one activity of the corresponding full-length protein. A biologically active portion of a marker protein can be a polypeptide which is, for example, 10, 25, 50, 100 or more amino acids in length. Moreover, other biologically active portions, in which other regions of the marker protein are deleted, can be prepared by recombinant techniques and evaluated for one or more of the functional activities of the native form of the marker protein. In certain embodiments, useful proteins are substantially identical (e.g., at least about 40%, and in certain embodiments, 50%, 60%, 70%, 80%, 90%, 95%, or 99%) to one of these sequences and retain the functional activity of the corresponding naturally-occurring marker protein yet differ in amino acid sequence due to natural allelic variation or mutagenesis.

In addition, libraries of segments of a marker protein can be used to generate a variegated population of polypeptides for screening and subsequent selection of variant marker proteins or segments thereof.

### Predictive Medicine

Uses of animal models and markers in the field of predictive medicine in which diagnostic assays, prognostic assays, pharmacogenomics, and monitoring clinical trials are used for prognostic (predictive) purposes to thereby treat an individual prophylactically are also within the contemplated scope of the present invention. In one non-limiting example, diagnostic assays are used for determining the level of expression of one or more marker proteins or nucleic acids, in order to determine whether an individual is at risk of developing a particular disorder and/or disease. Such assays can be used for prognostic or predictive purposes to thereby prophylactically treat an individual prior to the onset of the disorder and/or disease.

In another aspect, the methods are useful for at least periodic screening of the same individual to see if that individual has been exposed to chemicals or toxins that change his/her expression patterns.

Yet another aspect pertains to monitoring the influence of agents (e.g., drugs or other compounds) administered either to inhibit a disorder and/or disease or to treat or prevent any other disorder and/or disease (e.g., in order to understand any system effects that such treatment may have) on the expression or activity of a marker in clinical trials.

### Pharmaceutical Compositions

Pharmaceutical compositions are also within the contemplated scope of the present invention. In one non-limiting example, the compounds may be in a formulation for administration topically, locally or systemically in a suitable pharmaceutical carrier. Remington's Pharmaceutical Sciences, 15th Edition by E. W. Martin (Mark Publishing Company, 1975), discloses typical carriers and methods of preparation. The compound may also be encapsulated in suitable biocompatible microcapsules, microparticles or microspheres formed of biodegradable or non-biodegradable polymers or proteins or liposomes for targeting to cells. Such systems are well known to those skilled in the art and may be optimized for use with the appropriate nucleic acid.

As another example, the pharmaceutical compositions can be comprised of various methods for nucleic acid delivery described in, for example, Sambrook et al., 1989, Molecular Cloning: A Laboratory Manual, Cold Spring Harbor Laboratory, New York; and Ausubel et al., 1994, Current Protocols in Molecular Biology, John Wiley & Sons, New York. Such nucleic acid delivery systems comprise the desired nucleic acid, by way of example and not by limitation, in either "naked" form as a "naked" nucleic acid, or formulated in a vehicle suitable for delivery, such as in a complex with a cationic molecule or a liposome forming lipid, or as a component of a vector, or a component of a pharmaceutical composition. The nucleic acid delivery system can be provided to the cell either directly, such as by contacting it with the cell, or indirectly, such as through the action of any biological process.

Formulations for topical administration may include ointments, lotions, creams, gels, drops, suppositories, sprays, liquids and powders. Conventional pharmaceutical carriers, aqueous, powder or oily bases, or thickeners can be used as desired.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions, solutions or emulsions that can include suspending agents, solubilizers, thickening agents, dispersing agents, stabilizers, and preservatives. Formulations for injection may be presented in unit dosage form, e.g., in ampules or in multi-dose containers, with an added preservative. Those of skill in the art can readily determine the various parameters for preparing and formulating the compositions without resort to undue experimentation. The compound can be used alone or in combination with other suitable components.

In general, methods of administering compounds are well known in the art. In particular, the routes of administration already in use for various therapeutics, along with formulations in current use, provide preferred routes of administration and formulation will depend, of course, upon factors such as the particular formulation, the severity of the state of the subject being treated, and the dosage required for therapeutic efficacy. As generally used herein, an "effective amount" is that amount which is able to treat one or more symptoms of the disorder, reverse the progression of one or more symptoms of the disorder and/or disease, halt the progression of one or more symptoms of the disorder and/or disease, or prevent the occurrence of one or more symptoms of the disorder and/or disease in a subject to whom the formulation is administered, as compared to a matched subject not receiving the compound. The actual effective amounts of compound can vary according to the specific compound or combination thereof being utilized, the particular composition formulated, the mode of administration, and the age, weight, condition of the individual, and severity of the symptoms or condition being treated.

Any acceptable method known to one of ordinary skill in the art may be used to administer a formulation to the subject. The administration may be localized (i.e., to a particular region, physiological system, tissue, organ, or cell type) or systemic, depending on the condition being treated.

### Pharmacogenomics

Pharmacogenomic markers are also within the contemplated scope of the present invention. In one non-limiting example, a "pharmacogenomic marker" is an objective biochemical marker whose expression level correlates with a specific clinical drug response or susceptibility in a subject. The presence or quantity of the pharmacogenomic marker expression is related to the predicted response of the subject, and more particularly the subject's tumor, to therapy with a specific drug or class of drugs. By assessing the presence or quantity of the expression of one or more pharmacogenomic markers in a subject, a drug therapy which is most appropriate for the subject, or which is predicted to have a greater degree of success, may be selected.

### Monitoring Clinical Trials

Monitoring the influence of agents (e.g., drug compounds) on the level of expression of a marker is also within the contemplated scope of the present invention. In one non-limiting example, the monitoring can be applied not only in basic drug screening, but also in clinical trials. For example, the effectiveness of an agent to affect marker expression can be monitored in clinical trials of subjects receiving treatment for a disorder and/or disease.

In one non-limiting embodiment, the present invention provides a method for monitoring the effectiveness of treatment of a subject with an agent (e.g., an agonist, antagonist, peptidomimetic, protein, peptide, nucleic acid, small molecule, or other drug candidate) comprising the steps of:
obtaining a pre-administration sample from a subject prior to administration of the agent;
detecting the level of expression of one or more selected markers in the pre-administration sample;
obtaining one or more post-administration samples from the subject;
detecting the level of expression of the marker(s) in the post-administration samples;
comparing the level of expression of the marker(s) in the pre-administration sample with the level of expression of the marker(s) in the post-administration sample or samples; and
altering the administration of the agent to the subject accordingly.

For example, increased expression of the marker during the course of treatment may indicate ineffective dosage and the desirability of increasing the dosage. Conversely, decreased expression of the marker may indicate efficacious treatment and no need to change dosage.

Electronic Apparatus Readable Media, Systems, Arrays and Methods of Using Same

Electronic apparatus readable media, systems, arrays and method of using the same are also within the contemplated scope of the present invention. In one non-limiting example, "electronic apparatus readable media" refers to any suitable medium for storing, holding or containing data or information that can be read and accessed directly by an electronic apparatus. Such media can include, but are not limited to: magnetic storage media, such as floppy discs, hard disc storage medium, and magnetic tape; optical storage media such as compact discs; electronic storage media such as RAM, ROM, EPROM, EEPROM and the like; and general hard disks and hybrids of these categories such as magnetic/optical storage media. The medium is adapted or configured for having recorded thereon a marker as described herein.

As used herein, the term "electronic apparatus" is intended to include any suitable computing or processing apparatus or other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phone, pager and the like; and local and distributed processing systems.

As used herein, "recorded" refers to a process for storing or encoding information on the electronic apparatus readable medium. Those skilled in the art can readily adopt any method for recording information or media to generate materials comprising the markers described herein.

A variety of software programs and formats can be used to store the marker information of the present invention on the electronic apparatus readable medium. Any number of data processor structuring formats (e.g., text file or database) may be employed in order to obtain or create a medium having recorded thereon the markers. By providing the markers in readable form, one can routinely access the marker sequence information for a variety of purposes. For example, one skilled in the art can use the nucleotide or amino acid sequences in readable form to compare a target sequence or target structural motif with the sequence information stored within the data storage means. Search means are used to identify fragments or regions of the sequences which match a particular target sequence or target motif.

Thus, there is also provided herein a medium for holding instructions for performing a method for determining whether a subject has a disorder and/or disease or a pre-disposition to a disorder and/or disease, wherein the method comprises the steps of determining the presence or absence of a marker and based on the presence or absence of the marker, determining whether the subject has a disorder and/or disease or a pre-disposition thereto and/or recommending a particular treatment for such disorder and/or disease or predisposition to such disorder and/or disease condition.

There is also provided herein an electronic system and/or in a network, a method for determining whether a subject has a disorder and/or disease or a pre-disposition thereto associated with a marker wherein the method comprises the steps of determining the presence or absence of the marker, and based on the presence or absence of the marker, determining whether the subject has a particular disorder and/or disease or a pre-disposition to such disorder and/or disease, and/or recommending a particular treatment for such disease or disorder and/or such preconditions for the disorder and/or disease condition. The method may further comprise the step of receiving phenotypic information associated with the subject and/or acquiring from a network phenotypic information associated with the subject.

Also provided herein is a network, a method for determining whether a subject has a disorder and/or disease or a pre-disposition to a disorder and/or disease associated with a marker, the method comprising the steps of receiving information associated with the marker, receiving phenotypic information associated with the subject, acquiring information from the network corresponding to the marker and/or disorder and/or disease, and based on one or more of the phenotypic information, the marker, and the acquired information, determining whether the subject has a disorder and/or disease or a pre-disposition thereto. The method may further comprise the step of recommending a particular treatment for the disorder and/or disease or pre-disposition thereto.

There is also provided herein a business method for determining whether a subject has a disorder and/or disease or a pre-disposition thereto, the method comprising the steps of receiving information associated with the marker, receiving phenotypic information associated with the subject, acquiring information from the network corresponding to the marker and/or a disorder and/or disease, and based on one or more of the phenotypic information, the marker, and the acquired information, determining whether the subject has a disorder and/or disease or a pre-disposition thereto. The method may further comprise the step of recommending a particular treatment therefor.

There is also provided herein an array that can be used to assay expression of one or more markers in the array. In one embodiment, the array can be used to assay expression in a tissue to ascertain tissue specificity of the markers in the array. In this manner, up to about 7000 or more markers can be simultaneously assayed for expression. This allows a profile to be developed showing a battery of markers specifically expressed in one or more tissues.

In addition to such qualitative determination, there is provided herein the quantitation of such expression. Thus, not only tissue specificity, but also the level of expression in the tissue is ascertainable. Thus, markers can be grouped on the basis of their tissue expression per se and level of expression in that tissue. This is useful, for example, in ascertaining the relationship of expression between or among tissues. Thus, one tissue can be perturbed and the effect on expression in a second tissue can be determined. In this context, the effect of one cell type on another cell type in response to a biological stimulus can be determined.

Such a determination is useful, for example, to know the effect of cell-cell interaction at the level of expression. If an agent is administered therapeutically to treat one cell type but has an undesirable effect on another cell type, the method provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more markers in the array. This can occur in various biological contexts, as disclosed herein, for example, development of a disorder and/or disease, progression thereof, and processes, such as cellular transformation associated therewith.

The array is also useful for ascertaining the effect of the expression or the expression of other markers in the same cell or in different cells. This provides, for example, for a selection of alternate molecular targets for therapeutic intervention if the ultimate or downstream target cannot be regulated.

The array is also useful for ascertaining differential expression patterns of one or more markers in normal and abnormal cells. This provides a battery of markers that could serve as a molecular target for diagnosis or therapeutic intervention.

### Surrogate Markers

Surrogate markers are also within the contemplated scope of the present invention. In one non-limiting example, the markers may serve as surrogate markers for one or more disorders or disease states or for conditions leading up thereto. "Surrogate marker" can be an objective biochemical marker which correlates with the absence or presence of a disorder and/or disease, or with the progression of a disease and/or disorder. The presence or quantity of such markers is independent of the disorder and/or disease. Therefore, these markers may serve to indicate whether a particular course of treatment is effective in lessening a disorder and/or disease state. Surrogate markers are of particular use when the presence or extent of a disorder and/or disease state is difficult to assess through standard methodologies, or when an assessment of progression is desired before a potentially dangerous clinical endpoint is reached.

The markers are also useful as pharmacodynamic markers. As used herein, a "pharmacodynamic marker" is an objective biochemical marker which correlates specifically with drug effects. The presence or quantity of a pharmacodynamic marker is not related to the disorder and/or disease state for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug *in vivo.*

Pharmacodynamic markers are of particular use in increasing the sensitivity of detection of drug effects, particularly when the drug is administered in low doses. Since even a small amount of a drug may be sufficient to activate multiple rounds of marker transcription or expression, the amplified marker may be in a quantity which is more readily detectable than the drug itself. Also, the marker may be more easily detected due to the nature of the marker itself; for example, using the methods described herein, antibodies may be employed in an immune-based detection system for a protein marker, or marker-specific radiolabeled probes may be used to detect a mRNA marker. Furthermore, the use of a pharmacodynamic marker may offer mechanism-based prediction of risk due to drug treatment beyond the range of possible direct observations.

### Protocols for Testing

Protocols for testing are also within the contemplated scope of the present invention. In one non-limiting example, the method of testing for a disorder and/or disease may comprise, for example, measuring the expression level of each marker in a biological sample from a subject over time and comparing the level with that of the marker in a control biological sample.

When the marker is one of the markers described herein and the expression level is differentially expressed (for example, higher or lower than that in the control), the subject is judged to be affected with a disorder and/or disease. When the expression level of the marker falls within the permissible range, the subject is unlikely to be affected therewith.

The standard value for the control may be pre-determined by measuring the expression level of the marker in the control, in order to compare the expression levels. For example, the standard value can be determined based on the expression level of the above-mentioned marker; in certain embodiments, the permissible range is taken as ± 2S.D. based on the standard value. Once the standard value is determined, the testing method may be performed by measuring only the expression level in a biological sample from a subject and comparing the value with the determined standard value for the control. In another example, expression levels of marker include transcription of the marker to mRNA, and translation into proteins. Therefore, one method of testing for a disorder and/or disease is performed based on a comparison of the intensity of expression of mRNA corresponding to the marker genes, or the expression level of proteins encoded by the marker genes.

The measurement of the expression levels of marker in the testing for a disorder and/or disease can be carried out according to various analysis methods. For example, one can use a hybridization technique using nucleic acids that hybridize to these genes as probes, or a gene amplification technique using DNA that hybridize to the marker genes as primers.

The probes or primers used for the testing can be designed based on the nucleotide sequences of the marker. The identification numbers for the nucleotide sequences of the respective marker genes are described herein.

Further, it is to be understood that genes of higher animals generally accompany polymorphism in a high frequency. There are also many molecules that produce isoforms comprising mutually different amino acid sequences during the splicing process. Any marker associated with a disorder and/or disease that has an activity similar to that of a marker is included in the markers, even if it has nucleotide sequence differences due to polymorphism or being an isoform.

It is also to be understood that the marker can include homo logs of other species in addition to humans. Thus, unless otherwise specified, the expression "marker" refers to a homolog of the marker unique to the species or a foreign marker which has been introduced into an individual.

Also, it is to be understood that a "homolog of a marker" refers to a marker derived from a species other than a human, which can hybridize to the human marker as a probe under stringent conditions. Such stringent conditions are known to one skilled in the art who can select an appropriate condition to produce an equal stringency experimentally or empirically. For example, a polynucleotide comprising the nucleotide sequence of a marker or a nucleotide sequence that is complementary to the complementary strand of the nucleotide sequence of a marker and has at least 15 nucleotides, can be used as a primer or probe. Thus, a "complementary strand" means one strand of a double stranded DNA with respect to the other strand and which is composed of A:T (U for RNA) and G:C base pairs. In addition, "complementary" means not only those that are completely complementary to a region of at least 15 continuous nucleotides, but also those that have a nucleotide sequence homology of at least 40% in certain instances, 50% in certain instances, 60% in certain instances, 70% in certain instances, 80% in certain instances, 90% in certain instances, and 95% in certain instances, or higher. The degree of homology between nucleotide sequences can be determined by an algorithm, BLAST, etc. Such polynucleotides are useful as a probe to detect a marker, or as a primer to amplify a marker. When used as a primer, the polynucleotide comprises usually 15 bp to 100 bp, and in certain embodiments 15 bp to 35 bp of nucleotides. When used as a probe, a DNA comprises the whole nucleotide sequence of the marker gene (or the complementary strand thereof), or a partial sequence thereof that has at least 15 bp nucleotides. When used as a primer, the 3' region must be complementary to the marker gene, while the 5' region can be linked to a restriction enzyme-recognition sequence or a tag. "Polynucleotides" may be either DNA or RNA. These polynucleotides may be either synthetic or naturally-occurring. Also, DNA used as a probe for hybridization is usually labeled. Those skilled in the art readily understand such labeling methods. Herein, the term "oligonucleotide" means a polynucleotide with a relatively low degree of polymerization. Oligonucleotides are included in polynucleotides.

Tests for a disorder and/or disease using hybridization techniques can be performed using, for example, Northern hybridization, dot blot hybridization, or the DNA microarray technique. Furthermore, gene amplification techniques, such as the RT-PCR method may be used. By using the PCR amplification monitoring method during the amplification step in RT-PCR, one can achieve a more quantitative analysis of the expression of a marker.

In the PCR gene amplification monitoring method, the detection target (DNA or reverse transcript of RNA) is hybridized to probes that are labeled with a fluorescent dye and a quencher which absorbs the fluorescence. When the PCR proceeds and Taq polymerase degrades the probe with its 5'-3' exonuclease activity, the fluorescent dye and the quencher draw away from each other and the fluorescence is detected. The fluorescence is detected in real time. By simultaneously measuring a standard sample in which the copy number of a target is known, it is possible to determine the copy number of the target in the subject sample with the cycle number where PCR amplification is linear. Also, one skilled in the art recognizes that the PCR amplification monitoring method can be carried out using any suitable method.

The method of testing for a disorder and/or disease can be also carried out by detecting a protein encoded by a marker. Hereinafter, a protein encoded by a marker is described as a "marker protein." For such test methods, for example, the Western blotting method, the immunoprecipitation method, and the ELISA method may be employed using an antibody that binds to each marker protein. Antibodies used in the detection that bind to the marker protein may be produced by any suitable technique. Also, in order to detect a marker protein, such an antibody may be appropriately labeled. Alternatively, instead of labeling the antibody, a substance that specifically binds to the antibody, for example, protein A or protein G, may be labeled to detect the marker protein indirectly. More specifically, such a detection method can include the ELISA method. A protein or a partial peptide thereof used as an antigen may be obtained, for example, by inserting a marker or a portion thereof into an expression vector, introducing the construct into an appropriate host cell to produce a transformant, culturing the transformant to express the recombinant protein, and purifying the expressed recombinant protein from the culture or the culture supernatant. Alternatively, the amino acid sequence encoded by a marker or an oligopeptide comprising a portion of the amino acid sequence encoded by a full-length cDNA are chemically synthesized to be used as an immunogen.

Furthermore, a test for a disorder and/or disease can be performed using as an index not only for the expression level of a marker, but also for the activity of a marker protein in a biological sample. Activity of a marker protein means the biological activity intrinsic to the protein. Various methods can be used for measuring the activity of each protein.

Even if a subject is not diagnosed as being affected with a disorder and/or disease in a routine test in spite of symptoms suggesting such disorder and/or disease, whether or not such a subject is suffering from a disorder and/or disease can be easily determined by performing a test according to the methods described herein.

More specifically, in certain embodiments, when the marker is one of the markers described herein, an increase or decrease in the expression level of the marker in a subject whose symptoms suggest at least a susceptibility to a disorder and/or disease indicates that the symptoms are primarily caused thereby.

In addition, the tests are useful to determine whether a disorder and/or disease is improving in a subject. In other words, the methods described herein can be used to judge the therapeutic effect of a treatment therefor. Furthermore, when the marker is one of the genes described herein, an increase or decrease in the expression level of the marker in a subject, who has been diagnosed as being affected thereby, implies that the disorder and/or disease has progressed more.

The severity and/or susceptibility to a disorder and/or disease may also be determined based on the difference in expression levels. For example, when the marker e is one of the markers described herein, the degree of increase in the expression level of the marker is correlated with the presence and/or severity of a disorder and/or disease.

### Animal Models

Animal models for a disorder and/or disease where the expression level of one or more marker or a marker functionally equivalent to the marker has been elevated in the animal model are also within the contemplated scope of the present invention. In one nonlimiting example, a "functionally equivalent marker" as used herein generally is a marker having an activity similar to a known activity of the marker.

The animal model is useful for detecting physiological changes due to a disorder and/or disease. In certain embodiments, the animal model is useful to reveal additional functions of markers and to evaluate drugs whose targets are the markers.

An animal model can be created by controlling the expression level of a counterpart gene or administering a counterpart gene. The method can include creating an animal model by controlling the expression level of a gene. In another embodiment, the method can include creating an animal model by administering the protein encoded by a gene, or administering an antibody against the protein. It is to be also understood, that in certain other embodiments, the marker can be over-expressed such that the marker can then be measured using appropriate methods. In another embodiment, an animal model can be created by introducing a gene selected from such groups of genes, or by administering a protein encoded by such a gene. In another embodiment, a disorder and/or disease can be induced by suppressing the expression of a gene selected from such groups of genes or the activity of a protein encoded by such a gene. An antisense nucleic acid, a ribozyme, or an RNAi can be used to suppress the expression. The activity of a protein can be controlled effectively by administering a substance that inhibits the activity, such as an antibody.

The animal model is useful to elucidate the mechanism underlying a disorder and/or disease and also to test the safety of compounds obtained by screening. For example, when an animal model develops the symptoms of a particular disorder and/or disease, or when a measured value involved in a certain disorder and/or disease alters in the animal, a screening system can be constructed to explore compounds having activity to alleviate the disorder and/or disease.

As used herein, the expression "an increase in the expression level" refers to any one of the following: where a marker gene introduced as a foreign gene is expressed artificially; where the transcription of a marker gene intrinsic to the subject animal and the translation thereof into the protein are enhanced; or where the hydrolysis of the protein, which is the translation product, is suppressed.

As used herein, the expression "a decrease in the expression level" refers to either the state in which the transcription of a marker of the subject animal and the translation thereof into the protein are inhibited, or the state in which the hydrolysis of the protein, which is the translation product, is enhanced. The expression level of a gene can be determined, for example, by a difference in signal intensity on a DNA chip. Furthermore, the activity of the translation product--the protein--can be determined by comparing with that in the normal state.

It is also within the contemplated scope that the animal model can include transgenic animals, including, for example animals where a marker gene has been introduced and expressed artificially; marker gene knockout animals; and knock-in animals in which another gene has been substituted for a marker gene. A transgenic animal, into which an antisense nucleic acid of a marker gene, a ribozyme, a polynucleotide having an RNAi effect, or a DNA functioning as a decoy nucleic acid or such has been introduced, can be used as the transgenic animal. Such transgenic animals also include, for example, animals in which the activity of a marker protein has been enhanced or suppressed by introducing a mutation(s) into the coding region of the gene, or the amino acid sequence has been modified to become resistant or susceptible to hydrolysis. Mutations in an amino acid sequence include substitutions, deletions, insertions, and additions.

"Subject" means a human or non-human animal selected for treatment or therapy. "Subject suspected of having" means a subject exhibiting one or more clinical indicators of a disorder, disease or condition.

"Preventing" or "prevention" refers to delaying or forestalling the onset, development or progression of a condition or disorder and/or disease for a period of time, including weeks, months, or years. "Treatment" or "treat" means the application of one or more specific procedures used for the cure or amelioration of a disorder and/or disease. In certain embodiments, the specific procedure is the administration of one or more pharmaceutical agents.

"Amelioration" means a lessening of severity of at least one indicator of a disorder and/or disease. In certain embodiments, amelioration includes a delay or slowing in the progression of one or more indicators of a disorder and/or disease. The severity of indicators may be determined by subjective or objective measures which are known to those skilled in the art.

"Subject in need thereof' means a subject identified as in need of a therapy or treatment.

"Administering" means providing a pharmaceutical agent or composition to a subject, and includes, but is not limited to, administering by a medical professional and self-administering.

"Improves function" means the changes function toward normal parameters. In certain embodiments, function is assessed by measuring molecules found in a subject.

"Modulation" means a perturbation of function or activity. In certain embodiments, modulation means an increase in gene expression. In certain embodiments, modulation means a decrease in gene expression.

"Expression" means any functions and steps by which a gene's coded information is converted into structures present and operating in a cell.

The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims. It will also be readily apparent to a person skilled in the art that varying substitutions and modifications may be made to the invention disclosed herein without departing from the scope and spirit of the invention.

It should be understood that although the present invention has been specifically disclosed by preferred embodiments and optional features, modifications and variations of the concepts herein disclosed may be resorted to by those skilled in the art, and that such modifications and variations are considered to be within the scope of this invention as defined by the appended claims.

While the invention has been described and exemplified in sufficient detail for those skilled in this art to make and use it, various alternatives, modifications and improvements should be apparent without departing from the scope of the invention. In addition, many modifications may be made to adapt a particular situation or material to the teachings of the invention without departing from the essential scope thereof.

### REFERENCES

The publication and other material used herein to illuminate the invention or provide additional details respecting the practice of the invention, are incorporated by reference herein, and for convenience are provided in the following bibliography.

Citation of the any of the documents recited herein is not intended as an admission that any of the foregoing is pertinent prior art. All statements as to the date or representation as to the contents of these documents is based on the information available to the applicant and does not constitute any admission as to the correctness of the dates or contents of these documents.
1 Bruno L, Seidl T, Lanzavecchia A: Mouse pre-immunocytes as non-proliferating multipotent precursors of macrophages, interferon-producing cells, CDBalpha(+) and CD8alpha(-) dendritic cells. Eur J Immunol 31:3403-3412 (2001).
2 Bunt SK, Sinha P, Clements VK, Leips J, Ostrand-Rosenberg S: Inflammation induces myeloid-derived suppressor cells that facilitate tumor progression. J Immunol 176:284-290 (2006).
3 D'Amico A, Wu L: The early progenitors of mouse dendritic cells and plasmacytoid predendritic cells are within the bone marrow hemopoietic precursors expressing Flt3. J Exp Med 198:293-303 (2003).
4 Dakic A, Wu L: Hemopoietic precursors and development of dendritic cell populations. Leuk Lymphoma 44:1469-1475 (2003).
5 del Hoyo GM, Martin P, Vargas HH, Ruiz S, Arias CF, Ardavin C: Characterization of a common precursor population for dendritic cells. Nature 415:1043-1047 (2002).
6 Diao J, Winter E, Cantin C, Chen W, Xu L, Kelvin D, Phillips J, Cattral MS: In situ replication of immediate dendritic cell (DC) precursors contributes to conventional DC homeostasis in lymphoid tissue. J Immunol 176:7196-7206 (2006).
7 Diao J, Winter E, Chen W, Cantin C, Cattral MS: Characterization of distinct conventional and plasmacytoid dendritic cell-committed precursors in murine bone marrow. J Immunol 173:1826-1833 (2004).
8 Fogg DK, Sibon C, Miled C, Jung S, Aucouturier P, Littman DR, Cumano A, Geissmann F: A clonogenic bone marrow progenitor specific for macrophages and dendritic cells. Science 311:83-87 (2006).
9 Gallina G, Dolcetti L, Serafini P, De SC, Marigo I, Colombo MP, Basso G, Brombacher F, Borrello I, Zanovello P, Bicciato S, Bronte V: Tumors induce a subset of inflammatory monocytes with immunosuppressive activity on CD8+ T cells. J Clin Invest 116:2777-2790 (2006).
10 Ginhoux F, Tacke F, Angeli V, Bogunovic M, Loubeau M, Dai XM, Stanley ER, Randolph GJ, Merad M: Langerhans cells arise from monocytes in vivo. Nat Immunol 7:265-273 (2006).
11 Iijima N, Linehan MM, Saeland S, Iwasaki A: Vaginal epithelial dendritic cells renew from bone marrow precursors. Proc Natl Acad Sci U S A 104:19061-19066 (2007).
12 Karsunky H, Merad M, Cozzio A, Weissman IL, Manz MG: Flt3 ligand regulates dendritic cell development from Flt3+ lymphoid and myeloid-committed progenitors to Flt3+ dendritic cells in vivo. J Exp Med 198:305-313 (2003).
13 Larregina AT, Morelli AE, Spencer LA, Logar AJ, Watkins SC, Thomson AW, Falo LD, Jr.: Dermal-resident CD14+ cells differentiate into Langerhans cells. Nat Immunol 2:1151-1158 (2001).
14 Makarenkova VP, Bansal V, Matta BM, Perez LA, Ochoa JB: CD11b+/Gr-1+ myeloid suppressor cells cause T cell dysfunction after traumatic stress. J Immunol 176:2085-2094 (2006).
15 Marhaba R, Vitacolonna M, Hildebrand D, Baniyash M, Freyschmidt-Paul P, Zoller M: The importance of myeloid-derived suppressor cells in the regulation of autoimmune effector cells by a chronic contact eczema. J Immunol 179:5071-5081 (2007).
16 Mende I, Karsunky H, Weissman IL, Engleman EG, Merad M: Flk2+ myeloid progenitors are the main source of Langerhans cells. Blood 107:1383-1390 (2006).
17 Mizumoto N, Gao J, Matsushima H, Ogawa Y, Tanaka H, Takashima A: Discovery of novel immunostimulants by dendritic cell-based functional screening. Blood 106:3082-3089 (2005a).
18 Mizumoto N, Hui F, Edelbaum D, Weil MR, Wren JD, Shalhevet D, Matsue H, Liu L, Garner HR, Takashima A: Differential activation profiles of multiple transcription factors during dendritic cell maturation. J Invest Dermatol 124:718-724 (2005b).
19 Naik SH: Demystifying the development of dendritic cell subtypes, a little. Immunol Cell Biol 86:439-452 (2008).
20 Naik SH, Metcalf D, van NA, Wicks I, Wu L, O'Keeffe M, Shortman K: Intrasplenic steady-state dendritic cell precursors that are distinct from monocytes. Nat Immunol 7:663-671 (2006).
21 Naik SH, Sathe P, Park HY, Metcalf D, Proietto AI, Dakic A, Carotta S, O'Keeffe M, Bahlo M, Papenfuss A, Kwak JY, Wu L, Shortman K: Development of plasmacytoid and conventional dendritic cell subtypes from single precursor cells derived in vitro and in vivo. Nat Immunol 8:1217-1226 (2007).
22 O'Keeffe M, Hochrein H, Vremec D, Scott B, Hertzog P, Tatarczuch L, Shortman K: Dendritic cell precursor populations of mouse blood: identification of the murine homologues of human blood plasmacytoid pre-DC2 and CD11c+ DC1 precursors. Blood 101:1453-1459 (2003).
23 Onai N, Obata-Onai A, Schmid MA, Ohteki T, Jarrossay D, Manz MG: Identification of clonogenic common Flt3+M-CSFR+ plasmacytoid and conventional dendritic cell progenitors in mouse bone marrow. Nat Immunol 8:1207-1216 (2007).
24 Randolph GJ, Inaba K, Robbiani DF, Steinman RM, Muller WA: Differentiation of phagocytic monocytes into lymph node dendritic cells in vivo. Immunity 11:753-761 (1999).
25 Rossner S, Voigtlander C, Wiethe C, Hanig J, Seifarth C, Lutz MB: Myeloid dendritic cell precursors generated from bone marrow suppress T cell responses via cell contact and nitric oxide production in vitro. Eur J Immunol 35:3533-3544 (2005).
26 Shortman K, Liu YJ: Mouse and human dendritic cell subtypes. Nat Rev Immunol 2:151-161 (2002).
27 Shortman K, Naik SH: Steady-state and inflammatory dendritic-cell development. Nat Rev Immunol 7:19-30 (2007).
28 Sinha P, Clements VK, Ostrand-Rosenberg S: Reduction of myeloid-derived suppressor cells and induction of M1 macrophages facilitate the rejection of established metastatic disease. J Immunol 174:636-645 (2005).
29 Villadangos JA, Schnorrer P: Intrinsic and cooperative antigen-presenting functions of dendritic-cell subsets in vivo. Nat Rev Immunol 7:543-555 (2007).
30 Wang Y, Zhang Y, Yoneyama H, Onai N, Sato T, Matsushima K: Identification of CD8alpha+CD11c- lineage phenotype-negative cells in the spleen as committed precursor of CD8alpha+ dendritic cells. Blood 100:569-577 (2002).
31 Welner RS, Pelayo R, Garrett KP, Chen X, Perry SS, Sun XH, Kee BL, Kincade PW: Interferon-producing killer dendritic cells (IKDCs) arise via a unique differentiation pathway from primitive c-kitHiCD62L+ lymphoid progenitors. Blood 109:4825-4931 (2007).
32 Wu L, Dakic A: Development of dendritic cell system. Cell Mol Immunol 1:112-118 (2004).
33 Wu L, Liu YJ: Development of dendritic-cell lineages. Immunity 26:741-750 (2007).
34 Zhu B, Bando Y, Xiao S, Yang K, Anderson AC, Kuchroo VK, Khoury SJ: CD11b+Ly-6C(hi) suppressive monocytes in experimental autoimmune encephalomyelitis. J Immunol 179:5228-5237 (2007).
35 Zuniga EI, McGavern DB, Pruneda-Paz JL, Teng C, Oldstone MB: Bone marrow plasmacytoid dendritic cells can differentiate into myeloid dendritic cells upon virus infection. Nat Immunol 5:1227-1234 (2004).

## Claims

1. An *in vitro* method for isolating a dendritic cell precursor cell line, comprising:
isolating dendritic cell precursor cells from a subject;
placing in culture the isolated cells in a suitable culture medium containing an effective amount of GM-CSF to generate a dendritic cell precursor cell line from the isolated cells, and
multiplying the generated cell line by means of successive cell divisions, so as to obtain a dendritic cell line specific to the subject, wherein the isolated cells have a CD48 negative/MHC class I-negative phenotype.

2. The method of claim 1, wherein the cells are isolated after at least 20, 30, 40, 50, 60, 70, 80, 90, and preferably at least 100, cell divisions.

3. The method of claim 1, further including cloning the dendritic cell line obtained so as to obtain various dendritic cell lines or clones, wherein the cloning of a line denotes the individualization of cells of this line, and a collection of genetically identical cells obtained from a single cell.

4. The method of claim 3, further including selecting one of the various dendritic cell lines or clones, so as to identify at least one clone having a phenotype of interest.

## Patentansprüche

1. *In*-*vitro*-Verfahren zum Isolieren einer dendritischen Zellvorläuferzelllinie, wobei das Verfahren Folgendes umfasst:
Isolieren von dendritischen Zellvorläuferzellen von einer Person;
Geben der isolierten Zellen in Kultur in einem geeigneten Kulturmedium, das eine wirksame Menge an GM-CSF enthält, um eine dendritische Zellvorläuferzelllinie aus den isolierten Zellen zu erzeugen, und
Vervielfältigen der erzeugten Zelllinie mittels aufeinanderfolgender Zellteilungen, um eine dendritische Zelllinie zu erhalten, die für die Person spezifisch ist, wobei die isolierten Zellen einen CD48-negativen/MHC-Klasse-I-negativen Phänotypen aufweisen.

2. Verfahren nach Anspruch 1, wobei die Zellen nach mindestens 20, 30, 40, 50, 60, 70, 80, 90 und vorzugsweise mindestens 100 Zellteilungen isoliert werden.

3. Verfahren nach Anspruch 1, das weiterhin das Klonieren der erhaltenen dendritischen Zelllinie beinhaltet, um verschiedene dendritische Zelllinien oder Klone zu erhalten, wobei das Klonieren einer Linie die Individualisierung von Zellen dieser Linie und eine Sammlung von genetisch identischen Zellen, die aus einer einzigen Zelle erhalten werden, bezeichnet.

4. Verfahren nach Anspruch 3, das weiterhin das Auswählen einer der verschiedenen dendritischen Zelllinien oder Klone beinhaltet, um mindestens einen Klon zu identifizieren, der einen Phänotypen von Interesse aufweist.

## Revendications

1. Procédé *in vitro* permettant d'isoler une lignée de cellules précurseurs de cellules dendritiques, consistant à :
isoler des cellules précurseurs de cellules dendritiques provenant d'un sujet ;
mettre en culture les cellules isolées dans un milieu de culture approprié contenant une quantité efficace de GM-CSF pour générer une lignée de cellules précurseurs de cellules dendritiques à partir des cellules isolées et
multiplier la lignée cellulaire générée au moyen de divisions cellulaires successives, de manière à obtenir une lignée de cellules dendritiques spécifique du sujet, dans lequel les cellules isolées ont un phénotype CD48 négatif/CMH de classe I négatif.

2. Procédé selon la revendication 1, dans lequel les cellules sont isolées au bout d'au moins 20, 30, 40, 50, 60, 70, 80, 90, et de préférence au moins 100, divisions cellulaires.

3. Procédé selon la revendication 1, comprenant en outre le clonage de la lignée de cellules dendritiques obtenue de manière à obtenir diverses lignées de cellules dendritiques ou divers clones, dans lequel le clonage d'une lignée désigne l'individualisation de cellules de cette lignée et un recueil de cellules génétiquement identiques obtenues à partir d'une seule cellule.

4. Procédé selon la revendication 3, comprenant en outre la sélection de l'une des diverses lignées de cellules dendritiques ou de l'un des divers clones, de manière à identifier au moins un clone ayant un phénotype présentant de l'intérêt.
